# EUROPEAN PATENT APPLICATION

(11) **EP 4 477 163 A1**
(43) Date of publication of application: **18.12.2024**
(21) Application number: 23778497.0
(22) Date of filing: 31.03.2023
(51) Int. Cl.: A61B 17/34, A61B 34/10, G06T 7/00, G06T 7/187

(54) **SYSTEM AND METHOD FOR PUNCTURE PATH PLANNING**

(30) Priority: 02.04.2022 CN 202210342911; 25.05.2022 CN 202210577448; 30.06.2022 CN 202210764219
(71) Applicant: Wuhan United Imaging Surgical Co., Ltd., Wuhan, Hubei 430073 (CN)
(72) Inventor: WANG, Guoqiang, Wuhan, Hubei 430073 (CN); LIAO, Mingzhe, Wuhan, Hubei 430073 (CN); WANG, Ke, Wuhan, Hubei 430206 (CN); FANG, Wei, Wuhan, Hubei 430206 (CN); ZHANG, Jing, Wuhan, Hubei 430206 (CN); ZHANG, Tian, Wuhan, Hubei 430206 (CN)
(74) Representative: Wang, Bo
(86) International application number: PCT/CN2023/085618
(87) International publication number: WO 2023/186133

(57) **Abstract**

A system (100) and a method for puncture path planning are provided. The system (100) comprises: at least one storage medium including a set of instructions; and one or more processors (210) communicating with the at least one storage medium. When executing the instructions, the one or more processors (210) are used to: determine a target point (410) based on a target image; determine one or more candidate paths (420) based on the target point and at least two constraints, wherein in the process of determining the one or more candidate paths, a path planning condition is adaptively adjusted based on a first preset condition; and determine a target path (430) based on the one or more candidate paths.

## Description

### CROSS-REFERENCE TO RELATED APPLICATION

This application claims priority to Chinese Patent Application No. 202210342911.7, filed on April 2, 2022, Chinese Patent Application No. 202210577448.4, filed on May 25, 2022, and Chinese Patent Application No. 202210764219.3, filed on June 30, 2022, the entire contents of each of which are incorporated herein by reference.

### TECHNICAL FIELD

The present disclosure relates to the field of medical technology, and in particular to a method and a system for puncture path planning.

### BACKGROUND

Puncture biopsy is a method of obtaining a small amount of tissue for pathological examination and diagnosis by puncturing into a target organ (e.g., a diseased organ or an organ to be tested) for suction under the guidance of medical imaging equipment. As the main method for pathological diagnosis, puncture biopsy is widely used in clinical scenarios. Planning a puncture path is crucial in the puncture biopsy, which requires to select an appropriate length of a puncture needle, a skin entry point and an insertion angle, and requires maintaining a certain safe distance from sensitive tissues (e.g., blood vessels and bones) inside and/or around the target organ to avoid complications caused by the puncture.

### SUMMARY

One of the embodiments of the present disclosure provides a system for puncture path planning. The system may include at least one storage medium including a set of instructions; and one or more processors in communication with the at least one storage medium. When executing the instructions, the one or more processors may be configured to determine a target point based on a target image, and determine one or more candidate paths based on the target point and at least two constraints. A path planning condition may be adaptively adjusted based on a first preset condition in the process of determining the one or more candidate paths. The one or more processors may be further configured to determine a target path based on the one or more candidate paths.

In some embodiments, the determining the target point based on a target image may include: obtaining a target structure mask by performing rough segmentation on a target structure in the target image; determining positioning information of the target structure mask based on soft connected domain analysis; obtaining a segmentation result by performing precise segmentation on the target structure based on the positioning information of the target structure mask; and determining the target point based on the segmentation result.

In some embodiments, the determining positioning information of the target structure mask based on soft connected domain analysis may include: determining a count of connected domains in the target structure mask; and determining the positioning information of the target structure mask based on the count of the connected domains.

In some embodiments, the positioning information of the target structure mask may include position information of a bounding rectangle of the target structure mask; and/or the determining the positioning information of the target structure mask may include: positioning the target structure mask based on positioning coordinates of a preset structure.

In some embodiments, the performing precise segmentation on the target structure based on the positioning information of the target structure mask may include: obtaining a preliminary precise segmentation result by performing preliminary precise segmentation on the target structure; determining whether the positioning information of the target structure mask is accurate based on the preliminary precise segmentation result; and in response to determining that the positioning information of the target structure mask is accurate, using the preliminary precise segmentation result as a target segmentation result; or in response to determining that the positioning information of the target structure mask is not accurate, determining the target segmentation result of the target structure by an adaptive sliding window mode.

In some embodiments, the one or more processors may be further configured to: obtain a first segmentation result of the target image based on a first segmentation model; obtain a first duct skeleton set by performing skeletonization processing on the first segmentation result, wherein the first duct skeleton set includes at least one first duct skeleton of a determined type; obtain a second segmentation result of the target image based on a second segmentation model, wherein the second segmentation result includes at least one duct of an undetermined type; obtain a fusion result by fusing the first segmentation result and the second segmentation result; and determine a dangerous region based on the fusion result.

In some embodiments, at least one duct in the second segmentation result may not be included in the first segmentation result; and the determining a dangerous region based on the fusion result may include: obtaining a second duct skeleton of one of the at least one duct of the undetermined type by performing the skeletonization processing on the fusion result; obtaining one or more first duct skeletons, a minimum spatial distance between each of the one or more first duct skeletons and the second duct skeleton is less than a second threshold, designating the one or more first duct skeletons as one or more reference duct skeletons; determining one or more spatial distances each of which is between the second duct skeleton and one of the one or more reference duct skeletons, and determining two points with a minimum spatial distance among the one or more spatial distances as a set of closest points; determining a duct type of the one of the at least one duct of the undetermined type based on the set of closest points; and determining the dangerous region based on the duct type.

In some embodiments, the at least two constraints may include: a distance between a path and a dangerous region may be greater than a preset distance threshold, the path may be located in a slice layer adjacent to a slice layer where a target region is located, a needle entry point on a body contour that contacts a bed board may be excluded, a puncture depth of the path may be less than a preset depth threshold, or an angle between the path and a vertical line of a flat surface of a flat lesion may be within a preset range.

In some embodiments, the determining one or more candidate paths based on the target point and at least two constraints may include: determining initial paths based on the target point and a first constraint; determining the one or more candidate paths from the initial paths based on a second constraint; wherein the first constraint may include that: the path may be located in the slice layer adjacent to the slice layer where the target region is located, the needle entry point on the body contour that contacts the bed board may be excluded, the puncture depth of the path may be less than the preset depth threshold, or the angle between the path and the vertical line of the flat surface of the flat lesion may be within a preset range; and the second constraint may include that the distance between the path and the dangerous region may be greater than the preset distance threshold.

In some embodiments, the adaptively adjusting a path planning condition based on a first preset condition may include: when no candidate path meets the path planning condition, resetting puncture parameters, the puncture parameters including at least a length and/or a diameter of a puncture needle.

In some embodiments, the determining a target path based on the one or more candidate paths may include: in response to determining that the one or more candidate paths include one or more coplanar candidate paths and one or more non-coplanar candidate paths, determining the target path based on a shortest puncture depth D₁ in the one or more non-coplanar candidate paths, a shortest puncture depth D₂ in in the one or more coplanar candidate paths with a small angle deflection perpendicular to a direction of a bed board, and a shortest puncture depth D₃ in in the one or more coplanar candidate paths with a non-small angle deflection; if each of the one or more candidate paths include is a non-coplanar candidate path, determining the target path based on the Di; and if each of the one or more candidate paths is a coplanar candidate path, determining the target path based on the D₂ and the D₃.

One of the embodiments of the present disclosure provides a system for medical image segmentation. The system may comprise: at least one storage medium including a set of instructions; and one or more processors in communication with the at least one storage medium. When executing the instructions, the one or more processors may be configured to: obtain a target image; obtain a target structure mask by performing rough segmentation on a target structure in the target image; determine positioning information of the target structure mask based on soft connected domain analysis; and obtain a segmentation result by performing precise segmentation on the target structure based on the positioning information of the target structure mask.

One of the embodiments of the present disclosure provides a system for duct recognition in an organism. The system may comprise at least one storage medium comprising a set of instructions; and one or more processors in communication with the at least one storage medium. When executing the instructions, the one or more processors may be configured to: obtain a target image of the organism; obtain a first segmentation result of the target image based on a first segmentation model; obtain a first duct skeleton set by performing skeletonization processing on the first segmentation result, wherein the first duct skeleton set may include at least one first duct skeleton of a determined type; obtain a second segmentation result of the target image based on a second segmentation model, wherein the second segmentation result may include at least one duct of an undetermined type; and obtain a fusion result by fusing the first segmentation result and the second segmentation result.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering indicates the same structure, wherein:
FIG. 1 is a schematic diagram illustrating an application scenario of an exemplary system for puncture path planning according to some embodiments of the present disclosure;
FIG. 2 is a schematic diagram illustrating a hardware and/or a software of an exemplary computing device according to some embodiments of the present specification;
FIG. 3 is a module diagram illustrating an exemplary device for puncture path planning according to some embodiments of the present disclosure;
FIG. 4 is a flowchart illustrating an exemplary process of puncture path planning according to some embodiments of the present disclosure;
FIG. 5 is a module diagram illustrating an exemplary device for image segmentation according to some embodiments of the present disclosure;
FIG. 6 is a flowchart illustrating an exemplary process of image segmentation according to some embodiments of the present disclosure;
FIG. 7 is a flowchart illustrating an exemplary process of determining positioning information of a target structure mask according to some embodiments of the present disclosure;
FIG. 8 is a flowchart illustrating an exemplary process of determining positioning information of a target structure mask according to some embodiments of the present disclosure;
FIG. 9 is a schematic diagram illustrating an exemplary process of determining positioning information of a target structure mask according to some embodiments of the present disclosure;
FIG. 10 is a schematic diagram illustrating a comparison of exemplary rough segmentation results according to some embodiments of the present disclosure;
FIG. 11 is a flowchart illustrating an exemplary precise segmentation process according to some embodiments of the present disclosure;
FIG. 12 is a schematic diagram illustrating an exemplary process of determining positioning information of a target structure mask according to some embodiments of the present disclosure;
FIG. 13 is a schematic diagram illustrating an exemplary process of determining a sliding direction according to some embodiments of the present disclosure;
FIG. 14 is a schematic diagram illustrating an exemplary process of performing precise segmentation after sliding window according to some embodiments of the present disclosure;
FIG. 15 is a schematic diagram illustrating a comparison of exemplary segmentation results according to some embodiments of the present disclosure;
FIG. 16 is a module diagram illustrating an exemplary device for duct recognition according to some embodiments of the present disclosure;
FIG. 17 is a flowchart illustrating an exemplary process for duct recognition according to some embodiments of the present disclosure;
FIG. 18 is a schematic diagram illustrating an exemplary duct recognition result according to some embodiments of the present disclosure;
FIG. 19 is a flowchart illustrating an exemplary process of determining a duct type according to some embodiments of the present disclosure;
FIG. 20 is a flowchart illustrating an exemplary process of determining a duct type according to some embodiments of the present disclosure;
FIG. 21 is a schematic diagram illustrating an exemplary process of determining a duct type according to some embodiments of the present disclosure;
FIG. 22 is a schematic diagram illustrating an exemplary process of determining a duct type according to some embodiments of the present disclosure;
FIG. 23 is a schematic diagram illustrating an exemplary process of model training according to some embodiments of the present disclosure;
FIG. 24 is a flowchart illustrating an exemplary process of puncture path planning according to some embodiments of the present disclosure;
FIG. 25 is a schematic diagram illustrating an exemplary process of determining a target point according to some embodiments of the present disclosure;
FIGs. 26A-26C are schematic diagrams illustrating an exemplary process of determining initial paths according to some embodiments of the present disclosure;
FIG. 27 is a schematic diagram illustrating exemplary candidate paths according to some embodiments of the present disclosure; and
FIG. 28 is a schematic diagram illustrating an exemplary process of puncture path planning according to other embodiments of the present disclosure.

### DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for a person of ordinary skill in the art to apply the present disclosure to other similar scenarios in accordance with these drawings without creative labor. Unless obviously obtained from the context or the context illustrates otherwise, the same numeral in the drawings refers to the same structure or operation.

It should be understood that the terms "system", "device", "unit" and/or "module" used herein are a way to distinguish between different components, elements, parts, sections, or assemblies at different levels. However, the terms may be replaced by other expressions if other words accomplish the same purpose.

As shown in the present disclosure and in the claims, unless the context clearly suggests an exception, the words "one", "a", "an", "one kind", and/or "the" do not refer specifically to the singular, but may also include the plural. Generally, the terms "including" and "comprising" suggest only the inclusion of clearly identified steps and elements, however, the steps and elements that do not constitute an exclusive list, and the method or apparatus may also include other steps or elements.

Flowcharts are used in the present disclosure to illustrate the operations performed by a system according to embodiments of the present disclosure, and the related descriptions are provided to aid in a better understanding of the magnetic resonance imaging method and/or system. It should be appreciated that the preceding or following operations are not necessarily performed in an exact sequence. Instead, steps can be processed in reverse order or simultaneously. Also, it is possible to add other operations to these processes or to remove a step or steps from these processes.

The method for duct recognition in an organism provided in the embodiments of the present disclosure can be applied to the determination of a duct type in an animal. For the convenience of explanation, the specific embodiments of the present disclosure will be mainly described by taking the determination of a blood vessel type in a human body as an example. However, for those having ordinary skills in the art, the present disclosure can be applied to other similar scenarios without creative effort, such as the determination of other ducts and/or duct types in the human body, or blood vessel types or other duct types of other animals (e.g., a dog, a cat, etc.).

In conventional puncture methods, medical staff generally select an appropriate puncture path based on the experience, which needs high requirements for the medical staff and results in low puncture efficiency. A method for puncture path planning provided in the embodiments of the present disclosure may include performing automatically organ segmentation on a target image to locate an optimal target point, and adaptively selecting an optimal puncture instrument and puncture path based on the target point and at least two constraints, making the selection of the puncture path more intelligent and more in line with clinical needs, thereby improving the accuracy and efficiency of puncture biopsy.

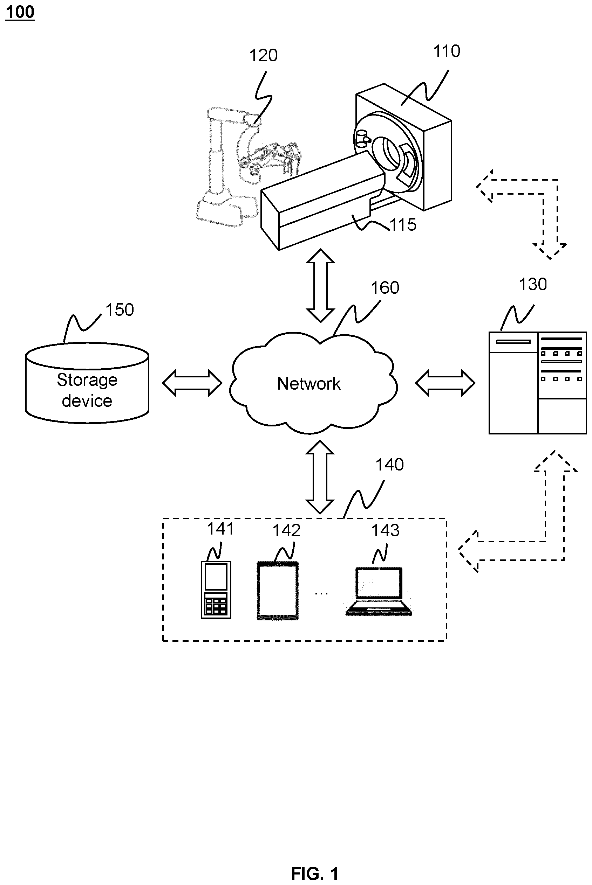

FIG. 1 is a schematic diagram illustrating an application scenario of an exemplary system for puncture path planning according to some embodiments of the present disclosure.

As shown in FIG. 1, a system 100 for puncture path planning may include an imaging device 110, an end effector 120, a processing device 130, a terminal device 140, a storage device 150, and a network 160. In some embodiments, the processing device 130 may be a part of the imaging device 110 and /or the end effector 120.

Connections between components in the system 100 for puncture path planning may be variable. As shown in FIG. 1, in some embodiments, the imaging device 110 may be connected with the processing device 130 via the network 160. As another example, the imaging device 110 may be directly connected with the processing device 130, as indicated by a dashed double-sided arrow connecting the imaging device 110 and the processing device 130. As another example, the storage device 150 may be connected with the processing device 130 directly or via the network 160. Merely by way of example, the terminal device 140 may be directly connected with the processing device 130 (as indicated by a dashed arrow connecting the terminal device 140 and the processing device 130), or may be connected with the processing device 130 via the network 160.

The imaging device 110 may be configured to scan a target object (a scanning object) in a detection region or a scanning region to obtain scanning data (e.g., a target image) of the target object. For example, the imaging device 110 may be configured to scan the target object using high-energy rays (e.g., X-rays, Gamma rays, etc.) to collect the scanning data related to the target object, such as a three-dimensional (3D) image. The target object may include a biological or non-biological object. Merely by way of example, the target object may include a patient, an artificial object (e.g., an artificial phantom), etc. As another example, the target object may include a specific part, organ, and/or tissue (e.g., head, ear, nose, mouth, neck, chest, abdomen, liver, gallbladder, pancreas, spleen, kidney, spine, heart, or a tumor tissue, etc.) of the patient.

In some embodiments, the imaging device 110 may include a single-modal scanner and/or a multi-modal scanner. The single-modal scanner may include, for example, an X-ray scanner, a computed tomography (CT) scanner, a magnetic resonance imaging (MRI) scanner, a positron emission computed tomography (PET) scanner, an optical coherence tomography (OCT) scanner, an ultrasound (US) scanner, an intraduct ultrasound (IVUS) scanner, a near-infrared spectroscopy (NIRS) scanner, a far infrared (FIR) scanner, a digital radiography (DR) scanner (e.g., a mobile digital radiography), a digital subtraction angiography (DSA) scanner, a dynamic spatial reconstruction (DSR) scanner, etc. The multi-modal scanner may include, for example, an X-ray imaging-magnetic resonance imaging (X-ray-MRI) scanner, a positron emission tomography-X-ray imaging (PET-X-ray) scanner, a single photon emission computed tomography-magnetic resonance imaging (SPECT-MRI) scanner, a positron emission tomography-computed tomography (PET-CT) scanner, a digital subtraction angiography-magnetic resonance imaging (DSA-MRI) scanner, etc. The above description of the imaging device is for illustrative purposes only and is not intended to limit the scope of the present disclosure.

In some embodiments, the imaging device 110 may include a medical bed 115. The medical bed 115 may be configured to carry the target object so as to scan the target object and obtain the target image. In some embodiments, the medical bed 115 may include an automatic medical bed and/or a hand-propelled medical bed. In some embodiments, the medical bed 115 may be independent of the imaging device 110.

In some embodiments, the imaging device 110 may include a display device. The display device may be configured to display the scanning data (e.g., the target image, a segmented image, a puncture path, etc.) of the target object. In some embodiments, the imaging device 110 may further include a gantry, a detector, a workbench, a radiation source, etc. (not shown in the figure). The gantry may be configured to support the detector and the radiation source. The target object may be placed on the workbench for scanning. The radiation source may be configured to emit radioactive rays to the target object. The detector may be configured to detect the radioactive rays (e.g., the X-rays) emitted from the radiation source. In some embodiments, the detector may include one or more detector units. The one or more detector units may include a scintillation detector (e.g., a cesium iodide detector), a gas detector, etc. The one or more detector units may include a single-row detector and/or a multi-row detector.

The end effector 120 refers to a robot that performs one or more end operations (e.g., ablation, puncture, and implantation of radioactive particles). In some embodiments, the processing device 130 may guide the end effector 120 to perform corresponding operations (e.g., a puncture operation) via remote operation and control. In some embodiments, the end effector 120 may include a robotic arm end, a functional component (e.g., a puncture needle), and a robot host. In some embodiments, the robotic arm end may be configured to carry the functional component, and the robot host may be a robotic arm body, which is used to drive the robotic arm end to move to adjust the posture (e.g., an angle, a position, etc.) of the functional component.

In some embodiments, the processing device 130 may be connected with the robotic arm body or the end of the robotic arm end via a communication device (e.g., the network 160) to control the robotic arm end to drive the functional component (e.g., the puncture needle, etc.) to perform a synchronous operation. For example, the processing device 130 may drive the puncture needle to perform the puncture operation by controlling the robotic arm end to rotate, translate, advance, etc.

In some embodiments, the end effector 120 may further include a master hand manipulator. The master hand manipulator may be electrically connected with the robot host or the robotic arm end via the communication device (e.g., the network 160) to control the robotic arm end to drive the functional component (e.g., the puncture needle, etc.) to perform the puncture operation.

The processing device 130 may be configured to process data and/or information obtained from the imaging device 110, the end effector 120, the terminal device 140, the storage device 150, or other components of the system 100 for puncture path planning. For example, the processing device 130 may obtain the target image (e.g., a CT image, a PET image, an MR image, etc.) from the imaging device 110, analyze and process (e.g., perform rough segmentation, precise segmentation, etc., on a target structure, and/or perform duct recognition, duct type recognition, etc.) the target image to determine a target point, and determine a target path based on the target point, etc. In some embodiments, the processing device 130 may be local or remote. For example, the processing device 130 may access the information and/or data from the imaging device 110, the end effector 120, the terminal device 140, and/or the storage device 150 via the network 160.

In some embodiments, the processing device 130 and the imaging device 110 may be integrated as a whole. In some embodiments, the processing device 130 and the imaging device 110 may be directly or indirectly connected to jointly implement the methods and/or functions described in the present disclosure.

In some embodiments, the processing device 130 and the end effector 120 may be integrated as a whole. In some embodiments, the processing device 130 and the end-effector 120 may be directly or indirectly connected to jointly implement the methods and/or functions described in the present disclosure. In some embodiments, the imaging device 110, the end effector 120, and the processing device 130 may be integrated as a whole. In some embodiments, the imaging device 110, the end effector 120, and the processing device 130 may be directly or indirectly connected to jointly implement the methods and/or functions described in the present disclosure.

In some embodiments, the processing device 130 may include an input device and/or an output device. Interaction with a user (e.g., displaying the target image, the segmented image, the target paths, etc.) may be achieved through the input device and/or the output device. In some embodiments, the input device and/or the output device may include a display screen, a keyboard, a mouse, a microphone, or the like, or any combination thereof.

The terminal device 140 may be connected and/or communicate with the imaging device 110, the end effector 120, the processing device 130, and/or the storage device 150. For example, the terminal device 140 may obtain the target image after organ or tissue segmentation is completed from the processing device 130 and display the target image such that the user may understand patient information. As another example, the terminal device 140 may obtain an image after the duct recognition from the processing device 130 and display the image. In some embodiments, the terminal device 140 may include a mobile device 141, a tablet computer 142, a laptop computer 143, or the like, or any combination thereof. In some embodiments, the terminal device 140 (or all or part of the functions thereof) may be integrated in the processing device 130.

The storage device 150 may be configured to store data, instructions, and/or any other information. In some embodiments, the storage device 150 may store the data (e.g., the target image, the segmented image, the initial paths, one or more candidate paths, target path, puncture parameters, etc.) obtained from the imaging device 110, the end effector 120, and/or the processing device 130. In some embodiments, the storage device 150 may be configured to store computer instructions for implementing the method for puncture path planning, etc.

In some embodiments, the storage device 150 may include one or more storage components. Each of the one or more storage components may be an independent device or a part of another device. In some embodiments, the storage device 150 may include a random-access memory (RAM), a read-only memory (ROM), a mass storage device, a removable memory, a volatile read-write memory, or the like, or any combination thereof. For example, the mass storage device may include a magnetic disk, an optical disk, a solid-state disk, etc. The RAM may include a dynamic RAM (DRAM), a double data rate synchronous dynamic RAM (DDR SDRAM), a static RAM (SRAM), a thyristor RAM (T-RAM), zero capacitance (Z-RAM), etc. The ROM may include a mask ROM (MROM), a programmable ROM (PROM), an erasable programmable ROM (PEROM), an electrically erasable programmable ROM (EEPROM), an optical disk ROM (CD-ROM), a digital universal disk ROM, etc. In some embodiments, the storage device 150 may be implemented on a cloud platform.

The network 160 may include any suitable network capable of facilitating information and/or data exchange. In some embodiments, at least one component (e.g., the imaging device 110, the end effector 120, the processing device 130, the terminal device 140, and the storage device 150) of the system 100 for puncture path planning may exchange information and/or data with at least one other component of the system 100 for puncture path planning via the network 160. For example, the processing device 130 may obtain the target image from the imaging device 110 via the network 160.

It should be noted that the system 100 for puncture path planning is provided for illustrative purposes only and is not intended to limit the scope of the present disclosure. For those of ordinary skill in the art, various modifications or variations can be made based on the description of the present disclosure. For example, the system 100 for puncture path planning can implement similar or different functions on other devices. However, these changes and modifications do not deviate from the scope of the present disclosure.

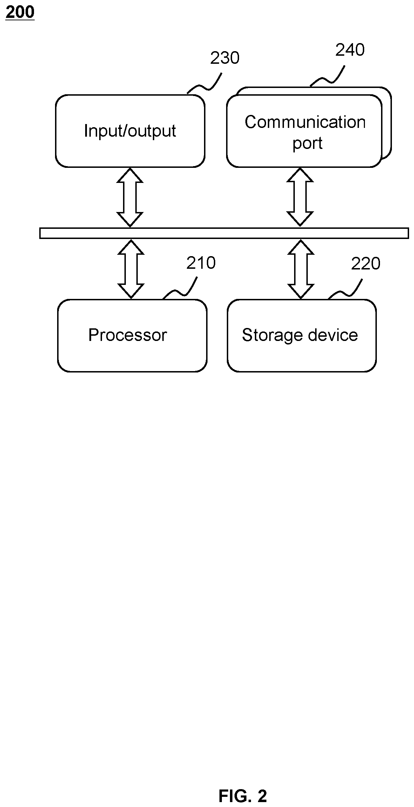

FIG. 2 is a schematic diagram illustrating a hardware and/or a software of an exemplary computing device according to some embodiments of the present specification.

As shown in FIG. 2, a computing device 200 may include a processor 210, a storage device 220, an input/output interface 230, and a communication port 240.

The processor 210 may execute computing instructions (program codes) and functions of the system 100 for puncture path planning described in the present disclosure. The computing instructions may include programs, objects, components, data structures, processes, modules, and functions (the functions refer to specific functions described in the present disclosure). For example, the processor 210 may process images and/or data obtained from any component of the system 100 for puncture path planning. For example, the processor 210 may obtain a target structure mask by performing rough segmentation on a target structure in a target image obtained from the imaging device 110; determine positioning information of the target structure mask based on soft connected domain analysis; and obtain a segmentation result of the target image by performing precise segmentation on the target structure based on the positioning information of the target structure mask, thereby performing puncture path planning. As another example, the processor 210 may obtain a target image of an organism from the imaging device 110; obtain a first segmentation result of the target image based on a first segmentation model; obtain a second segmentation result of the target image based on a second segmentation model; and obtain a fusion result by fusing the first segmentation result and the second segmentation result. In some embodiments, the processor 210 may include a microcontroller, a microprocessor, a reduced instruction set computer (RISC), an application-specific integrated circuit (ASIC), an application-specific instruction set processor (ASIP), a central processing unit (CPU), a graphics processing unit (GPU), a physical processing unit (PPU), a microcontroller unit, a digital signal processor (DSP), a field programmable gate array (FPGA), an advanced RISC machine (ARM), a programmable logic device, and any circuit and processor capable of performing one or more functions, etc., or any combination thereof. For illustration purposes only, the computing device 200 in FIG. 2 only describes one processor, but it should be noted that the computing device 200 in the present disclosure may also include a plurality of processors.

The storage device 220 may store data/information obtained from any other component of the system 100 for puncture path planning. In some embodiments, the storage device 220 may include a mass storage device, a removable memory, a volatile read/write memory, a ROM, or the like, or any combination thereof.

The input/output interface 230 may be configured to input or output signals, data or information. In some embodiments, the input/output interface 230 enables a user to communicate with the system 100 for puncture path planning. In some embodiments, the input/output interface 230 may include an input device and an output device. The communication port 240 may be connected to a network for data communication. The connection may be a wired connection, a wireless connection, or a combination thereof. The wired connection may include an electric cable, an optical cable, a telephone line, or the like, or any combination thereof. The wireless connection may include one or more of Bluetooth^{™}, Wi-Fi, WiMax, WLAN, ZigBee^{™}, a mobile network (e.g., 3G, 4G or 5G, etc.), etc. In some embodiments, the communication port 240 may be a standardized port, such as RS232, RS485, etc. In some embodiments, the communication port 240 may be a specially set port. For example, the communication port 240 may be set according to a digital imaging and medical communication protocol (DICOM).

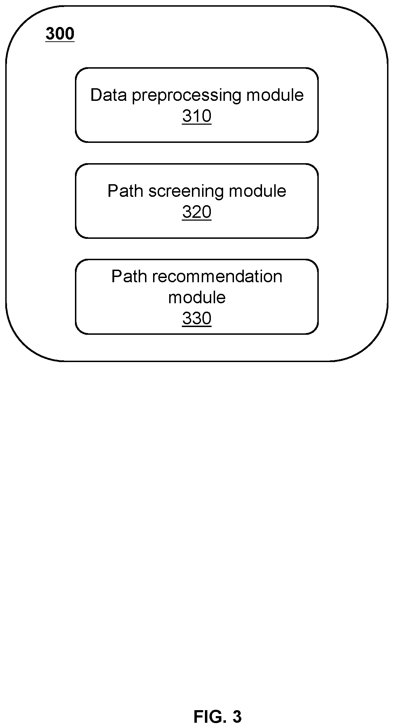

FIG. 3 is a module diagram illustrating an exemplary device for puncture path planning according to some embodiments of the present disclosure.

As shown in FIG. 3, in some embodiments, a device 300 for puncture path planning may include a data preprocessing module 310, a path screening module 320, and a path recommendation module 330. In some embodiments, functions corresponding to the device 300 for puncture path planning device may be implemented by the processing device 130.

The data preprocessing module 310 may be configured to preprocess a target image. In some embodiments, the data preprocessing module 310 may be configured to determine a target point based on the target image. For example, the data preprocessing module 310 may obtain a target structure mask by performing rough segmentation on a target structure in the target image; determine positioning information of the target structure mask based on soft connected domain analysis; and determine the target point by performing precise segmentation on the target structure based on the positioning information of the target structure mask. In some embodiments, the data preprocessing module 310 may be configured to determine a dangerous region. For example, the data preprocessing module 310 may obtain a first segmentation result of the target image based on a first segmentation model; obtain a second segmentation result of the target image based on a second segmentation model; obtain a fusion result by fusing the first segmentation result and the second segmentation result; and determine the dangerous region based on the fusion result.

The path screening module 320 may be configured to determine one or more initial paths and/or candidate paths. In some embodiments, the path screening module 320 may determine the one or more candidate paths based on the target point and at least two constraints. In some embodiments, the at least two constraints may include that: a distance between the path and the dangerous region is greater than a preset distance threshold, the path is located in a slice layer adjacent to a slice layer where the target region is located, a needle entry point on a body contour that contacts a bed board is excluded, a puncture depth of the path is less than a preset depth threshold, or an angle between the path and a vertical line of a flat surface of a flat lesion is within a preset range, etc.

The path recommendation module 330 may be configured to determine a target path based on the one or more candidate paths. In some embodiments, in response to determining that the one or more candidate paths include both a coplanar candidate path and a non-coplanar candidate path, the path recommendation module 330 may determine the target path based on a shortest puncture depth D₁ in the one or more non-coplanar candidate paths, a shortest puncture depth D₂ in the one or more coplanar candidate paths with a small angle deflection perpendicular to a direction of a bed board, and a shortest puncture depth D₃ in the one or more coplanar candidate paths with a non-small angle deflection. In some embodiments, in response to determining that the one or more candidate paths include only the non-coplanar candidate path, the path recommendation module 330 may determine the target path based on the D₁. In some embodiments, in response to determining that the one or more candidate paths include only the coplanar candidate path, the path recommendation module 330 may determine the target path based on the D₂ and the D₃ of the coplanar candidate path.

In some embodiments, the path recommendation module 330 may be configured to recommend the target path. For example, the path recommendation module 330 may transmit a determined target path to the terminal device 140 to output the determined target path to a doctor for selection.

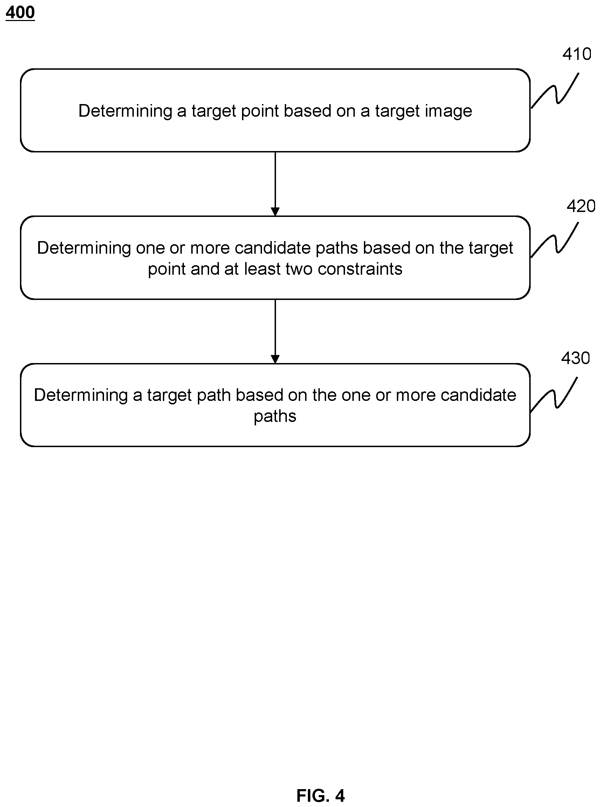

More descriptions regarding the data preprocessing module 310, the path screening module 320, and the path recommendation module 330 may be found elsewhere in present disclosure (e.g., FIGs. 4-28 and related descriptions thereof).

It should be understood that the system and the modules thereof shown in FIG. 3 can be implemented in various ways. For example, in some embodiments, the system and the modules thereof can be implemented by hardware, software, or a combination of software and hardware.

It should be noted that the above description of the device 300 for puncture path planning and the modules thereof is only for convenience of description and used as an illustration, and cannot limit the present disclosure to the scope of the embodiments. It should be understood that for those skilled in the art, after understanding the principle of the system, it is possible to arbitrarily combine the modules or form a subsystem to connect with other modules without deviating from this principle. For example, the data preprocessing module 310 may further include: an image acquisition unit, configured to obtain a target image; an image segmentation unit, configured to perform organ segmentation; a duct recognition unit, configured to recognize a duct and/or a duct type in the target image; and a target point determination unit, configured to determine a target point based on a segmented image or an image after duct recognition. As another example, the path screening module 320 may further include an initial path determination unit and a candidate path determination unit, which may be configured to determine the one or more initial paths based on the target point and a first constraint, and determine the one or more candidate paths from the one or more initial paths based on a second constraint, respectively. Such variations are all within the scope of protection of the present disclosure.

FIG. 4 is a flowchart illustrating an exemplary method for puncture path planning according to some embodiments of the present disclosure. In some embodiments, a process 400 may be performed by the system 100 for puncture path planning (e.g., the processing device 130 of the system 100 for puncture path planning) or the device 300 for puncture path planning. For example, the process 400 may be stored in a storage device (e.g., the storage device 150, and the storage unit of the system) in the form of a program or instruction. The process 400 may be implemented when the processor or the module shown in FIG. 3 executes the program or the instruction. As shown in FIG. 4, in some embodiments, the process 400 may include the following operations.

In 410, a target point may be determined based on a target image. In some embodiments, the operation 410 may be performed by the processing device 130 or the data preprocessing module 310.

The target image refers to an image capable of reflecting a structure, a composition, etc., of an organ and/or tissue in a human body. In some embodiments, the target image may include a medical image generated based on various imaging mechanisms. For example, the target image may be a CT image, an MR image, an ultrasound scan image, an X-ray scan image, an MRI image, a PET image, an OCT image, a NIRS image, an FIR image, an X-ray-MRI image, a PET-X-ray image, a SPECT-MRI image, a DSA-MRI image, a PET-CT image, a US image, etc. In some embodiments, the target image may include a two-dimensional (2D) image, a 3D image, or a four-dimensional (4D) image. The 3D image of an organism may reflect a structure, a density, and other information of an internal tissue and organ of the organism. In some embodiments, the 3D image may be an image that converts a 2D tomographic data sequence obtained by a medical imaging device (e.g., the imaging device 110) into 3D data to intuitively and stereoscopically display 3D morphology, spatial information, or the like, of the organism.

In some embodiments, the target image of a target object may be obtained. In some embodiments, the target image of the target object may be obtained through the imaging device 110. For example, before puncture, the imaging device 110 may scan the target object located in a detection region to obtain the target image, and transmit the target image to the device 300 for puncture path planning or the processing device 130. In some embodiments, the target image of the target object may be obtained from the processing device 130, the terminal device 140, or the storage device 150. In some embodiments, the processing device 130 may obtain the target image of the target object by reading from the storage device 150 and a database, calling a data interface, or the like. In some embodiments, the target image may be obtained in other feasible modes. For example, the target image of the target object may be obtained from a cloud server and/or a medical system (e.g., a medical system center of a hospital, etc.) via the network 160, which is not particularly limited in the embodiments of the present disclosure.

In some embodiments, the target point may reflect an end point of a puncture path. In some embodiments, the target point may be a volume center or a center of gravity of a lesion region (e.g., a diseased organ or tissue) or a region to be detected (e.g., an organ or tissue to be detected). For the convenience of description, the lesion region or the region to be detected is collectively referred to as a "target organ".

In some embodiments, the target point may be determined based on a segmentation result by segmenting (e.g., organ or tissue segmentation) the target image. Different tissues or organs have different grayscales on a scanning image (e.g., CT scanned image). In addition, the organ or tissue has its own shape features or position features, and the organ or tissue segmentation may be achieved based on the features. For example, the lesion region may have a different development (e.g., the lesion tissue generally appears as a low-density development region in a CT plain scan image and generally appears as edge brightening in a CT enhanced image) in the target image from other regions due to a tissue lesion. The segmentation of the lesion region may be achieved based on the development difference in combination with the lesion features.

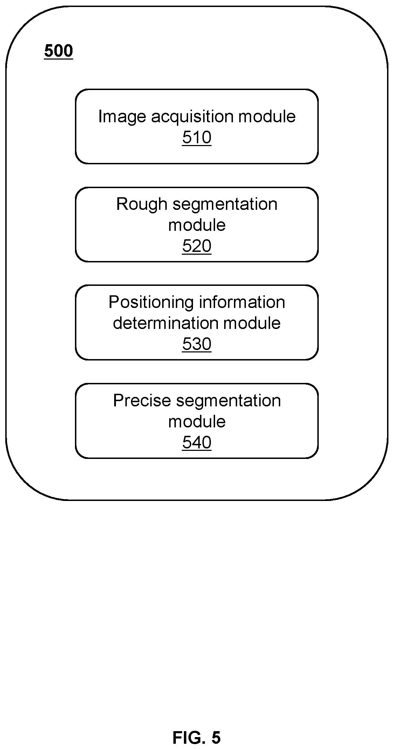

In some embodiments, the organ or tissue segmentation may be performed on the target image by a deep learning model, threshold segmentation, a level set, or other methods. Taking thoracoabdominal puncture as an example, the organ or tissue segmentation may be performed on a thoracoabdominal target image to determine the skin, bones, liver, kidneys, heart, lungs, internal and external blood vessels of the organs, spleen, pancreas, etc. In some embodiments, a target structure mask may be obtained by performing rough segmentation on the target image, and positioning information of the target structure mask may be determined. A segmentation result may be obtained by performing precise segmentation based on the positioning information of the target structure mask. More descriptions regarding obtaining the segmentation result by rough segmentation and precise segmentation may be found in FIGs. 5-15 and related descriptions thereof, which are not repeated here.

In some embodiments, a target image after segmentation and/or a target image of a determined duct type may be displayed on a terminal device (e.g., the terminal device 140) to be output to a user, such that the user can understand the structure and/or lesion information of the organ and/or tissue of the target object.

In 420, one or more candidate paths may be determined based on the target point and at least two constraints. In some embodiments, operation 420 may be performed by the processing device 130 or the path screening module 320.

In some embodiments, the at least two constraints may include, but are not limited to that: a distance between a path and a dangerous region is greater than a preset distance threshold, the path is located in a slice layer adjacent to a slice layer where a target region is located, a needle entry point on a body contour that contacts a bed board is excluded, a puncture depth of the path is less than a preset depth threshold, an angle between the path and a vertical line of a flat surface of a flat lesion is within a preset range, etc.

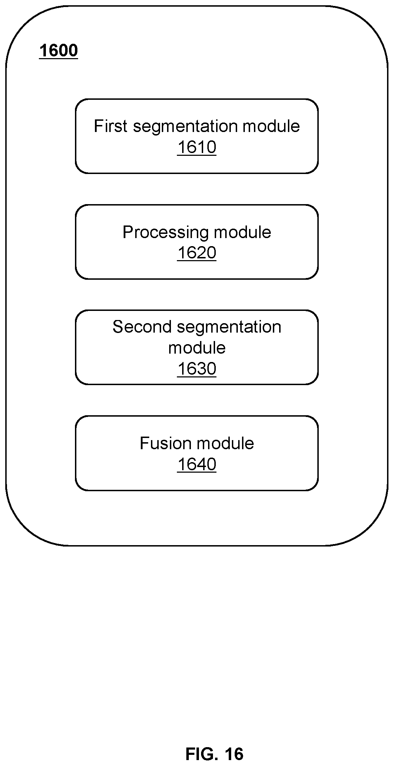

In some embodiments, a duct and/or a duct type in the target image may be recognized, and the dangerous region may be determined based on the duct and/or the duct type. In some embodiments, the processing device 130 may obtain a first segmentation result using a first segmentation model and a second segmentation result of the target image and a second segmentation model, and obtain a fusion result by fusing the first segmentation result and the second segmentation result. Further, the processing device 130 may obtain a first duct skeleton set by performing skeletonization processing on the first segmentation result. The first duct skeleton set may include at least one first duct skeleton of a determined type. The processing device 130 may obtain a second duct skeleton of a duct of an undetermined type by performing the skeletonization processing on the fusion result, and determine a duct type of a second duct skeleton based on the at least one first duct skeleton, thereby determining the dangerous region based on the duct type. More descriptions regarding the duct type may be found in FIGs. 16-23 and related descriptions thereof, which are not be repeated here.

In some embodiments, the one or more candidate paths may be determined based on any two or more of the at least two constraints. In some embodiments, the one or more candidate paths may be determined based on the distance between the path and the dangerous region being greater than the preset distance threshold and any one or more of other constraints. In some embodiments, a type and/or count of the at least two constraints may be determined based on an actual condition. For example, the processing device 130 may determine paths that simultaneously meet the plurality of constraints as the candidate paths.

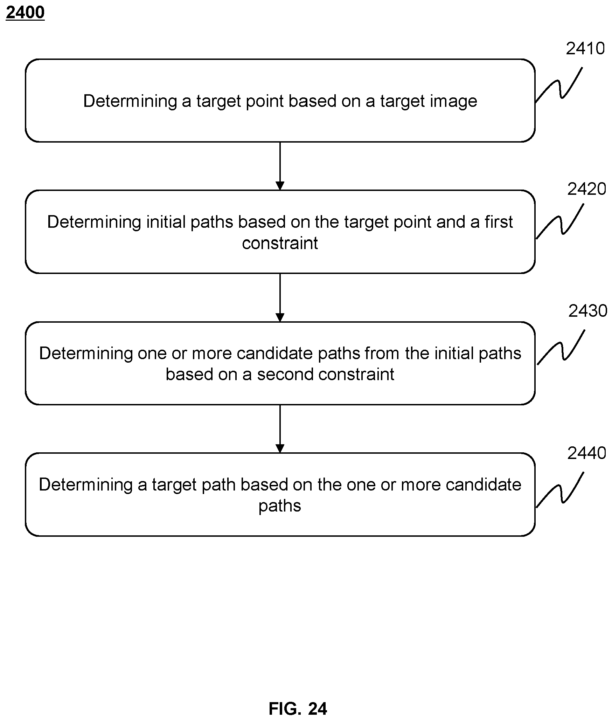

In some embodiments, the initial paths may be determined based on the first constraint, and the one or more candidate paths may be determined from the initial paths based on the second constraint. More descriptions regarding determining the one or more candidate paths may be found in FIG. 24 and related descriptions thereof, which are not repeated here.

In 430, a target path may be determined based on the one or more candidate paths. In some embodiments, the operation 430 may be performed by the processing device 130 or the path recommendation module 330.

In some embodiments, the one or more candidate paths may include one or more coplanar candidate paths and/or one or more non-coplanar candidate paths. A coplanar candidate path refers to a path that is located in the same slice (e.g., the same cross-sectional plane in CT imaging) or several adjacent slices as the target region. A non-coplanar candidate path refers to a path that is not in the same slice or several adjacent slices as the target region. In some embodiments, the target path may be determined based on coplanar and non-coplanar features of the one or more candidate paths. More descriptions regarding determining the target path may be found in FIG. 24 and related descriptions thereof, which are not repeated here.

In some embodiments, after the target path is determined, the target path may be recommended to the user. For example, the processing device 130 may send the target path to the terminal device 140 or the imaging device 110 to output the target path to a doctor for reference. In some embodiments, a puncture operation may be performed based on the target path. For example, the processing device 130 may control the end effector 120 to perform the puncture operation according to the target path. In some embodiments, relevant parameters (e.g., a puncture depth, a puncture angle, the dangerous region, a preset safety distance, a preset depth threshold, a third preset value, a preset range, whether to pass through a thin blood vessel, etc.) of the initial paths, the one or more candidate paths, and/or the target path may be recorded for user reference and/or subsequent determination of the target path.

It should be noted that the above description of the process 400 is only for example and illustration, and does not limit the scope of application of the present disclosure. For those skilled in the art, various modifications and changes can be made to the process 400 under the guidance of the present disclosure. However, such modifications and changes are still within the scope of the present disclosure.

Medical image (e.g., the target image) segmentation (e.g., the organ or tissue segmentation) may be used not only for puncture path planning, but also for medical research, clinical diagnosis, image information processing, etc. In some embodiments, a coarse-to-fine organ segmentation mode may be used. The advantages of this mode are that the accuracy of segmentation can be effectively improved, the occupied hardware resources can be reduced, and the time consumed by segmentation can be reduced. However, the segmentation result of this mode is heavily dependent on the accuracy of rough positioning. In clinical application, situations such as variable organ morphology, small size, lesions, etc. may occur, which may cause inaccurate rough positioning. Inaccurate positioning of rough segmentation may also seriously affect the accuracy of precise segmentation, resulting in poor processing effect of medical image segmentation.

The embodiments of present disclosure provide a method for image segmentation. By using a method of soft connected domain analysis in the rough segmentation stage, the target structure region can be accurately retained while the false positive region can be effectively excluded, which not only improves the accuracy of positioning of the target structure in the rough positioning stage, but also helps the subsequent precise segmentation, thereby improving the segmentation efficiency and accuracy. The method for image segmentation will be described in detail below with reference to the accompanying drawings (e.g., FIGs. 5-15).

FIG. 5 is a module diagram illustrating an exemplary device for image segmentation according to some embodiments of the present disclosure.

As shown in FIG. 5, in some embodiments, a device 500 for image segmentation may include an image acquisition module 510, a rough segmentation module 520, a positioning information determination module 530, and a precise segmentation module 540. In some embodiments, functions corresponding to the image segmentation device 500 may be implemented by the processing device 130 or the device 300 for puncture path planning (e.g., the data preprocessing module 310).

The image acquisition module 510 may be configured to obtain a target image. In some embodiments, the target image may include a 2D image, a 3D image, or a 4D image. In some embodiments, the image acquisition module 510 may be configured to obtain the target image of a target object.

The rough segmentation module 520 may be configured to obtain a target structure mask by performing rough segmentation on a target structure in the target image. In some embodiments, the rough segmentation module 520 may be configured to obtain at least one target structure mask by performing rough segmentation on at least one target structure in the target image.

The positioning information determination module 530 may be configured to determine positioning information of the target structure mask based on soft connected domain analysis. In some embodiments, the positioning information determination module 530 may be configured to determine a count of connected domains in the target structure mask, and determine the positioning information of the target structure mask based on the count of connected domains. In some embodiments, the positioning information determination module 530 may be configured to locate the target structure mask based on positioning coordinates of a preset structure.

The precise segmentation module 540 may be configured to perform precise segmentation on the target structure based on the positioning information of the target structure mask. In some embodiments, the precise segmentation module 540 may be configured to obtain a preliminary precise segmentation result by performing preliminary precise segmentation on the target structure; determine whether the positioning information of the target structure mask is accurate based on the preliminary precise segmentation result; if the positioning information of the target structure mask is accurate, using the preliminary precise segmentation result as a target segmentation result; or if the positioning information of the target structure mask is not accurate, determine a target segmentation result of the target structure by an adaptive sliding window mode.

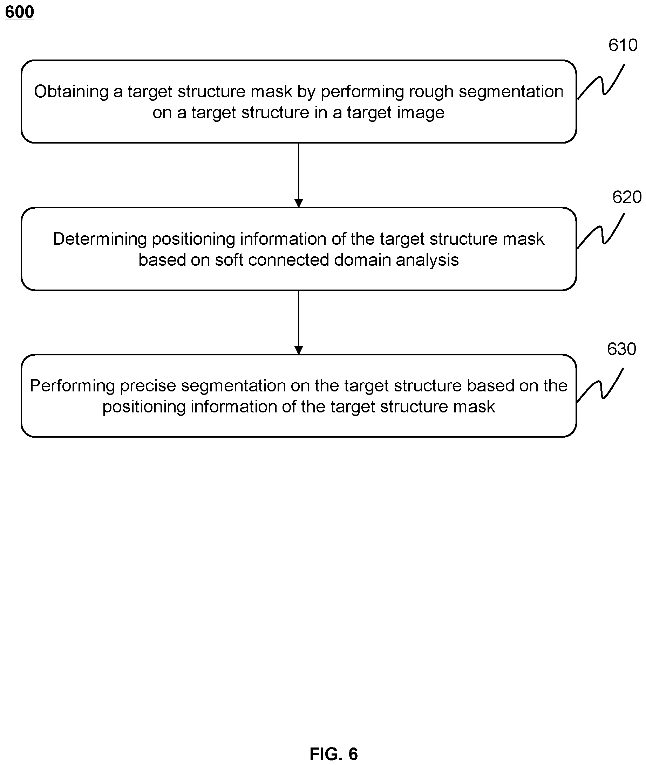

It should be noted that for more technical details about the rough segmentation module 520, the positioning information determination module 530, and the precise segmentation module 540 performing corresponding processes or functions to achieve organ segmentation may be found in the related descriptions of the method for image segmentation described in any embodiment shown in FIGs. 6-15, which are not repeated here.

The above description regarding the image segmentation device 500 is only for illustrative purposes and is not intended to limit the scope of the present disclosure. For those having ordinary skilled in the art, various forms and details of improvements and changes can be made to the application of the above method and system without departing from the principle of the present disclosure. However, such changes and modifications will not depart from the scope of the present disclosure.

FIG. 6 is a flowchart illustrating an exemplary process for image segmentation according to some embodiments of the present disclosure. In some embodiments, a process 600 may be performed by the system 100 for puncture path planning (e.g., the processing device 130 of the system 100 for puncture path planning) or the device 500 for image segmentation. For example, the process 600 may be stored in a storage device (e.g., the storage device 150, and the storage unit of the system) in the form of a program or instruction. The process 600 may be implemented when the processor or the module shown in FIG. 5 executes the program or instruction. As shown in FIG. 6, in some embodiments, the process 600 may include the following operations.

In 610, a target structure mask may be obtained by performing rough segmentation on a target structure in a target image. In some embodiments, the operation 610 may be performed by the processing device 130 or the rough segmentation module 520.

The target structure refers to a target organ and/or organ tissue for segmentation, such as a target organ, a blood vessel in the target organ, etc. In some embodiments, the target image may include one or more target structures. In some embodiments, the target structure may include the heart, a liver, the spleen, the kidneys, blood vessels, and/or any other possible organ or organ tissue.

The target structure mask (or Mask) refers to an image including pixel values representing pixel-level classification labels. Taking an abdominal target image as an example, the target structure mask represents classification of each pixel in the target image. For example, the target image may be divided into a background, the liver, the spleen, the kidneys, etc. A summary region of a specific class may be represented by a corresponding label value (e.g., all pixels classified as the liver may be summarized, and the summary region may be represented by a label value corresponding to the liver). The label value may be set according to a specific rough segmentation task. In some embodiments, the target structure mask obtained by the rough segmentation may be a relatively rough organ mask. The target structure mask obtained by the rough segmentation is also referred to as a first mask.

In some embodiments, the target image may be preprocessed, and the target structure mask may be obtained by performing rough segmentation on at least one target structure in a preprocessed target image. For example, the preprocessing may include normalization processing and/or background removal processing.

In some embodiments, the rough segmentation may be performed on the at least one target structure in the target image using a threshold segmentation method, a regional growth method, or a level set method. For example, the processing device 130 may classify all pixels in the target image by setting a plurality of different pixel threshold ranges according to an input pixel value of the target image, and divide pixel points with pixel values within the same pixel threshold range into the same region, thereby achieving rough segmentation of the target image. As another example, the processing device 130 may preset a similarity determination condition based on known pixels on the target image or a preset region composed of the pixel points according to needs, and compare the pixel points with surrounding pixel points or compare the preset region with surrounding regions based on the preset similarity determination condition, merge pixel points or regions with high similarity, and stop merging until the above process cannot be repeated to complete the rough segmentation process, thereby achieving rough segmentation of the target image. The preset similarity determination condition may be determined according to preset image features, such as a grayscale, a texture, or other image features. As another example, the processing device 130 may set a target contour of the target image as a zero level set of a high-dimensional function, differentiate the function, obtain the target contour by extracting the zero level set from the output, and then segment out a pixel region within the contour to achieve rough segmentation of the target image.

In some embodiments, the rough segmentation may be performed on the at least one target structure in the target image using a trained deep learning model (e.g., UNet). For example, after the target image is input into a trained convolutional neural network (CNN), an encoder of the CNN may perform feature extraction on the target image through convolution, and then a decoder of the CNN may restore features into a pixel-level segmentation probability map, which represents a probability that each pixel in the image belongs to a specific class. Finally, the segmentation probability map may be output as a segmentation mask, thereby completing the rough segmentation.

In 620, positioning information of the target structure mask may be determined based on soft connected domain analysis. In some embodiments, the operation 620 may be performed by the processing device 130 or the positioning information determination module 530.

A connected domain (i.e., a connected region) refers to an image region of the target image composed of foreground pixel points having the same pixel value and adjacent to each other. In some embodiments, the target structure mask may include one or more connected domains.

In some embodiments, the positioning information (also referred to as first positioning information) of the target structure mask may be determined by performing the soft connected domain analysis on the target structure mask. The soft connected domain analysis refers to analyzing and calculating a count of connected domains in the target structure mask and areas corresponding to the connected domains.

In some embodiments, the count of connected domains in the target structure mask may be determined, and the positioning information of the target structure mask may be determined based on the count of connected domains. In some embodiments, when the target image includes a plurality of connected domains, position information of the plurality of connected domains may be determined first, and then the positioning information of the target structure mask may be obtained based on the position information of the plurality of connected domains. In some embodiments, retained connected domains may be determined based on the count of connected domains, and the positioning information of the target structure mask may be determined based on the position information of the retained connected domains.

In some embodiments, when the count of connected domains is greater than a first preset value, the processing device 130 may determine connected domains satisfying a set condition as the retained connected domains. In some embodiments, the set conditions may be a limiting condition on areas of the connected domains. In some embodiments, when the count of connected domains is less than or equal to the first preset value, all the connected domains may be determined as the retained connected domains (e.g., the count of connected domains is 1) or the output retained connected domains may be null (e.g., the count of connected domains is 0).

In some embodiments, when the count of connected domains is greater than the first preset value, whether all or part of the plurality of connected domains (e.g., connected domains of which areas are within a preset order n) are the retained connected domains may be determined.

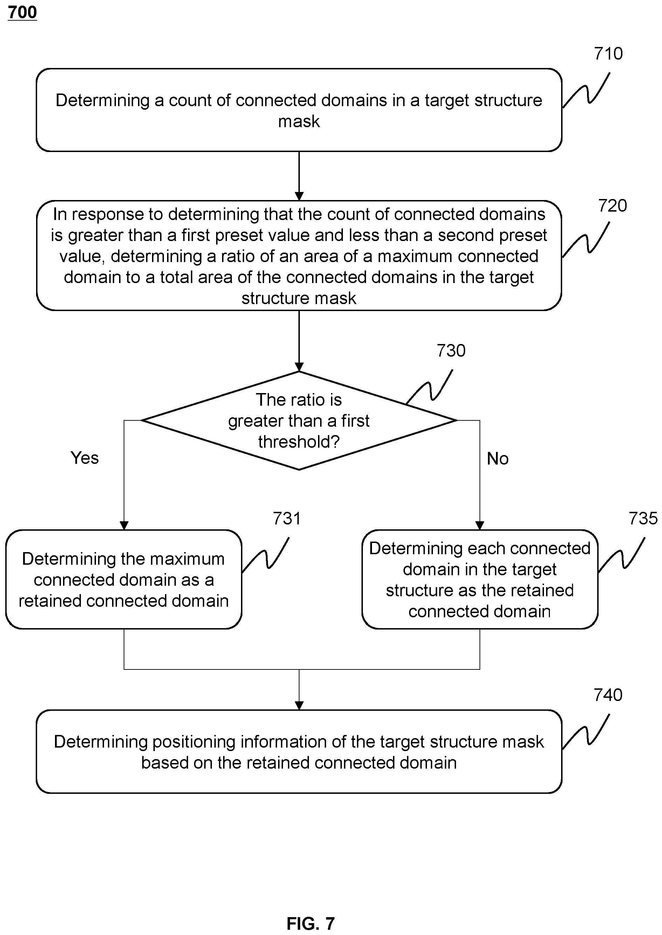

In some embodiments, when the count of connected domains is greater than the first preset value and less than a second preset value, a ratio of an area of a maximum connected domain in the target structure mask to a total area of the connected domains may be determined; whether the ratio is greater than a first threshold may be determined; in response to determining that the ratio is greater than the first threshold, the maximum connected domain may be determined as the retained connected domain; in response to determining that the ratio is not greater than the first threshold, each connected domain in the target structure mask may be determined as the retained connected domain. The maximum connected domain refers to a connected domain with the largest area in the target structure mask. The total area of the connected domains refers to a sum of the areas of all the connected domains in the target structure mask. More descriptions may be found in FIG. 7 and related descriptions thereof, which are not repeated here.

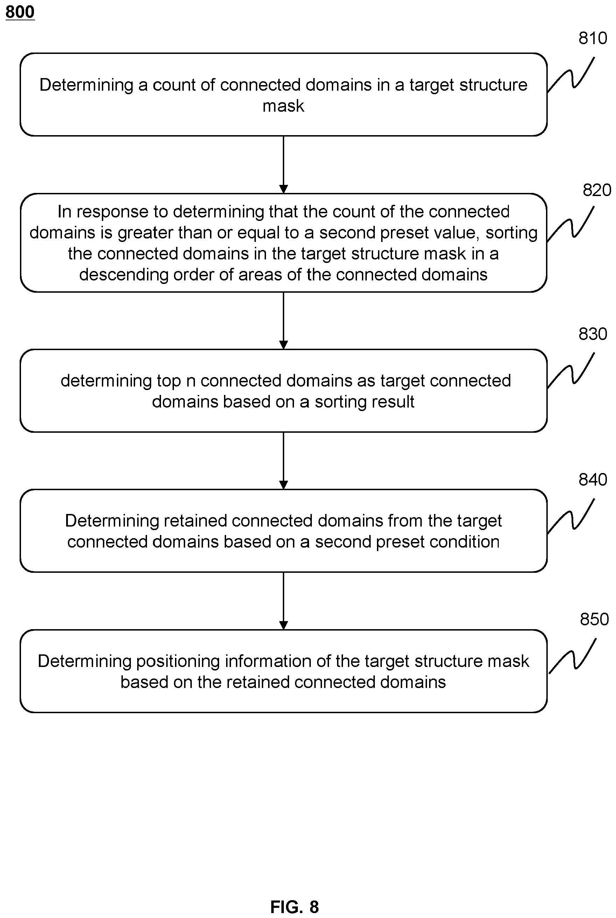

In some embodiments, when the count of connected domains is greater than or equal to the second preset value, each connected domain in the target structure mask may be sorted in a descending order of area; connected domains ranked in the top n (i.e., the preset order n) may be determined as target connected domains based on a sorting result; and the retained connected domains may be determined from the target connected domains based on a second preset condition. For example, the processing device 130 may sort the plurality of connected domains with different areas in a descending order of area, and denote the sorted connected domains as a first connected domain, a second connected domain, ..., and a kth connected domain. The first connected domain refers to a connected domain with the largest area of the plurality of connected domains, and is also referred to the maximum connected domain. When the preset order n is 3, i.e., the target connected domains are the first connected domain, the second connected domain, and a third connected domain, the processing device 130 may determine whether one or more of the first connected domain, the second connected domain, and the third connected domain are the retained connected domains in order of areas based on the second preset condition. That is, whether the first connected domain is a retained connected domain may be determined first, then whether the second connected domain is a retained connected domain may be determined, until the determination of an (n-1)th connected domain is completed. More descriptions may be found in FIG. 8 and related descriptions thereof, which are not repeated here.

It can be understood that when the count of connected domains is within different ranges or satisfies different threshold conditions (e.g., the first preset value, and the second preset value), the set condition for determining connected domains of different areas in order as the retained connected domains may be different. More descriptions may be found in the related descriptions of FIGs. 7-8.

In 630, precise segmentation may be performed on the target structure based on the positioning information of the target structure mask. In some embodiments, the operation 630 may be performed by the processing device 130 or the precise segmentation module 540.

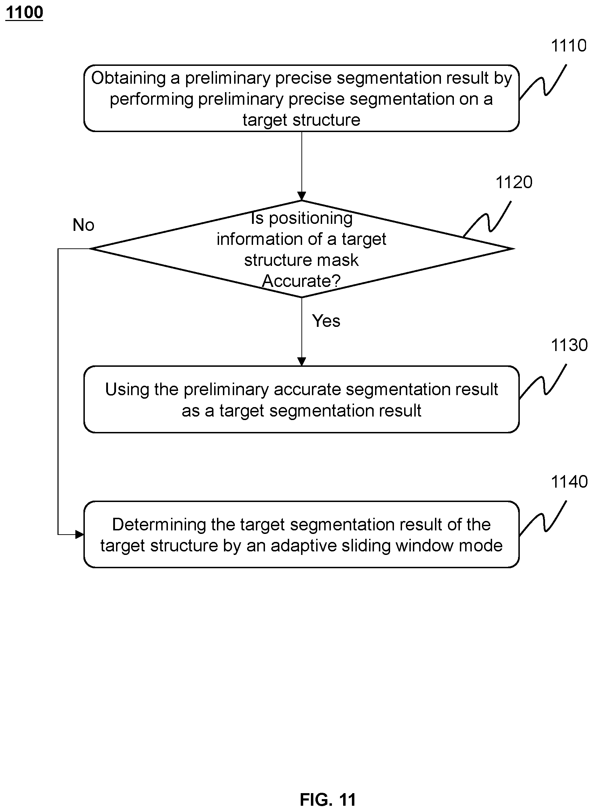

In some embodiments, the precise segmentation may include performing preliminary precise segmentation on the target structure, and determining whether the positioning information of the target structure mask is accurate based on a preliminary precise segmentation result; if the positioning information of the target structure mask is accurate, using the preliminary precise segmentation result as a target segmentation result; if the positioning information of the target structure mask is not accurate, determining the target segmentation result of the target structure by an adaptive sliding window mode. More descriptions may be found in FIG. 11 and related descriptions thereof, which are not repeated here.

It should be noted that the above description of the process 600 is only for example and illustration, and does not limit the scope of application of the present disclosure. For those skilled in the art, various modifications and changes can be made to the process 600 under the guidance of the present disclosure. However, such modifications and changes are still within the scope of the present disclosure.

FIG. 7 is a flowchart illustrating an exemplary process of determining positioning information of a target structure mask according to some embodiments of the present disclosure. In some embodiments, a process 700 may be performed by the system 100 for puncture path planning (e.g., the processing device 130 of the system 100 for puncture path planning) or the image segmentation device 500 (e.g., the positioning information determination module 530). For example, the process 700 may be stored in a storage device (e.g., the storage device 150, and the storage unit of the system) in the form of a program or instruction. The process 700 may be implemented when the processor or the module shown in FIG. 5 executes the program or instruction. As shown in FIG. 7, in some embodiments, the process 700 may include the following operations.

In 710, a count of connected domains in a target structure mask may be determined.

In some embodiments, a plurality of connected domains in the target structure mask may have different areas. In some embodiments, the count of connected domains in the target structure mask may be determined in any feasible way, which is not limited to the present disclosure.

In 720, in response to determining that the count of connected domains is greater than a first preset value and less than a second preset value, a ratio of an area of a maximum connected domain to a total area of the connected domains in the target structure mask may be determined.

In some embodiments, a first preset value may be 1.

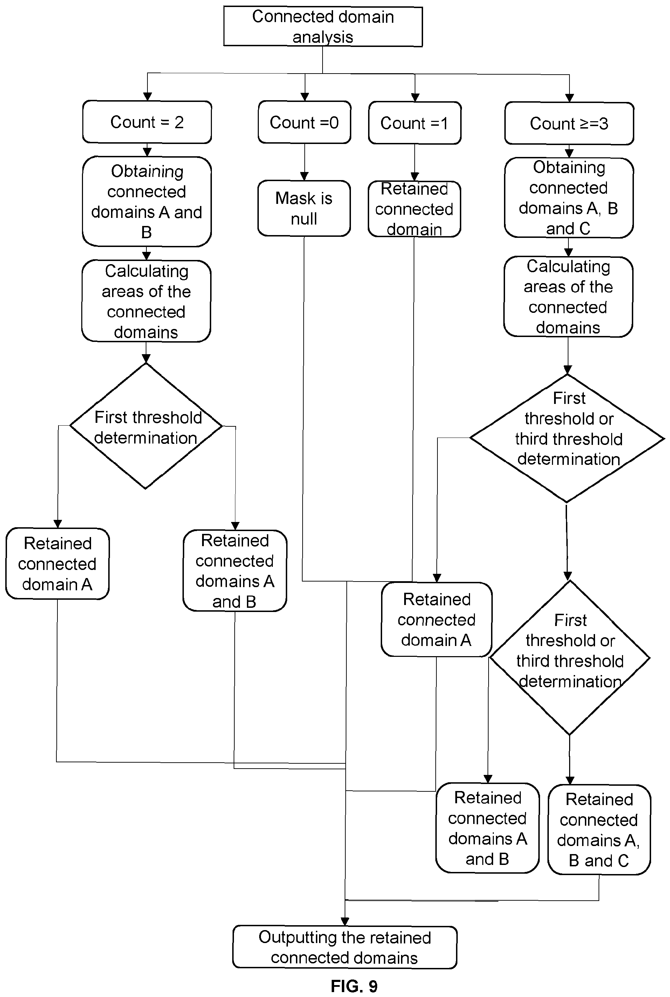

In some embodiments, as shown in FIG. 9, when the count of connected domains is 0, it indicates that the corresponding mask may be null, that is, mask acquisition of the target structure fails, or the rough segmentation fails, or a segmentation object does not exist. For example, when the spleen in an abdominal cavity is segmented, a situation of splenectomy may exist. At this time, the mask of the spleen is empty and the number of connected domains is 0. In this case, an output retained connected domain may be null. When the count of connected domains is 1, it indicates that only one connected domain exists, and no false positive or segmentation disconnection exists. In this case, the connected domain may be retained, i.e., the connected domain may be determined as the retained connected domain. It is understood that when the count of connected domains is 0 and 1, whether the connected domain is the retained connected domain may not need to be determined according to the set condition.

In some embodiments, when the count of connected domains is greater than the first preset value and less than the second preset value, the positioning information of the target structure mask may be determined through the operations 730-740. In some embodiments, the second preset value may be 3. For example, when the count of connected domains of the target structure mask is greater than 1 and less than 3 (e.g., the count of connected domains is 2), the processing device 130 may determine the ratio of the area of the maximum connected domain to the total area of the connected domains in the target structure mask.

When the count of connected domains is greater than or equal to the second preset value, the positioning information of the target structure mask may be determined through operations in a process 800. More descriptions may be found in operations 820-840, which are not repeated here.

In 730, whether the ratio of the area of the maximum connected domain to the total area of the connected domains is greater than a first threshold may be determined.

In some embodiments, a value of the first threshold may be within a range of 0.8-0.95. The value of the first threshold may be within the range of 0.8-0.95, which can ensure that soft connected domain analysis has an expected accuracy. In some embodiments, the value of the first threshold may be within a range of 0.9-0.95. The value of the first threshold may be within the range of 0.9-0.95, which can further improve the accuracy of the soft connected domain analysis. In some embodiments, the first threshold may be set based on a category (e.g., a chest target structure, and an abdominal target structure) of the target structure. In some embodiments, the first threshold may be reasonably set based on machine learning and/or big data, which is not limited here.

If the ratio of the area of the maximum connected domain to the total area of the connected domains in the target structure mask is greater than the first threshold, an operation 731 may be performed: the maximum connected domain may be determined as the retained connected domain. in response to determining that the ratio of the area of the maximum connected domain to the total area of the connected domains in the target structure mask is not greater than the first threshold, an operation 735 may be performed: each connected domain in the target structure mask may be determined as the retained connected domain.

Merely by way of example, as shown in FIG. 9, when the count of connected domains in the target structure mask is greater than 1 and less than 3 (i.e., 2), the processing device 130 may obtain connected domains A and B, respectively, according to a size of an area (S), wherein an area of the connected domain A may be greater than an area of the connected domain B, i.e., S(A)>S(B). With reference to the description above, the connected domain A may also be referred to as the first connected domain or the maximum connected domain; the connected domain B may be referred to as the second connected domain. By calculating the connected domains, when a ratio of the area of the connected domain A to a total area of the connected domains A and B is greater than the first threshold, i.e., S(A)/S(A+B) > the first threshold, the connected domain B may be determined as a false positive region, and only the connected domain A may be retained, i.e., the maximum connected domain A may be determined as the retained connected domain. When the ratio of the area of the connected domain A to the total area of the connected domains A and B is less than or equal to the first threshold, both the connected domains A and B may be determined as a part of the target structure mask, and the connected domains A and B may be retained simultaneously, i.e., the connected domains A and B may be determined as the retained connected domain.

In 740, positioning information of the target structure mask may be determined based on retained connected domains.

In some embodiments, the positioning information of the target structure mask may include position information of a bounding rectangle of the target structure mask, such as coordinate information of a border line of the bounding rectangle. In some embodiments, the bounding rectangle of the target structure mask may cover a positioning region of the target structure. In some embodiments, the bounding rectangle of the target structure mask may be displayed in the target image in the form of a bounding rectangle frame. In some embodiments, the bounding rectangle frame relative to the target structure mask may be constructed based on bottom edges (e.g., the bottom edges of the connected regions in upper, lower, left, and right directions) of the connected regions in the target structure in various directions.

In some embodiments, the bounding rectangle of the target structure mask may include a bounding rectangle frame having only one rectangle frame. For example, when there is only one connected region (e.g., a blood vessel or an organ in the abdominal cavity) in the target structure (e.g., an organ), a bounding rectangle with a relatively large area may be constructed based on the bottom edges of the connected region in all directions. In some embodiments, the bounding rectangle with a relatively large area may be applied to an organ having one connected region.

In some embodiments, the bounding rectangle of the target structure mask may include a bounding rectangle formed by combining and splicing a plurality of rectangle frames. For example, when there are a plurality of connected domains in an organ, the plurality of connected domains may correspond to a plurality of rectangle frames, and the bounding rectangle with the relatively large area may be constructed according to the bottom edges of the plurality of rectangle frames. When the bounding rectangle of the target structure mask is formed by combining and splicing a plurality of small rectangle frames (e.g., the bottom edges of three rectangle frames corresponding to three connected domains form a total bounding rectangle frame), the calculation may be processed according to the total bounding rectangle frame, thereby reducing the amount of calculation while ensuring the expected accuracy.

In some embodiments, when the bounding rectangle of the target structure mask fails to be positioned, the target structure mask may be located based on positioning coordinates of a preset structure. It is understood that when the coordinates of the bounding rectangle of the target structure mask do not exist, it is determined that the corresponding organ fails to be positioned.

In some embodiments, the preset structure may select a target structure (e.g., an organ with relatively stable positioning) with relatively stable positioning. A probability of positioning failure during positioning such target structure may be low, thereby achieving accurate positioning of the target structure mask. For example, since the probability of positioning failure of the liver, stomach, spleen, and kidneys in the abdominal cavity is low, and the probability of positioning failure of the lung in the thoracic cavity is low, i.e., the positioning of these organs is relatively stable, the liver, stomach, spleen, and kidneys may be used as preset organs in the abdominal cavity. That is, the preset structure may include the liver, stomach, spleen, kidneys, lung, or any other possible organ tissue.

In some embodiments, the target structure mask may be repositioned using positioning coordinates of the preset structure as reference coordinates. For example, when the target structure that fails to be positioned is located in the abdominal cavity, the positioning coordinates of the liver, stomach, spleen, and kidney may be used as coordinates for repositioning, and the target structure that fails to be positioned in the abdominal cavity may be repositioned accordingly. In some embodiments, the target structure mask in a thoracic cavity may be positioned based on the positioning coordinates of the lung. For example, when the target structure that fails to be positioned is located in the thoracic cavity, the positioning coordinates of the lung may be used as the coordinates for repositioning, and the target structure that fails to be positioned in the thoracic cavity may be repositioned accordingly.

Merely by way of example, when the target structure that fails to be positioned is located in the abdominal cavity, positioning coordinates of liver top, kidney bottom, spleen left, and liver right may be used as coordinates for repositioning in a cross-sectional direction (upper and lower sides) and a coronal direction (left and right sides), and a frontmost end and a rearmost end of the coordinates of the liver, the kidney, the spleen, and the liver may be used as coordinates for new positioning in a sagittal direction (front and back sides). In this way, the target structure that fails to be positioned in the abdominal cavity may be repositioned. Merely by way of example, when the target structure that fails to be positioned is located in the thoracic cavity, a bounding rectangle frame formed by the positioning coordinates of the lung may be expanded by a certain count of pixels, and the target structure that fails to be positioned in the thoracic cavity may be repositioned accordingly.

The positioning information of the target structure can be determined by accurately positioning the target structure mask based on the positioning coordinates of the preset structure, such that the segmentation accuracy and efficiency can be improved while reducing the amount of segmentation calculation, thereby saving memory resources.

In some embodiments, determining the positioning information of the target structure mask may further include the following operations: reducing noise and optimizing the image display effect by post-processing the target structure mask. For example, the post-processing may include the following image post-processing operations: edge smoothing and/or image denoising, etc. In some embodiments, edge smoothing may include smoothing or blurring to reduce noise or distortion of a medical image. In some embodiments, smoothing or blurring may be performed in the following ways: mean filtering, median filtering, Gaussian filtering, and bilateral filtering.

It should be noted that the above description of the process 700 is only for example and explanation, and does not limit the scope of application of the present disclosure. For those skilled in the art, various modifications and changes can be made to the process 700 under the guidance of the present disclosure. However, such modifications and changes are still within the scope of the present disclosure.

FIG. 8 is a flowchart illustrating an exemplary process of determining positioning information of a target structure mask according to some embodiments of the present disclosure. In some embodiments, a process 800 may be performed by the system 100 for puncture path planning (e.g., the processing device 130 of the system 100 for puncture path planning) or the image segmentation device 500 (e.g., the positioning information determination module 530). For example, the process 800 may be stored in a storage device (e.g., the storage device 150, and the storage unit of the system) in the form of a program or instruction. The process 800 may be implemented when the processor or the module shown in FIG. 5 executes the program or instruction. As shown in FIG8, in some embodiments, the process 800 may include the following operations.

In 810, a count of connected domains in a target structure mask may be determined. More descriptions may be found in the operation 710 and related descriptions thereof.

In 820, in response to determining that the count of the connected domains is greater than or equal to a second preset value, the connected domains in the target structure mask may be sorted in a descending order of the areas of the connected domains.

According to the description above, the second preset value may be 3. When the count of connected domains is greater than or equal to 3, the processing device 130 may sort the connected domain in the target structure mask in a descending order of the areas of the connected domains.

In 830, top n connected domains may be determined as target connected domains based on a sorting result.

In some embodiments, the processing device 130 may determine the top n (e.g., 3) connected domains as the target connected domains based on the sorting result. In some embodiments, the preset order n may be set based on a category of a target structure (e.g., a chest target structure, and abdominal target structure). In some embodiments, the preset order n may be reasonably set based on machine learning and/or big data, which is not limited here.

In 840: retained connected domains may be determined from the target connected domains based on a second preset condition.

In some embodiments, whether connected domains (or the connected domains in the target structure mask) of which area orders are in the preset order n are the retained connected domains may be determined based on an order of areas of the connected domains according to the second preset condition, and finally the retained connected domains may be output.

The second preset condition refers to a limiting condition related to the areas of the connected domains.

In some embodiments, the second preset condition may include a relationship between a ratio of an area of a specific connected domain (e.g., the maximum connected domain, or a connected domain of which an area order is within a preset order m, m being less than or equal to n) to the total area of the connected domains and a threshold (e.g., the first threshold). For example, if the maximum connected domain in the preset order n needs to be determined as the retained connected domain, the condition to be satisfied may be that the ratio of the area of the maximum connected domain to the total area of the connected domains is greater than the first threshold. As another example, if the second connected domain (the second connected domain in sorting) in the preset order n needs to be determined as the retained connected domain, the condition to be satisfied may be that when a ratio of a sum of the area of the first connected domain (i.e., the maximum connected domain) and an area of the second connected domain (i.e., the area of the specific connected domain) to the total area of the connected domains is greater the first threshold, both the first connected domain and the second connected domain may be determined as the retained connected domains. As another example, if a third connected domain (the third connected domain in ranking) in the preset order n needs be determined as the retained connected domain, the condition to be satisfied may be that when a ratio of a sum of areas of the first connected domain, the second connected domain, and the third connected domain (i.e., the area of the specific connected domain) to the total area of the connected domains is greater than the first threshold, the first connected domain, the second connected domain, and the third connected domain may all be determined as the retained connected domains.

In some embodiments, the second preset condition may include a relationship between the ratio of the area of the first preset connected domain to the area of the second preset connected domain and a fifth threshold. For example, if the maximum connected domain in the preset order n needs to be determined as the retained connected domain, the condition to be satisfied may be that a ratio of the area of the second connected domain (i.e., a first preset connected domain) to the area of the maximum connected domain (i.e., a second preset connected domain) is greater than the fifth threshold. As another example, if the second connected domain in the preset order n needs to be determined as the retained connected domain, the condition to be satisfied may be that a ratio of the area of the third connected domain (i.e., the area of the first preset connected domain) to a sum of the area of the first connected domain and the area of the second connected domain (i.e., the area of the second preset connected domain) is less than the fifth threshold. As another example, if the third connected domain in the preset order n needs to be determined as the retained connected domain, the condition to be satisfied may be that when a ratio of an area of a fourth connected domain in order (i.e., the area of the first preset connected domain) to a sum of the areas of the first connected domain, the second connected domain, and the third connected domain (i.e., the area of the second preset connected domain) is less than the fifth threshold, the first connected domain, the second connected domain, and the third connected domain may also be determined as the retained connected domains.

In some embodiments, the fifth threshold may be within a range of 0.05-0.2, to ensure that the soft connected domain analysis obtains the expected accuracy. In some embodiments, the fifth threshold may be 0.05. In this case, a relatively good accuracy effect of the soft connected domain analysis can be obtained. In some embodiments, the fifth threshold may be other reasonable values, which are not limited in the present disclosure.

Merely by way of example, as shown in FIG. 9, when the count of connected domains in the target structure mask is greater than or equal to 3, the processing device 130 may obtain connected domains A, B, C, ..., P, respectively, according to the areas (S) thereof. The area of the connected domain A may be greater than the area of the connected domain B, the area of the connected domain B may be greater than the area of the connected domain C, and so on, i.e., S(A)>S(B)>S(C)>...>S(P). Further, the processing device 130 may calculate a total area S(T) of the connected domains A, B, C, ..., P to calculate the connected domains. Specifically, the processing device 130 may select connected domains (e.g., connected domains A, B, C) in the preset order n according to the order of areas of the connected domains, and sequentially determine whether each of the connected domains in the preset order n is the retained connected domain. When a ratio of the area of the connected domain A to the total area S(T) is greater than a first threshold M, i.e., S(A)/S(T)>M, or a ratio of the area of the connected domain B to the area of the connected domain A is less than a fifth threshold N, i.e., S(B)/S(A)<N, the connected domain A may be determined as a part of an organ mask and retained (i.e., the connected domain A may be determined as the retained connected domain), and the remaining connected domains may be determined as false positive regions; otherwise, the calculation continues, i.e., it continues to determine whether the second connected domain (i.e., the connected domain B) is the retained connected domain. When a ratio of the area of connected domain A and connected domain B to the total area S(T) is greater than the first threshold M, i.e., S(A+B)/S(T)>M, or a ratio of the area of the connected domain C to the areas of the connected domain A and the connected domain B is less than the fifth threshold N, i.e., S(C)/S(A+B)<N, the connected domains A and B may be determined as a part of the target structure mask and retained (i.e., the connected domain A and the connected domain B may be determined as the retained connected domains), and the remaining parts may all be determined as the false positive regions; otherwise, the calculation continues, i.e., it continues to determine whether the third connected domain (i.e., the connected domain C) is the retained connected domain. When a ratio of the areas of the connected domain A, the connected domain B, and the connected domain C to the total area S(T) is greater than the first threshold M, i.e., S(A+B+C)/S(T)>M, or a ratio of the area of the connected domain D (the fourth connected domain) to the areas of the connected domain A, the connected domain B and the connected domain C is less than the fifth threshold N, i.e., S(D)/S(A+B+C)<N, the connected domains A, B and C may all be determined as a part of the target structure mask and retained (i.e., the connected domain A, the connected domain B and the connected domain C may all be determined as retained connected domains). Referring to the above determination method, whether the connected domains A, B, C, D, ..., P in the target structure mask, or the part of the connected domains of which the area order is in the preset order n are the retained connected domains can be determined in turn.

It should be noted that only the determination of whether the three connected domains are the retained connected domains is shown in FIG. 9. It can also be understood that the value of the preset order n in FIG. 9 may be set to 4, so it is only necessary to determine whether the top 3 connected domains (e.g., the connected domain A, the connected domain B, and the connected domain C) are the retained connected domains.

In 850, positioning information of the target structure mask may be determined based on the retained connected domains. More descriptions may be found in the operation 740 and related descriptions thereof.

It should be noted that the above description of the process 800 is only for example and explanation, and does not limit the scope of application of the present disclosure. For those skilled in the art, various modifications and changes can be made to the process 800 under the guidance of the present disclosure. However, such modifications and changes are still within the scope of the present disclosure.

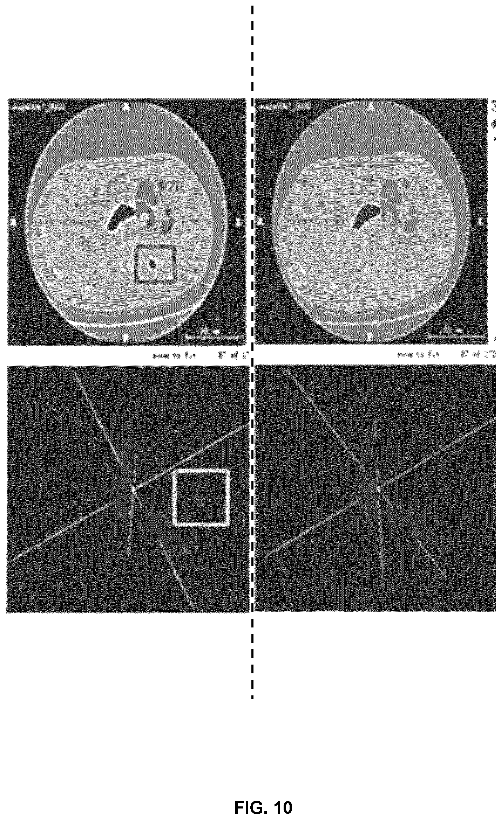

FIG. 10 is a schematic diagram illustrating a comparison of exemplary rough segmentation results according to some embodiments of the present disclosure. As shown in FIG. 10, the upper and lower figures on the left side of the dotted line illustrate a cross-sectional target image and a stereoscopic target image of a rough segmentation result without using soft connected domain analysis, and the right side of the dotted line illustrates a cross-sectional target image and a stereoscopic target image of the rough segmentation result using the soft connected domain analysis. By comparison, it can be seen that the result of the rough segmentation of the target structure mask based on the soft connected domain analysis shows that the false positive region framed by the box in the left image is removed. Compared with the previous connected domain analysis method, the accuracy and reliability of excluding the false positive regions are higher, directly contributing to the subsequent reasonable extraction of the bounding frame of the positioning information of the target structure mask, thereby improving the segmentation efficiency.

FIG. 11 is a flowchart illustrating an exemplary precise segmentation process according to some embodiments of the present disclosure. In some embodiments, a process 1100 may be performed by the system 100 for puncture path planning (e.g., the processing device 130 of the system 100 for puncture path planning) or the image segmentation device 500 (e.g., the precise segmentation module 540). For example, the process 1100 may be stored in a storage device (e.g., the storage device 150, and the storage unit of the system) in the form of a program or instruction. The process 1100 may be implemented when the processor or the module shown in FIG. 5 executes the program or instruction. As shown in FIG. 11, in some embodiments, the process 1100 may include the following operations.

In 1110, a preliminary precise segmentation result may be obtained by performing preliminary precise segmentation on a target structure.

The preliminary precise segmentation refers to precise segmentation performed based on positioning information of a target structure mask after rough segmentation.

In some embodiments, the preliminary precise segmentation result may be obtained by performing the preliminary precise segmentation on the target structure based on a bounding rectangle frame located by the rough segmentation. A more precise mask of the target structure may be generated through the preliminary precise segmentation, i.e., the preliminary precise segmentation result may include a target structure mask after the precise segmentation. The target structure mask obtained through the precise segmentation is also referred to as a second mask.

In 1120, whether positioning information of a target structure mask is accurate may be determined.

Through the operation 1120, whether the positioning information of the target structure mask obtained by the rough segmentation is accurate may be determined, i.e., whether the first positioning information determined based on soft connected domain analysis is accurate may be determined, thereby determining whether the rough segmentation is accurate.

In some embodiments, whether the positioning information of the target structure mask after the rough segmentation is accurate may be determined according to the positioning information of the target structure mask after the preliminary precise segmentation. In some embodiments, second positioning information (i.e., positioning information of the preliminary precise segmentation result) may be obtained by calculating the second mask, and the positioning information (the first positioning information) of the rough segmentation may be compared with the positioning information (second positioning information) of the precise segmentation to determine whether the first positioning information of the first mask (i.e., the target structure mask after the rough segmentation) is accurate. In some embodiments, the preliminary precise segmentation result may include the second mask and/or the positioning information of the second mask.

In some embodiments, a difference between the bounding rectangle of the target structure mask after the rough segmentation and the bounding rectangle of the target structure mask after the precise segmentation may be determined by comparing the bounding rectangle of the target structure mask after the rough segmentation with the bounding rectangle of the target structure mask after the precise segmentation. In some embodiments, the difference between the bounding rectangle of the target structure mask after the rough segmentation and the bounding rectangle of the target structure mask after the precise segmentation may be determined by comparing the bounding rectangle of the target structure mask after the rough segmentation with the bounding rectangle of the target structure mask after the precise segmentation in 6 directions (i.e., the entire bounding rectangle is a cube in the 3D space) of a 3D space. Merely by way of example, the processing device 130 may calculate an overlap rate of each side of the bounding rectangle of the target structure mask (the first mask) after the rough segmentation to each side of the bounding rectangle of the target structure mask (the second mask) after the precise segmentation, or calculate a difference between vertex coordinates of the bounding rectangle of the target structure mask after the rough segmentation and vertex coordinates of the bounding rectangle of the target structure mask after the precise segmentation.

In some embodiments, whether a result of the target structure mask after the rough segmentation is accurate may be determined according to a difference between the positioning information of the rough segmentation and the positioning information of the precise segmentation. In some embodiments, the positioning information may be a bounding rectangle (e.g. the bounding rectangle frame) of the target structure mask. Whether the bounding rectangle of the target structure mask after the rough segmentation is accurate may be determined according to the bounding rectangle of the target structure mask after the rough segmentation and the bounding rectangle of the target structure mask after the precise segmentation. In this case, the difference between the positioning information of the rough segmentation and the positioning information of the precise segmentation refers to a distance between the closest frame lines of the bounding rectangle of the rough segmentation and the bounding rectangle of the precise segmentation. In some embodiments, when the difference between the positioning information of the rough segmentation and the positioning information of the precise segmentation is relatively large, (i.e., the distance between the closest frame lines of the bounding rectangle of the rough segmentation and the bounding rectangle of the precise segmentation is relatively large), the positioning information of the rough segmentation may be determined to be accurate; when the difference is between the positioning information of the rough segmentation and the positioning information of the precise segmentation is relatively small, (i.e., the distance between the closest frame lines of the bounding rectangle of the rough segmentation and the bounding rectangle of the precise segmentation is relatively small), the positioning information of the rough segmentation may be determined to be inaccurate. It should be noted that the bounding rectangle frame of the rough segmentation may be obtained by performing pixel expansion (e.g., performing pixel expansion by 15-20 voxels) on frame lines of original rough segmentation close to the target structure. In some embodiments, whether the positioning information of the rough segmentation is accurate may be determined based on a relationship between the distance from the closest frame lines of the bounding rectangle of the rough segmentation and the bounding rectangle of the precise segmentation and a preset threshold. For example, when the distance is less than the preset threshold, the positioning information of the rough segmentation may be determined to be inaccurate; when the distance is greater than the preset threshold, the positioning information of the rough segmentation may be determined to be accurate. In some embodiments, in order to ensure the accuracy of determination the value of the preset threshold may be less than or equal to 5 voxels.

When the positioning information of the target structure mask after the rough segmentation is determined to be accurate, the operation 1130 may be performed: the preliminary accurate segmentation result may be used as a target segmentation result. When the positioning information of the target structure mask after the rough segmentation is determined to be inaccurate, the operation 1140 may be performed: the target segmentation result of the target structure may be determined by an adaptive sliding window mode.

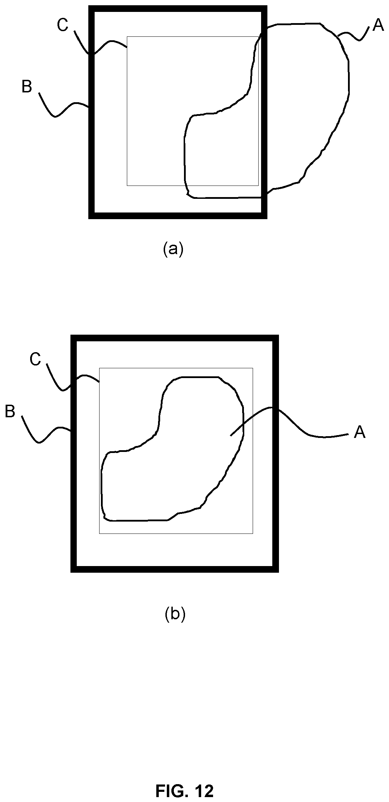

FIG. 12 is a schematic diagram illustrating an exemplary process of determining positioning information of a target structure mask according to some embodiments of the present disclosure. FIG. 12(a) and FIG. 12(b) illustrate a target structure mask A obtained by rough segmentation, a bounding rectangle frame B of the target structure mask A (i.e., positioning information of the target structure mask after the rough segmentation), and a bounding rectangle frame C (i.e., positioning information of the target structure mask after precise segmentation) after preliminary precise segmentation based on the bounding rectangle frame of the rough segmentation. For convenience, a plane rectangle frame within a plane of a 3D bounding rectangle frame is taken as an example. It can be understood that there are 5 other plane rectangle frames within the 3D bounding rectangle frame, i.e., there are frame lines in 6 directions during the specific calculation of the 3D bounding rectangle frame, and only 4 frame lines within a certain plane are used for illustration hereinafter.

Merely by way of example, as shown in FIG. 12(a), a difference between a right frame line in the bounding rectangle frame C of the precise segmentation and a frame line corresponding to the bounding rectangle frame B of the rough segmentation may be relatively small (i.e., a distance between the right frame line in the bounding rectangle frame C of the precise segmentation and the frame line corresponding to the bounding rectangle frame B of the rough segmentation may be relatively small), such that a direction corresponding to a right side of the bounding rectangle frame B of the rough segmentation may be inaccurate, and the right frame line needs to be adjusted. However, differences between upper, lower, and left frame lines of the bounding rectangle frame C and upper, lower, and left frame lines of the bounding rectangle frame B, respectively, may be relatively large, such that directions corresponding to the upper, lower, and left sides of the bounding rectangle frame B of the rough segmentation may be accurate. In this case, the positioning information of the target structure mask after the rough segmentation may be determined to be inaccurate, and the right frame line may be adjusted by the adaptive sliding window mode to determine the target segmentation result of the target structure. More descriptions may be found in the operation 1140.

Merely by way of example, as shown in FIG. 12(b), differences between frame lines of four sides in the bounding rectangle frame C of the precise segmentation and frame lines corresponding to the bounding rectangle frame B of the rough segmentation may be relatively large, such that the frame lines of the four sides in the bounding rectangle frame B of the rough segmentation may be determined to be accurate, i.e., the positioning information of the target structure mask after the rough segmentation may be accurate. In this case, the preliminary precise segmentation result may be used as the target segmentation result.

It should be noted that there are 6 directions for the target structure mask A, and only 4 frame lines are used for illustration in FIG. 12. In an actual situation, 12 frame lines in the 6 directions in the target structure mask A may be determined.

In 1130, the preliminary precise segmentation result may be used as a target segmentation result.

The accurate positioning information of the rough segmentation indicates that a result of the rough segmentation is accurate, and thus the preliminary precise segmentation result obtained based on the positioning information of the rough segmentation is also accurate. Therefore, the preliminary precise segmentation result may be output as the target segmentation result, i.e., a precise segmentation may be performed.

In 1140, the target segmentation result of the target structure may be determined by an adaptive sliding window mode.

The inaccurate positioning information of the rough segmentation indicates that the result of the rough segmentation is inaccurate. In this case, the target structure obtained by the precise segmentation is likely to be inaccurate. The corresponding adaptive sliding window calculation may be performed, and the accurate positioning information may be obtained to continue the precise segmentation.

In some embodiments, a direction in which the positioning information has a deviation may be determined as a target direction, and the adaptive sliding window calculation may be performed in the target direction according to an overlap rate parameter. In some embodiments, at least one direction in which the bounding rectangle is inaccurate may be determined as the target direction, such as a direction corresponding to a right side of the bounding rectangle B in FIG. 12 (a). After the bounding rectangle of the rough segmentation is determined to be inaccurate, the bounding rectangle of the rough segmentation may be slid in the target direction according to an input preset overlap rate parameter, i.e., a sliding window operation may be performed, and the sliding window operation may be repeated until all the bounding rectangles are completely accurate.

The overlap rate parameter refers to a ratio of an area of the overlapped portion between an initial bounding rectangle frame and a bounding rectangle frame after sliding to an area of the initial bounding rectangle frame. When the overlap rate parameter is high, a sliding step length of the sliding window operation may be short. For example, the overlap rate parameter may be set to be relatively small to make the sliding window calculation more concise (i.e., the steps of the sliding window operation are fewer); the overlap rate parameter may be set to be relatively large to make the result of the sliding window calculation more accurate. In some embodiments, the sliding step length of the sliding window operation may be calculated based on a current overlap rate parameter.

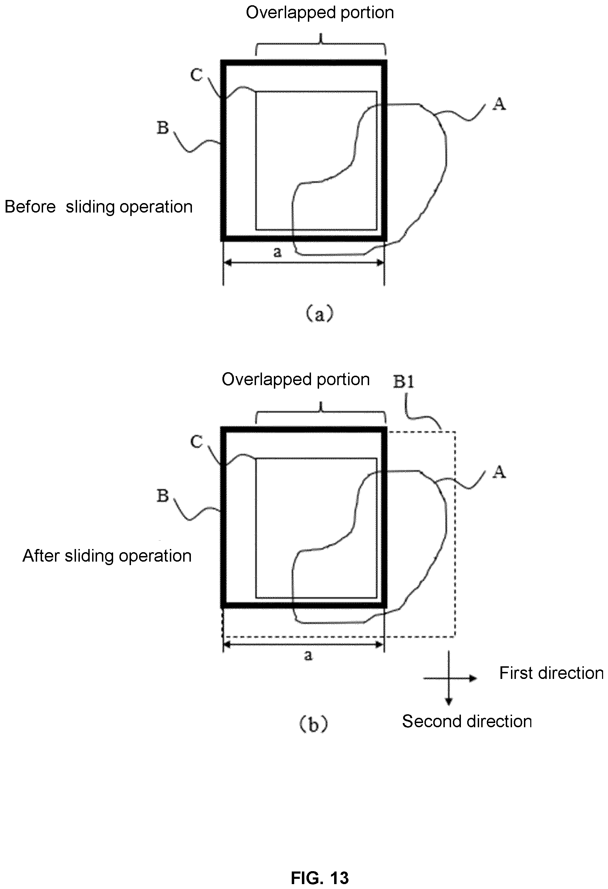

FIG. 13 is a schematic diagram illustrating an exemplary process of determining a sliding direction according to some embodiments of the present disclosure. FIG. 13 illustrates a sliding window B1 obtained after the bounding rectangle frame B of rough segmentation slides, wherein (a) is a schematic diagram before a sliding operation, and (b) is a schematic diagram after the sliding operation.

It can be seen from the determination method in FIG. 12(a) that directions corresponding to right and lower frame lines of the bounding rectangle frame B of the rough segmentation in FIG. 13 are inaccurate. For the convenience of description, the direction corresponding to the right frame line of the bounding rectangle frame B is recorded as a first direction, the first direction being perpendicular to the right frame line of the bounding rectangle frame B; and the direction corresponding to the lower frame line of the bounding rectangle frame is recorded as a second direction, the second direction being perpendicular to the lower frame line of the bounding rectangle frame B. Merely by way of example, as shown in FIG. 13, assuming that a length of the bounding rectangle frame B is a, when the overlap rate parameter is 60%, the corresponding step length may be determined to be a*(1-60%). As mentioned above, the right frame line of the bounding rectangle frame B may slide along the first direction by a*(1-60%). Similarly, the lower frame line of the bounding rectangle frame B may slide along the second direction by a corresponding step length. The right frame line and the lower frame line of the bounding rectangle frame B may repeat the corresponding sliding window operation, respectively, until the bounding rectangle frame B is completely accurate, as shown in the sliding window B1 in FIG. 13 (b). Referring to FIG. 12(a) and FIG. 13, when it is determined that the bounding rectangle frame (i.e., the positioning information of the target structure mask) of the rough segmentation is inaccurate, coordinate values of the frame lines in 6 directions of the bounding rectangle frame of the precise segmentation may be compared with coordinate values of the frame lines in the 6 directions of the bounding rectangle frame of the rough segmentation. When a difference value obtained by the comparison is less than a coordinate difference threshold (e.g., the coordinate difference threshold is 5pt), it is determined that the frame lines of the bounding rectangle frame are in inaccurate directions. The coordinate difference threshold may be set according to actual conditions, which is not limited here.

As another example, as shown in FIG. 12(a), pixel coordinates in the four directions corresponding to the four sides of the bounding rectangle frame C of the precise segmentation may be compared one by one with pixel coordinates in the four directions corresponding to the four frame lines of the bounding rectangle frame B of the rough segmentation. When a difference between the pixel coordinates in one direction is less than a coordinate difference threshold of 8 pt, it is determined that the direction of the bounding rectangle frame of the rough segmentation in FIG. 12 (a) is inaccurate. For example, if the difference between the upper sides is 20 pt, the difference between the lower sides is 30 pt, the difference between the right sides is 1 pt, and the difference between the left sides is 50 pt, then the direction corresponding to the right side may be inaccurate, and the directions corresponding to the upper side, the lower side, the left side may be accurate, and the direction corresponding to the right side may be determined as the target direction.

As another example, referring to FIG. 13 (a) and FIG. 13(b), B1 is a bounding rectangle frame (also referred to as a sliding window) obtained after the bounding rectangle frame B of the rough segmentation slides. It can be understood that the sliding window is the bounding rectangle frame of the rough segmentation that meets an expected accuracy standard, and the frame lines (e.g., the right frame line, and the lower frame line) of the bounding rectangle frame B of the rough segmentation needs to be slid along the corresponding directions (e.g., the first direction, and the second direction) by a corresponding step length to a position of the sliding window B1. The directions corresponding to the frame lines that do not meet the standard are moved in sequence. For example, the right frame line of the bounding rectangle frame B may be slid first, and then the lower frame line of the bounding rectangle frame B may be slid to a specified position of the sliding window. The directions corresponding to the left side and the top of the bounding rectangle frame may meet standard, so no sliding is required. It can be understood that the sliding step length of each side may depend on an overlap rate of B1 and B. The overlap rate may be a ratio of a current overlapped area of the bounding rectangle frame B of the rough segmentation and the sliding window B1 to a total area. For example, the current overlap rate may be 40%, etc. It should be noted that a sliding order of the frame lines of the bounding rectangle frame B of the rough segmentation may be from left to right, from top to bottom, or other feasible order, which is not limited here.

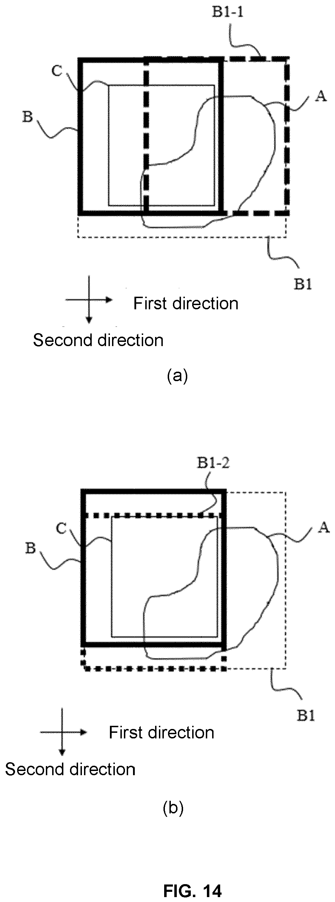

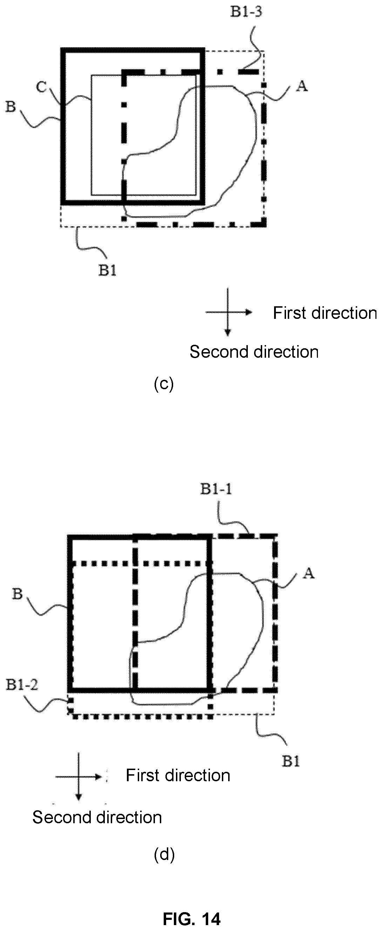

FIG. 14 is a schematic diagram illustrating an exemplary process of performing precise segmentation after sliding window according to some embodiments of the present disclosure.

In some embodiments, after an accurate bounding rectangle frame of rough segmentation is obtained by an adaptive sliding window based on an original bounding rectangle frame of the rough segmentation (also referred to as an original sliding window), coordinate values of the accurate bounding rectangle frame may be obtained. Precise segmentation may be performed on a new sliding window based on the coordinate values and an overlap rate parameter, and a precise segmentation result may be superimposed with a preliminary precise segmentation result to obtain a final precise segmentation result. Specifically, referring to FIG. 14(a), a sliding window B1 (a bounding rectangle frame of a maximum range after a sliding window operation) may be obtained by performing the sliding window operation on an original bounding rectangle frame B. The B may along a first direction by a corresponding step length to obtain a sliding window B1-1, and then a precise segmentation result of the sliding window B1-1 may be obtained by performing the precise segmentation on the entire domain range of the sliding window B1-1. Further, referring to FIG. 14(b), the B may slide along a second direction by a corresponding step length to obtain a sliding window B1-2, and then a precise segmentation result of the sliding window B1-2 may be obtained by performing the precise segmentation on the entire domain range of the sliding window B1-2. Furthermore, referring to FIG. 14(c), the B may slide to obtain a sliding window B1-3 (e.g., the B may slide as a sliding operation shown in FIG. 14(c) to obtain the sliding window B1-2, and then sliding window B1-2 may slide to obtain the sliding window B1-3), and then a precise segmentation result of the sliding window B1-3 may be obtained by performing the precise segmentation on the entire domain range of the sliding window B1-3. The precise segmentation results and the preliminary precise segmentation results of the sliding window B1-1, the sliding window B1-2, and the sliding window B1-3 may be superimposed to obtain the final precise segmentation result. It should be noted that sizes of the sliding window B1-1, the sliding window B1-2, and the sliding window B1-3 may be the same as a size of the B. The sliding window B1 may be a final sliding window result obtained by a continuous sliding window operation of the original sliding window B, i.e., the sliding window B1-1, the sliding window B1-2, and the sliding window B1-3. In some embodiments, when the precise segmentation results and the preliminary precise segmentation results of the sliding window B1-1, the sliding window B1-2, and the sliding window B1-3 are superimposed, repeated superimposed portions may exist. For example, in FIG. 14(d), an intersection may exist between the sliding window B1-1 and the sliding window B1-2. When the segmentation results are superimposed, the intersection may be repeatedly superimposed. In this case, the following mode may be adopted: for a portion of a target structure mask A, if a segmentation result of one sliding window for the portion is accurate and a segmentation result of another sliding window for the portion is inaccurate, the segmentation result of the sliding window for the portion being accurate may be used as a segmentation result of the portion; if the segmentation results of the two sliding windows for the portion are both accurate, the segmentation result of the right sliding window for the portion may be used as the segmentation result of the portion; if the segmentation results of the two sliding windows for the portion are inaccurate, the segmentation result of the right sliding window for the portion may be used as the segmentation result of the portion, and the precise segmentation may be continued until the segmentation result is accurate.

In some embodiments, when it is determined that positioning information of the target structure mask after the rough segmentation is inaccurate, obtaining accurate positioning information based on the adaptive sliding window may be a cyclic process, i.e., performing the same operation as the preliminary precise segmentation twice or more. For example, after frame lines of the preliminary precise segmentation are compared with the frame lines of the rough segmentation, updated coordinate values of a bounding rectangle frame of the precise segmentation may be obtained through the adaptive sliding window. The bounding rectangle frame of the precise segmentation may be expanded by a certain count of pixels and set as a bounding rectangle frame of the rough segmentation (also referred to as a target bounding rectangle frame) of a new cycle. Then a new bounding rectangle frame of the precise segmentation may be obtained by performing the precise segmentation on the new bounding rectangle frame (i.e., the target bounding rectangle frame), whether the target bounding rectangle frame is accurate may be calculated. If the target bounding rectangle frame is accurate, the cycle may be ended and the new bounding rectangle frame of the precise segmentation may be output as the target segmentation result; otherwise, the cycle may continue.

In some embodiments, the precise segmentation may be performed on at least one target structure obtained by the rough segmentation using a deep convolutional neural network (DCNN) model. For example, historical target images preliminarily obtained before the rough segmentation may be used as training data, and the DCNN model may be trained with historical precise segmentation result data. In some embodiments, the historical target images and the historical precise segmentation result data may be obtained from the imaging device 110, or obtained from the processing device 130, the terminal device 140, or the storage device 150.

In some embodiments, result data of the at least one target structure subjected to the precise segmentation, i.e., the target segmentation result, may be output. In some embodiments, in order to further reduce noise and optimize the film display effect, post-processing may be performed on the target segmentation result before the target segmentation result is output. For example, the post-processing operation may include edge smoothing and/or denoising of the film/image. In some embodiments, the edge smoothing may include smoothing or blurring to reduce noise or distortion of the image. In some embodiments, the smoothing or blurring may be performed in the following manners: mean filtering, median filtering, Gaussian filtering, bilateral filtering, or the like, or any combination thereof.

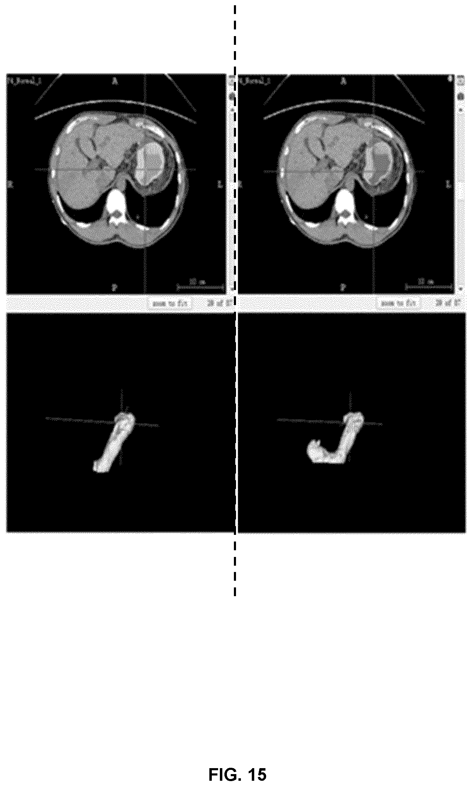

FIG. 15 is a schematic diagram illustrating a comparison of exemplary segmentation results according to some embodiments of the present disclosure.

As shown in FIG. 15, the upper and lower portions on the left side of the dotted line are a cross-sectional target image and a stereoscopic target image of a result of rough segmentation using the conventional technology, and the right side is a cross-sectional target image and a stereoscopic target image using a method for organ segmentation provided by the embodiments of the present disclosure. By comparison, it can be seen that a target structure segmentation result displayed by a segmentation result image on the right side is more complete than a target structure segmentation result displayed by a segmentation result image on the left side in terms of the target structure, which reduces the risk of missing a segmented target structure, improves the segmentation accuracy, and finally improves the overall segmentation efficiency.

It should be noted that the above description of the process 1100 is only for example and explanation, and does not limit the scope of application of the present disclosure. For those skilled in the art, various modifications and changes can be made to the process 1100 under the guidance of the present disclosure. However, such modifications and changes are still within the scope of the present disclosure.

Some embodiments of the present disclosure further provide a device for image segmentation. The device may include a processor configured to execute the method for image segmentation described in any embodiment. In some embodiments, the device for image segmentation may further include a display device. The display device may display a result of a method for medical image segmentation executed based on the processor. More descriptions may be found in FIGs. 5-15, which are not repeated here.

According to the method for image segmentation provided in the embodiments of the present disclosure, (1) the target structure region can be accurately retained while effectively excluding the false positive region by adopting the method of soft connected domain analysis in the rough segmentation stage, first improving the positioning accuracy of the target structure in the rough positioning stage, and directly helping to reasonably extract the bounding frame of the positioning information of the target structure mask in the subsequent stage, thereby improving the segmentation efficiency; (2) for the unfavorable situation that the rough positioning is inaccurate but not invalid in the rough segmentation stage, the missing portion of the positioning region can be filled using the calculation of the adaptive sliding window and the corresponding sliding window operation, and the reasonable sliding window operation can be automatically planned and executed, reducing the dependence of the precise segmentation stage on the result of the rough positioning, and improving the segmentation accuracy while without significant increase in the segmentation time and computing resources; (3) when the rough positioning fails, the target structure mask is accurately positioned based on the preset positioning coordinates of the target structure, which not only improves the segmentation accuracy, but also reduces the segmentation time, reduces the calculation amount of segmentation, thereby improving the segmentation efficiency; (4) since the overall workflow of the target structure segmentation fully considers various unfavorable situations that reduce the segmentation accuracy of the target structure, it is suitable for the effective implementation of different types of segmentation tasks of the target structure, which has high segmentation accuracy and segmentation robustness of the target structure.

An animal generally has various ducts in the body, such as blood vessels, trachea, bile ducts, or ureters, etc. There are often multiple ducts in an organism. The same duct can be divided into multiple types due to different structures and functions of the duct. For example, the blood vessels include at least two main types: arteries and veins. In some embodiments, the types of ducts in the organism may include subdivided types of ducts, such as pulmonary veins, pulmonary arteries, hepatic veins, hepatic portal veins, hepatic arteries, etc.

The embodiments of the present disclosure provide a method for duct recognition. First, a first segmentation model with low richness but accurate and a second segmentation model with high richness and unclassified may be trained. Then, duct growth may be performed on a result of a low richness model using a result of a high richness model with a post-processing algorithm. The low richness model and the high richness model may be fused. Finally, multi-class duct segmentation results with high richness and high accuracy may be accurately and effectively obtained. The specific operation regarding the duct recognition is described in detail below with reference to FIGs. 16-23.

FIG. 16 is a module diagram illustrating an exemplary device for duct recognition according to some embodiments of the present disclosure.

As shown in FIG. 16, in some embodiments, a device 1600 for duct recognition may include a first segmentation module 1610, a processing module 1620, a second segmentation module 1630, and a fusion module 1640. In some embodiments, corresponding functions of the device 1600 for duct recognition may be implemented by the processing device 130 or the device 300 for puncture path planning (e.g., the data preprocessing module 310).

The first segmentation module 1610 may be configured to obtain a first segmentation result of a target image based on a first segmentation model.

The processing module 1620 may be configured to obtain a first duct skeleton set by performing skeletonization processing on the first segmentation result. The first duct skeleton set may include at least one first duct skeleton of a determined type.

The second segmentation module 1630 may be configured to obtain a second segmentation result of the target image based on a second segmentation mode. The second segmentation result may include at least one duct of an undetermined type.

The fusion module 1640 may be configured to obtain a fusion result by fusing the first segmentation result and the second segmentation result. In some embodiments, the fusion module 1640 may also be configured to determine a duct type. Specifically, the fusion module 1640 may be configured to obtain a second duct skeleton of the one of the at least one duct of the undetermined type by performing the skeletonization processing on the fusion result; obtain one or more first duct skeletons, a minimum spatial distance between each of the one or more first duct skeletons and the second duct skeleton is less than a second threshold, and designate the one or more first duct skeletons as one or more reference duct skeletons; determine a spatial distance between the second duct skeleton and the reference duct skeleton, and determine two points with a minimum spatial distance as a set of closest points; and determine a duct type of one of the at least one duct of the undetermined type based on the set of closest points.

In some embodiments, the device 1600 for duct recognition may further include a calculation module, a determination module, and a training module (not shown in the figure). The calculation module may be configured to obtain the one or more first duct skeletons, and the minimum spatial distance between each of the one or more first duct skeletons and the second duct skeleton is less than the second threshold, and designate the one or more first duct skeletons as the one or more reference duct skeletons; and determine the spatial distance between the second duct skeleton and the reference duct skeleton, and determine the two points with the minimum spatial distance as the set of closest points. The determination module may be configured to determine the duct type of one of the at least one duct of the undetermined type based on the set of closest points. The training module may be configured to perform model training, such as training to obtain a machine learning model for determining the second threshold.

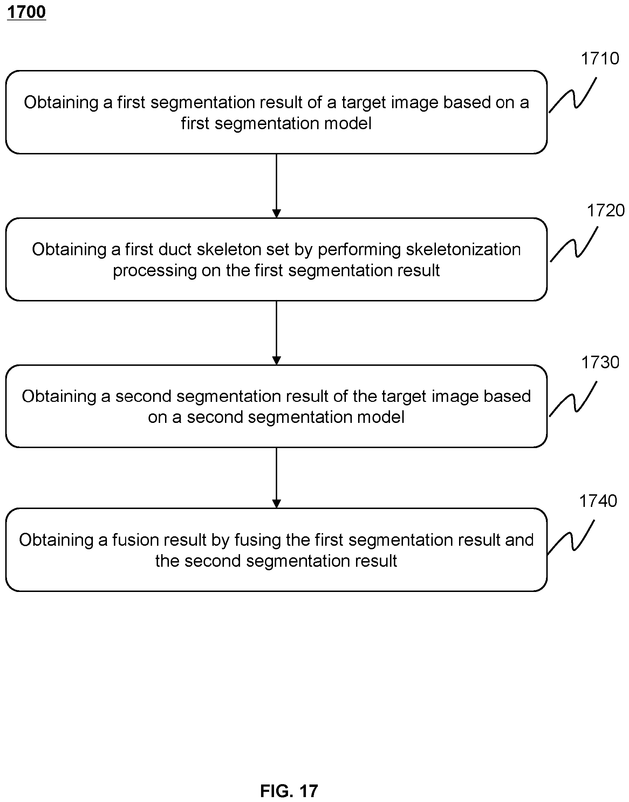

More descriptions regarding various modules of the device 1600 for duct recognition may be found in FIGs. 17-23 and related descriptions thereof, which are not repeated here.

The description of the device 1600 for duct recognition is for illustrative purposes only and is not intended to limit the scope of the present disclosure. For those having ordinary skills in the art, various forms and details of improvements and changes can be made to the application of the above method and system without departing from the principle of the present disclosure. However, such changes and modifications do not depart from the scope of the present disclosure.

FIG. 17 is a flowchart illustrating an exemplary method for duct recognition according to some embodiments of the present disclosure. In some embodiments, a process 1700 may be performed by the system 100 for puncture path planning (e.g., the processing device 130 of the system 100 for puncture path planning) or the device 1600 for duct recognition. For example, the process 1700 may be stored in a storage device (e.g., the storage device 150, and the storage unit of the system) in the form of a program or instruction. When the processor or the module shown in FIG. 16 executes the program or instruction, the process 1700 may be implemented. As shown in FIG. 17, in some embodiments, the process 1700 may include the following operations.

In 1710, a first segmentation result of a target image may be obtained based on a first segmentation model. In some embodiments, the operation 1710 may be performed by the processing device 130 or the first segmentation module 1610.

The first segmentation result may include a segmented image of a duct in a specific organism, i.e., a film or an image obtained after first segmentation is performed on the target image. In some embodiments, a type of at least one duct in the first segmentation result may be determined.

The first segmentation model may segment the ducts in the organism accurately and determine types of some of the ducts. Precise and/or subdivided types of the ducts in the organism in the target image may be obtained using the first segmentation model, such as a pulmonary vein, a pulmonary artery, a hepatic vein, a hepatic portal vein, etc. In some embodiments, the first segmentation model may include a multi-class segmentation model capable of classifying the ducts accurately. The first segmentation model may be configured to classify all or part of the ducts in the target image. In some embodiments, the first segmentation model may be configured to segment and classify the ducts within a set level range. In some embodiments, the first segmentation model may be configured to segment and classify some of the ducts within and outside the set level range. In some embodiments, the first segmentation model may be configured to segment the ducts within one set level range. In some embodiments, the first segmentation model may be configured to segment and/or classify a 3D image (i.e., the target image is the 3D image).

The types of ducts may include two or more types. For example, the types of ducts may include a first type and a second type. The first type and the second type are types of ducts that appear in the target image at the same time and are of different categories. The first type of ducts and the second type of ducts in the target image usually have close or similar features (e.g., contours, grayscale values, etc.). For example, the first type and the second type may be veins and arteries, respectively. As another example, under a CT image, the first type and the second type may be binary groups such as (renal vein, ureter), (abdominal portal vein, abdominal artery), etc. As another example, the types of ducts in the target image of an abdomen or liver region may include a hepatic portal vein, a hepatic vein, a hepatic artery, etc.

In some embodiments, the first segmentation model may be obtained by training. The first segmentation model may be a machine learning model. The machine learning model may include but is not limited to one or more of a neural network model, a support vector machine model, a k-nearest neighbor model, a decision tree model, or the like, or any combination thereof. The neural network model may include but is not limited to one or more of CNN, LeNet, GoogLeNeT, ImageNet, AlexNet, VGG, ResNet, or the like, or any combination thereof.

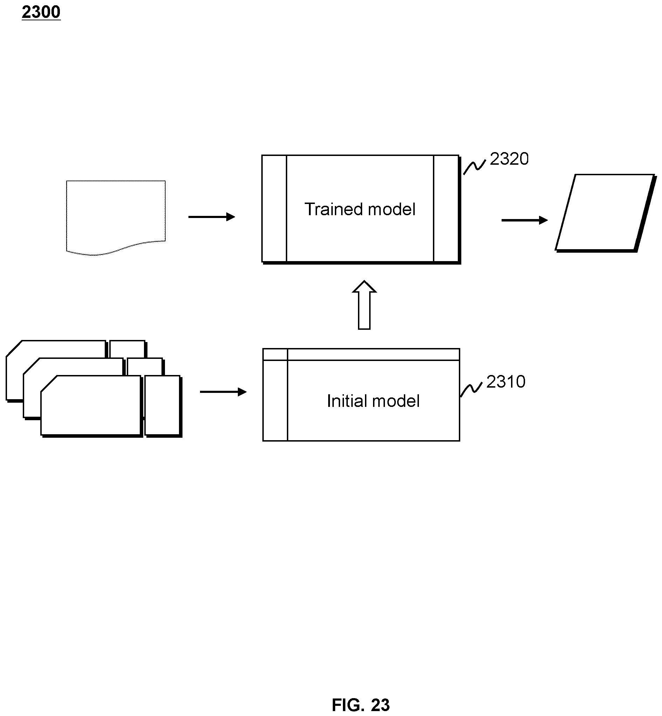

In some embodiments, the first segmentation model may include a CNN model. The processing device 130 may perform model training by improving a network receptive field, improving a network depth, etc., to improve the accuracy of the first segmentation model in classifying the ducts within the set level range in the organism. For example, the network receptive field may be improved using methods such as dilated convolution. More descriptions regarding the training of the first segmentation model may be found in the related descriptions of FIG. 23 of the present disclosure.

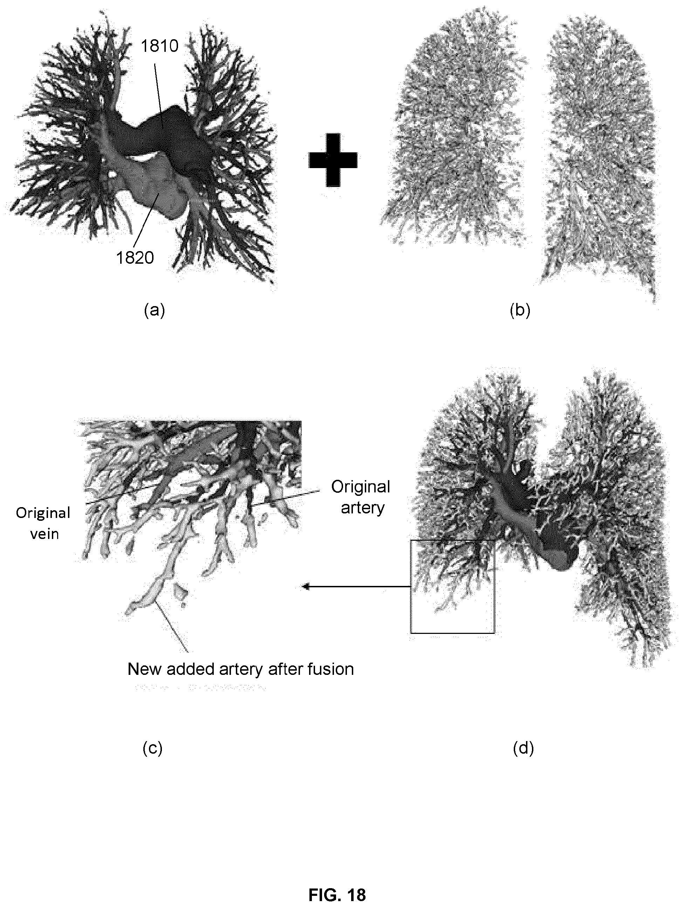

In some embodiments, an input of the first segmentation model may be the target image (e.g., the 3D image of the organism), and an output of the first segmentation model may be the first segmentation result. The first segmentation result may include a segmented image of a duct (e.g., a human blood vessel) in a specific organism. For example, the first segmentation result may include a segmented image of the pulmonary artery and the pulmonary vein, or a segmented image of the hepatic artery and the hepatic portal vein, etc. Different types of ducts in the organism in the first segmentation result may be distinguished by coloring separately or by different grayscale values. For example, as shown in FIG. 18(a) and FIG. 18(b), pixels (or voxels) of arteries in (a) may be uniformly set to a relatively dark grayscale, and pixels (or voxels) of veins in (b) may be uniformly set to a relatively light grayscale.

In 1720, a first duct skeleton set may be obtained by performing skeletonization processing on the first segmentation result. In some embodiments, the operation 1720 may be performed by the processing device 130 or the processing module 1620.

The skeletonization processing refers to a process of simplifying a duct image or film into a center line of a unit width (e.g., a unit pixel width, and unit voxel width). The skeletonization processing may retain a center line, line endpoints, intersections, etc. of an original image or film, thereby retaining the connectivity of the original image. The skeletonization processing may reduce redundant information and retain only useful information for topological analysis, shape analysis, etc. The skeletonization processing enables an object to be represented by a simpler data structure, thereby simplifying data analysis, reducing data storage, and reducing the requirements for transmission equipment.

In some embodiments, methods for the skeletonization processing may include a parallel fast refinement algorithm, a K3M algorithm, or the like.

In some embodiments, a type of at least one duct in the first segmentation result may be determined. Accordingly, a skeleton in the first duct skeleton set obtained by performing the skeletonization processing on the first segmentation result may correspond to the duct of a determined type. That is, the first duct skeleton set may include at least one first duct skeleton of the determined type. By performing the skeletonization processing on the first segmentation result, subsequent calculation may be facilitated, and the efficiency of the recognition method may be improved.

In 1730, a second segmentation result of the target image may be obtained based on a second segmentation model. In some embodiments, the operation 1730 may be performed by the processing device 130 or the second segmentation module 1630.

The second segmentation result may include a segmented image of the duct in the organism, i.e., a segmented film or image obtained after the target image is subjected to second segmentation. In some embodiments, the second segmentation result may include at least one duct of an undetermined type. The at least one duct of the undetermined type means that the type of the duct is undetermined. The at least one duct of the undetermined type may be any of the above types. For example, it is temporarily undetermined whether a blood vessel in the lung is a vein or artery blood vessel, it is temporarily undetermined whether a duct in the kidneys is a renal vein or ureter duct, and it is temporarily undetermined whether a duct in the liver is a hepatic vein, hepatic portal vein, or hepatic artery duct. In this case, more types may be classified, not just limited to the first and second types mentioned above, and there can also be a third type or even more. For example, under the MR image, the first type, the second type, and the third type may be triple groups such as (hepatic artery, hepatic vein, hepatic portal vein). In some embodiments, the at least one duct in the second segmentation result may not be included in the first segmentation result. In some embodiments, at least one duct in the second segmentation result in the second segmentation result that is not included in the first segmentation result may be the duct of the undetermined type.

The second segmentation model is a model configured to segment the ducts in the organism more abundantly, so as to segment the smaller ducts as much as possible. An image including deep branches and/or small ducts may be obtained using the second segmentation model. For example, the second segmentation model may be configured to segment an image including ducts of levels 1-6 or even smaller, an image including blood vessels of levels 1-6 or even smaller, etc. In some embodiments, the second segmentation model may include a single-category segmentation model capable of segmenting more ducts. The second segmentation model of the at least one blood vessel in the second segmentation result may be configured to segment all or part of the ducts in the target image.

In some embodiments, the second segmentation model may be obtained by training a machine learning model. The machine learning model may include but is not limited to one or more of a neural network model, a support vector machine model, a k-nearest neighbor model, a decision tree model, or the like, or any combination thereof.

In some embodiments, the second segmentation model may include a CNN model. When the second segmentation model is constructed, a count of downsampling times may be reduced to avoid loss of details caused by excessive downsampling, such that the second segmentation model may recognize more detailed ducts. More descriptions regarding the training of the second segmentation model may be found in the related descriptions of FIG. 23 of the present disclosure.

In some embodiments, an input of the second segmentation model may be the target image, and an output of the second segmentation model may be the second segmentation result. For example, edges of the ducts in the second segmentation result may be labeled, and the ducts in an output image may be uniformly colored. For example, as shown in the segmented image shown in FIG. 18(b), the edges of the ducts may be labeled, and pixels (or voxels) of the ducts in the image may be filled with the same grayscale value. In some embodiments, types of all or part of the ducts in the segmented image output by the second segmentation model may be undetermined.

The deep branches and/or fine ducts may be obtained using the second segmentation model. Compared with the first segmentation model, the second segmentation model may have a higher richness. In some embodiments, a range of a first segmentation level of the first segmentation model may be less than a range of a second segmentation level of the second segmentation model. The second segmentation model may be configured to segment a larger range of blood vessels than the first segmentation model. In some embodiments, the range of the second segmentation level of the second segmentation model and the range of the first segmentation level of the first segmentation model may have an intersection, but the second segmentation model may be configured to segment finer ducts than the first segmentation model. In some embodiments, the range of the first segmentation level of the first segmentation model may overlap with the range of the second segmentation level of the second segmentation model. However, when the second segmentation model is configured to segment the finer ducts, the richness and/or recognition may be better than the first segmentation model. For example, the first segmentation result may include ducts of levels 1-4, while the second segmentation result may include ducts of levels 1-6 or even finer levels. Ducts of levels 5-6 or even finer levels in the second segmentation result may not be included in the first segmentation result. The higher the level value, the more difficult it is to recognize the corresponding ducts. For example, the ducts of level 5 may be thinner than the ducts of level 4, and thus the ducts of level 5 may be more difficult to recognize than the ducts of level 4.

In 1740, a fusion result may be obtained by fusing the first segmentation result and the second segmentation result. In some embodiments, the operation 1740 may be performed by the processing device 130 or the fusion module 1640.

In some embodiments, the processing device 130 may obtain the fusion result by fusing information of the first segmentation result and the second segmentation result. The fusion result may be a film/image including the ducts in the target image and the types of all or part of the ducts.

In some embodiments, a union set of the first segmentation result and the second segmentation result may be obtained, and the fusion result may be obtained based on the union set and the first segmentation result. For example, the processing device 130 may calculate the union set of the first segmentation result and the second segmentation result and process the union set, and then remove a first segmentation result set from a processed union set, and use an obtained difference set as the fusion result. In some embodiments, the difference set may be a set of ducts of the undetermined type remaining after removing the ducts labeled in the first segmentation result from the second segmentation result. For example, the first segmentation result may mark categories of the blood vessels of levels 1-4, and the second segmentation result may include the blood vessels of levels 1-6 or even smaller blood vessels, and the fusion result may be a set of blood vessels of levels 5-6 or even smaller blood vessels of undetermined types.

In some embodiments, the processing device 130 may obtain the fusion result by fusing the first segmentation result and the second segmentation result based on a plurality of fusion methods. For example, the fusion methods may include a principal component transform fusion method, a product transform fusion method, a wavelet transform fusion method, a Laplace transform fusion method, or the like, or any combination thereof.

The second segmentation result may include more ducts than the first segmentation result. The second segmentation result may be fused with the first segmentation result, which is equivalent to a process of blood vessel growth. Since the first segmentation result has a relatively high accuracy and the second segmentation result has a relatively high richness, the ducts with a certain richness and sufficient accuracy and category information of all or part of the ducts may be obtained via fusion, thereby improving the accuracy and richness of the duct segmentation result.

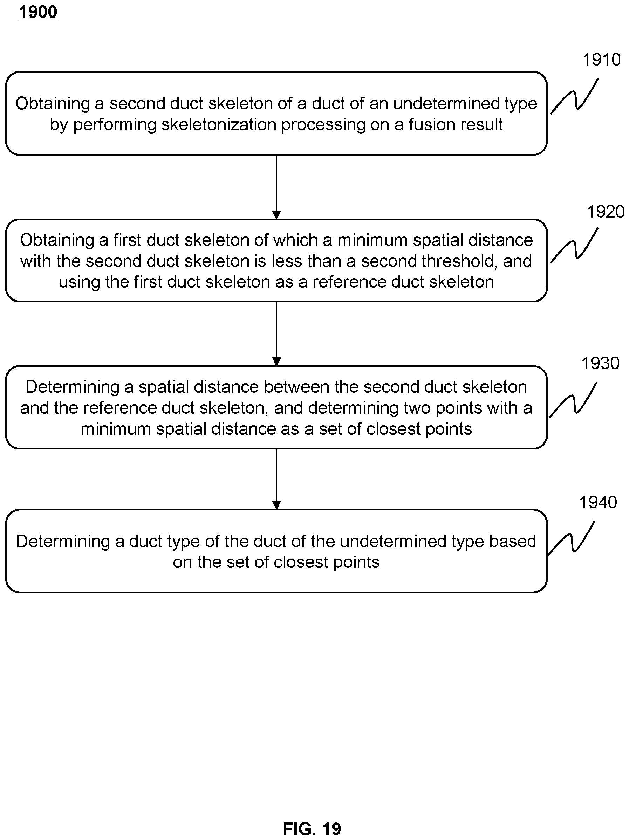

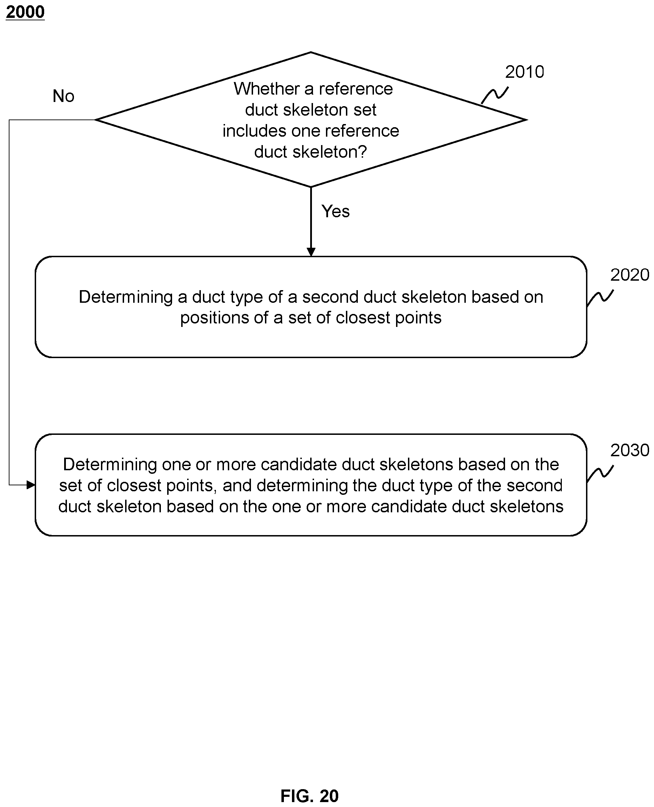

In some embodiments, the types of the ducts of the undetermined type may be determined based on the fusion result. For example, the types of the ducts of the undetermined type may be determined based on a connectivity relationship, a spatial relationship, etc. More descriptions may be found in the related descriptions in FIG. 19 and FIG. 20.

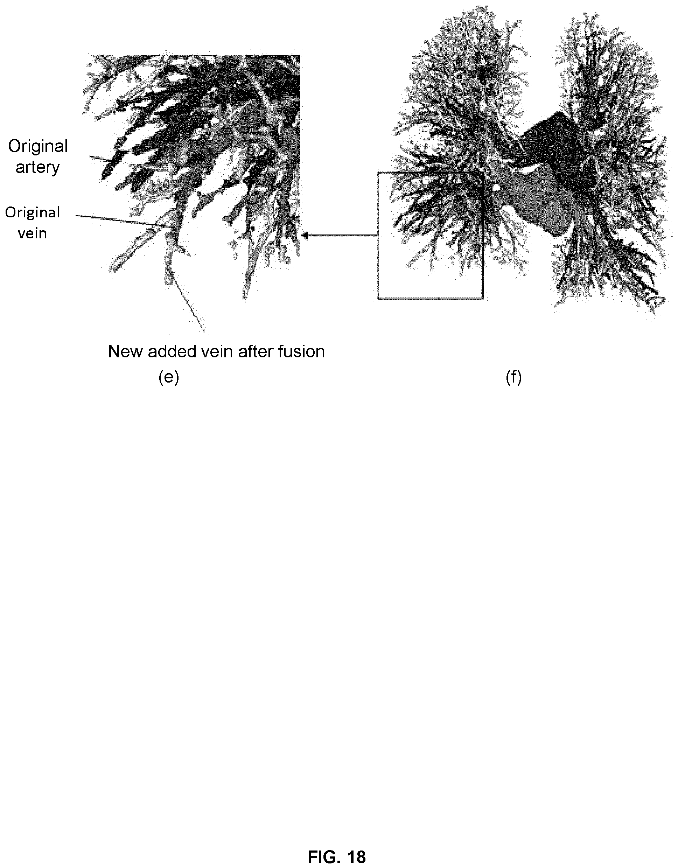

FIG. 18 is a schematic diagram illustrating an exemplary duct recognition result according to some embodiments of the present disclosure. As shown in FIGs. 18(a)-(f), types of ducts in a first segmentation result shown in (a) may be determined. Specifically, a duct 1810 colored in black and gray may be an artery, and a duct 1820 colored in dark gray may be a vein. A second segmentation result shown in (b) may mark the ducts, but may not distinguish the specific types of ducts, and a large number of small ducts may not be included in the first segmentation result. By fusing the first segmentation result in FIG. 18(a) and the second segmentation result in FIG. 18(b), more types of small ducts may be recognized. As shown in FIG. 18(d) and a partial enlargement view FIG. 18(c), in addition to the original vein and artery, the fusion result may add the artery (a light gray duct). As another example, as shown in FIG. 18(f) and a partial enlargement view FIG. 18(e), in addition to the original vein and artery, the fusion result may add the vein (a light-colored duct).

By fusing output results of the first segmentation model with high accuracy and the second segmentation model with high richness, and processing the fusion result, two or more types of ducts with similar grayscale values that are easy to misclassify may be recognized, thereby obtaining a recognition result of the ducts in the organism with both accuracy and richness. For example, the embodiments of the present disclosure can recognize the hepatic portal vein, the hepatic vein, the hepatic artery, etc., of levels 5-6.

In some embodiments, a target point may be determined based on the fusion result. In some embodiments, the target point may be determined based on the types of the ducts in the fusion result.

It should be noted that the above description of the process 1700 is only for example and explanation, and does not limit the scope of application of the present disclosure. For those skilled in the art, various modifications and changes can be made to the process 1700 under the guidance of the present disclosure. However, such modifications and changes are still within the scope of the present disclosure.

FIG. 19 is a flowchart illustrating an exemplary process of determining a duct type according to some embodiments of the present disclosure. In some embodiments, a process 1900 may be performed by the system 100 for puncture path planning (e.g., the processing device 130 of the system for puncture path planning) or the device 1600 for duct recognition. For example, the process 1900 may be stored in a storage device (e.g., the storage device 150, and the storage unit of the system) in the form of a program or instruction. The process 1900 may be implemented when a processor or a module shown in FIG. 16 executes the program or instruction. As shown in FIG. 19, in some embodiments, the process 1900 may include the following operations.

In 1910, a second duct skeleton of a duct of an undetermined type may be obtained by performing skeletonization processing on a fusion result. The operation 1910 may be performed by the processing device 130 or the device 1600 for duct recognition.

In some embodiments, the fusion result may be a set of ducts of the undetermined type. By performing skeletonization processing on the fusion result, a skeleton to be determined, i.e., a second duct skeleton of one of the at least one duct of the undetermined type, may be obtained. More descriptions regarding the skeletonization processing may be found in FIG. 17, which are not repeated here.

In 1920, one or more first duct skeletons may be obtained, a minimum spatial distance between each of the one or more first duct skeletons and the second duct skeleton may be less than a second threshold, the one or more first duct skeletons may be designated as one or more reference duct skeletons. In some embodiments, the operation 1920 may be performed by the processing device 130 or the device 1600 for duct recognition.

In some embodiments, a duct type of one of the at least one duct of the undetermined type may be determined based on a connectivity relationship between the second duct skeleton of one of the at least one duct of the undetermined type and the first duct skeleton in the first duct skeleton set. Specifically, if there is a first duct skeleton (e.g. a skeleton K2 of a determined type) in the first duct skeleton set that is connected with the second duct skeleton (e.g. a skeleton K1 of a determined type), a type of the second duct skeleton of one of the at least one duct of the undetermined type may be the same as the type of the first duct skeleton. In this way, the duct type of the second duct skeleton may be determined. For example, if a segment of a venous skeleton in the first duct skeleton set is connected with a segment of skeleton in the skeleton to be determined (i.e., the second duct skeleton), a blood vessel corresponding to the segment of the skeleton to be determined may also be a vein.

In some embodiments, for each second duct skeleton (e.g., a segment of duct skeleton of an undetermined type), one or more first duct skeletons may be obtained, a minimum spatial distance between each of the one or more first duct skeletons and the second duct skeleton may be less than a second threshold, the one or more first duct skeletons may be designated as one or more reference duct skeletons. One or more reference duct skeletons may form a reference duct skeleton set. Ducts in the reference duct skeleton set may be ducts most closely related to the undetermined duct.

The second threshold may determine a range of the reference duct skeleton, and a value of the second threshold may affect a final recognition effect. In some embodiments, based on different spatial distance calculation methods, the second threshold, as a comparison parameter of the spatial distance, may be different physical quantities. For example, when an actual spatial distance is used as the basis for distance measurement, the second threshold may be a physical quantity that specifically represents a length, such as 10 mm. In some embodiments, the calculation of the spatial distance may be performed after conversion based on voxel points in the image information. In this way, the actual distance value may be converted into a count of the voxel points in the image, and the second threshold may be expressed by the count of voxel points. For example, if the actual distance value is converted into five voxel points, the second threshold may be five. In some embodiments, when a projection angle of the 3D image is consistent, the actual distance value may be converted into a count of pixels, and the count of pixels may be determined as the second threshold. For example, if the actual distance value is converted into five pixels, the second threshold may be five.

In some embodiments, the second threshold may be obtained based on experience or demand. In some embodiments, the second threshold may be customized by a user. In some embodiments, the second threshold may be obtained based on a portion of the organism corresponding to the target image. In some embodiments, the second threshold value may be different based on a level of one of the at least one duct of the undetermined type.

In some embodiments, the second threshold may be obtained by a machine learning method. For example, by constructing a machine learning model, an optimized second threshold corresponding to portions of organisms may be obtained by machine learning based on training data of the portions of different organisms. In practical application, when the portion is recognized, the corresponding second threshold obtained after optimization training may be used. The machine learning model may include but is not limited to one or more of a neural network model, a support vector machine model, a k-nearest neighbor model, a decision tree model, or the like, or any combination thereof.

In some embodiments, the machine learning method of the second threshold may be obtained based on medical images of the portions corresponding to the same type of organisms and type determination results. For example, the second threshold of the organism may be obtained through training using the medical images of the portions corresponding to the same type of organisms as samples, and the type determination results as labels.

In some embodiments, machine training may use at least one of the sex, age, region, and race of the organism as a parameter, and obtain the second threshold value related to the sex, age, region, race, etc. through training. For example, the second threshold may be five for women over 50 years old, and six for women under 50 years old.

By obtaining the second threshold in various ways, manual operations can be reduced and the second threshold can be applied to various scenarios, thereby improving the universality.

In 1930, a spatial distance between the second duct skeleton and the reference duct skeleton may be determined, and two points with a minimum spatial distance may be determined as a set of closest points. In some embodiments, the operation 1930 may be performed by the processing device 130 or the device 1600 for duct recognition.

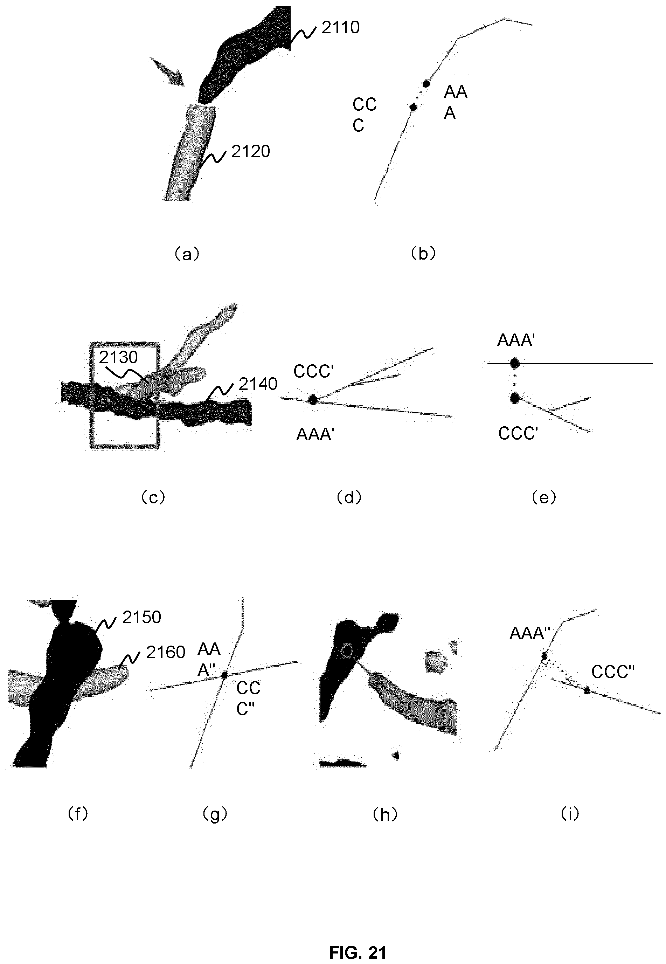

The set of closest points refer to a set of points consisting of two points with the minimum spatial distance between the second duct skeleton (i.e., the undetermined skeleton) of one of the at least one duct of the undetermined type and the reference duct skeleton. For example, as shown in FIG. 21(a) and FIG. 21(b), FIG. 21(a) shows a reconstructed local 3D image, and FIG. 21(b) is a skeleton simulation diagram corresponding to FIG. 21(a). In FIG. 21(a), two ducts are on the same plane in space (the same applies to ducts not on the same plane); a solid line in FIG. 21(b) is the skeleton, and a dotted line is the minimal distance. If the minimum spatial distance between an undetermined skeleton 2110 and a reference duct skeleton 2120 is less than the second threshold, the two points with the minimum spatial distance (AAA and CCC) may be determined as the set of closest points between the undetermined skeleton 2110 and a reference duct skeleton 2120.

In some embodiments, for each reference duct skeleton, the spatial distance between the second duct skeleton and the reference duct skeleton may be determined, and the two points with the smallest spatial distance may be determined as the closest point group.

In 1940, a duct type of one of the at least one duct of the undetermined type may be determined based on the set of closest points. The operation 1940 may be performed by the processing device 130 or the device 1600 for duct recognition.

In some embodiments, in response to determining that a count of the one or more reference duct skeletons equals 1, the duct type of the one of the at least one duct of the undetermined type may be determined based on positions of the set of closest points.

In some embodiments, in response to determining that a count of the one or more reference duct skeletons exceeds 1, i.e., the reference duct skeleton set includes a plurality of duct skeletons, one or more candidate duct skeleton may be determined based on the set of closest points, and the duct type of one of the at least one duct of the undetermined type may be determined based on the one or more candidate duct skeletons. For example, a generalized distance between the second duct skeleton and a duct skeleton of the one or more candidate duct skeletons may be determined, and the duct type of the second duct skeleton maybe determined based on the generalized distance.

More descriptions regarding determining the type of one of the at least one duct of the undetermined type based on the set of closest points may be found in the related descriptions in FIG. 20.

In some embodiments, the type of one of the at least one duct of the undetermined type may be determined based on other relationships between the second duct skeleton of one of the at least one duct of the undetermined type and the reference duct skeleton set. For example, the duct type of the second duct skeleton may be determined based on a spatial relationship, a topological relationship, etc. between the second duct skeleton and the reference duct skeletons in the reference duct skeleton set. In some embodiments, the duct type of the second duct skeleton may be determined based on a distance and an angle between the second duct skeleton of the undetermined duct and the reference duct skeleton.

It should be noted that the above description of the process 1900 is only for example and explanation, and does not limit the scope of application of the present disclosure. For those skilled in the art, various modifications and changes can be made to the process 1900 under the guidance of the present disclosure. However, such modifications and changes are still within the scope of the present disclosure.

FIG. 20 is a flowchart illustrating an exemplary process of determining a duct type according to some embodiments of the present disclosure. In some embodiments, a process 2000 may be performed by the system 100 for puncture path planning (e.g., the processing device 130 of the system 100 for puncture path planning) or the device 1600 for duct recognition. For example, the process 2000 may be stored in a storage device (e.g., the storage device 150, and the storage unit of the system) in the form of a program or instruction. The process 2000 may be implemented when a processor or a module shown in FIG. 16 executes the program or instruction.

As shown in FIG. 20, a duct type of a second duct skeleton may be determined in various ways based on a count of duct skeletons in a reference duct skeleton set. In operation 2010, whether the reference duct skeleton set includes one reference duct skeleton is determined. If the reference duct skeleton set includes one reference duct skeleton, the operation 2020 may be performed; otherwise, operation 2030 may be performed.

In 2020, a duct type of a second duct skeleton may be determined based on positions of a set of closest points.

In some embodiments, when the reference duct skeleton set includes only one duct skeleton, i.e., there is only one reference duct skeleton, the processing device 130 may determine the duct type of the second duct skeleton based on the positions of the set of closest points between the second duct skeleton of one of the at least one duct of the undetermined type and the reference duct skeleton.

In some embodiments, the duct type of the second duct skeleton may be determined based on a positional relationship between the positions of the set of closest points and an endpoint of the skeleton. The endpoint of the skeleton refers to a point with only one adjacent point on the skeleton. In some embodiments, if there is a point (e.g., a point AAA) in the set of closest points of which a closest distance to any endpoint of the skeleton is less than a preset value n1, the second duct skeleton and the reference duct skeleton may be considered to be the same type of duct. Based on different calculation methods of the spatial distance, the preset value n1, as a comparison parameter of the spatial distance, may be different physical quantities. For example, when an actual spatial distance is used as the basis for distance measurement, the preset value n1 may be a physical quantity specifically representing a length, such as 5 mm. In some embodiments, the calculation of the spatial distance may be performed based on voxel points in image information after conversion. For example, if the actual distance value is converted into five voxel points, the preset value n1 may be five. In some embodiments, if a projection angle of a 3D image is consistent, the actual distance value may be converted into a count of pixels, and the preset value n1 may be expressed by the count of pixels. For example, if the actual distance value is converted into five pixels, the preset value n1 may be five.

In some embodiments, the preset value n1 may be obtained based on experience or demand. In some embodiments, the preset value n1 may be customized by a user. In some embodiments, the preset value n1 may be different based on a level of one of the at least one duct of the undetermined type. For example, the lower the level of the duct, the smaller the preset value n1; the higher the level of the duct, the larger the preset value n1. In some embodiments, the preset value n1 may be related to a thickness of one of the at least one duct of the undetermined type. For example, the thinner the duct, the smaller the preset value n1; the thicker the duct, the larger the preset value n1.

In some embodiments, the preset value n1 may be obtained by a machine learning method. For example, by constructing a machine learning model, an optimized preset value n1 corresponding to portions of organisms may be obtained by machine learning based on training data of the portions of different organisms. In practical application, when the portion is recognized, the corresponding preset value n1 obtained after optimization training may be used. The machine learning model may include but is not limited to one or more of a neural network model, a support vector machine model, a k-nearest neighbor model, a decision tree model, or the like, or any combination thereof. In some embodiments, the machine learning method of the second threshold may be obtained based on medical images of the portions corresponding to the same type of organisms and type determination results. For example, the preset value n1 of the organism may be obtained through training using the medical images of the portions corresponding to the same type of organisms as samples, and the type determination results as labels.

Merely by way of example, as shown in FIG. 21(a) and FIG. 21(b), in the set of closest points (AAA and CCC), a skeleton where AAA is located may be the undetermined skeleton 2110, and a skeleton where CCC is located may be the reference duct skeleton 2120. If a distance between the AAA in the set of closest points and an endpoint of the skeleton 2110 is 0 pixel and within n1 pixels, and a distance between the point CCC and an endpoint of the skeleton 2120 is 0 pixels and within n1 pixels, the duct of the undetermined skeleton 2110 and the duct of the reference duct skeleton 2120 may be considered to be of the same type.

As another example, as shown in FIGs. 21(c)-(e), FIG. 21(c) is a reconstructed local 3D image at a top-down angle, FIG. 21(d) is a skeleton simulation image with the same viewing angle corresponding to FIG. 21(c), and FIG. 21(e) is a duct skeleton simulation image at a side-view angle corresponding to FIG. 21(c). In FIG. 21(c), two ducts may be on different planes in space (the same applies to ducts on the same plane), and a minimum spatial distance between the two ducts may be less than the second threshold. As shown in FIG. 21(d), in the set of closest points (AAA' and CCC'), a skeleton where the AAA' is located may be a dark-colored duct skeleton 2140, and a skeleton where the CCC' is located may be a light-colored duct skeleton 2130. The AAA' may block the CCC', i.e., a connection line between the CCC' and the AAA' may be perpendicular to a paper surface. As shown in FIG. 21(e), a dotted line represents a distance from the AAA' to the CCC'. In the set of closest point groups (AAA' and CCC'), if a distance between the AAA' and an endpoint of a skeleton 2140 is 0 pixel and is within n1 pixels, and a distance between the CCC' and an endpoint of the skeleton 2130 is 0 pixel and within n1 pixels, the duct corresponding to the skeleton 2130 and the duct corresponding to the skeleton 2140 may be considered to be the same type.

As another example, as shown in FIGs. 21(f)-(i), FIG. 21(f) is a reconstructed local 3D image at a top-view angle, FIG. 21(g is a skeleton simulation image with a consistent viewing angle corresponding to FIG. 21(f), FIG. 21(h) is a local 3D image at a side-view angle of FIG. 21(f), and FIG. 21(i) is a skeleton simulation image with a consistent viewing angle corresponding to FIG. 21(h). In FIG. 21(h) and FIG. 21(f), two ducts may be located on different planes in space (the same applies to ducts on the same plane). In FIG. 21(g), a skeleton where the AAA" is located may be a dark-colored duct skeleton 2150, and a skeleton where the CCC" is located may be a light-colored duct skeleton 2160. The point AAA" may block the point CCC", i.e., a connection line between the point CCC" and the point AAA" may be perpendicular to the paper surface. In FIG. 21(i), the dotted line represents a distance from the point AAA" to the point CCC". In the set of closest points (AAA" and CCC"), the AAA" and the CCC" may both be located in the middle of the respective skeletons, not near the endpoint. In this case, it is considered that the two ducts corresponding to the skeleton 2150 and the skeleton 2160, respectively may not be of the same type.

In 2030, one or more candidate duct skeletons may be determined based on the set of closest points, and a duct type of the second duct skeleton may be determined based on the one or more candidate duct skeletons.

When the reference duct skeleton set includes more than one duct skeleton, the duct type of the second duct skeleton may be determined based on a spatial relationship between the reference duct skeleton in the reference duct skeleton set and the second duct skeleton of one of the at least one duct of the undetermined type.

In some embodiments, when the reference duct skeleton set includes more than one duct skeleton, the one or more candidate duct skeletons may be determined from the reference duct skeleton set based on the set of closest points, i.e., only the reference duct skeletons that are suspected to be of the same category as the duct skeleton of the undetermined type may be retained. In combination with the discrimination method in the operation 2020, in some embodiments, the one or more candidate duct skeletons may be determined based on the set of closest points by determining whether each reference duct skeleton is of the same type as the second duct skeleton. For example, if there is a point MMM in the set of closest points between the reference duct skeleton and the second duct skeleton, and a minimum distance between the MMM and any endpoint of a skeleton in which the MMM is located is less than the preset value n1, the second duct skeleton may be suspected to be of the same category as the reference duct skeleton, and the reference duct skeleton may be determined as a candidate duct skeleton.

If the one or more candidate duct skeletons include only one duct skeleton, a duct type of the candidate duct skeleton (i.e., the reference duct skeleton suspected to be of the same category as the second duct skeleton) may be determined as the duct type of one of the at least one duct of the undetermined type. If the one or more candidate duct skeletons include a plurality of duct skeletons, and these duct skeletons are all of the same duct type, the duct type of these reference duct skeletons may be determined as the duct type of one of the at least one duct of the undetermined type. If the one or more candidate duct skeletons include a plurality of duct skeletons, and at least two of these duct skeletons do not belong to the same duct type, a generalized distance between the second duct skeleton and the one or more candidate duct skeletons may be determined; and the duct type of one of the at least one duct of the undetermined type may be determined based on the generalized distance.

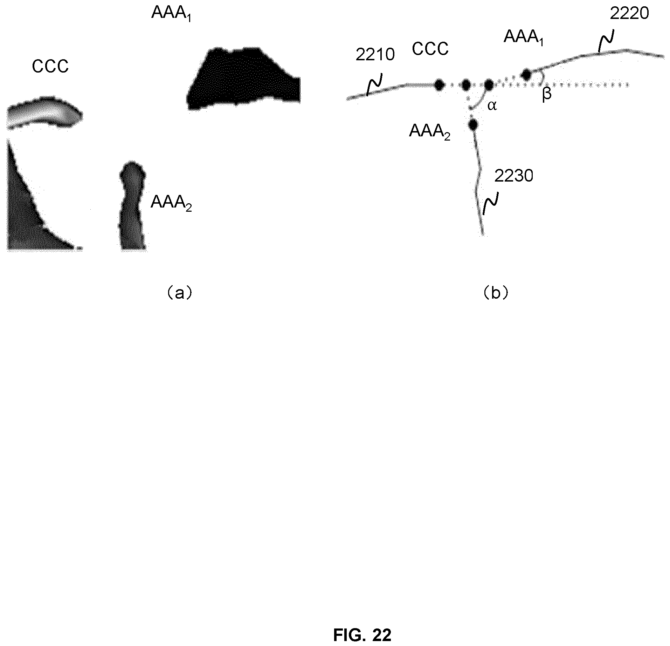

The generalized distance refers to a physical quantity that reflects a degree of proximity (e.g., a degree of distance proximity, a degree of directional proximity) between skeletons. In some embodiments, the generalized distance may be obtained based on the minimum spatial distance and a generalized angle. The generalized angle refers to a physical quantity that reflects the degree of directional proximity between the skeletons, such as angles α and β in FIG. 22(b).

In some embodiments, the generalized angle may be obtained based on a generalized angle of the set of closest points. Specifically, a point of the set of closest points may be used as a tangent point, and tangent lines of a skeleton where the point is located may be made, and an angle between the tangent lines may be determined as the generalized angle. For example, as shown in FIG. 22(b), if the one or more candidate duct skeletons corresponding to a second duct skeleton 2210 include two candidate duct skeletons: a reference duct skeleton 2220 and a reference duct skeleton 2230, for sets of closest points (AAA₁ and CCC) and (AAA₂ and CCC), a tangent line of the second duct skeleton 2210 where the point CCC is located may be made using the point CCC as the tangent point, a tangent line of the reference duct skeleton 2220 where the point AAA₁ is located may be made using the point AAA₁ as the tangent point, and a tangent line of the reference duct skeleton 2230 where the point AAA₂ is located may be made using the point AAA₂ as the tangent point. An angle (e.g., α, β) between the tangent lines corresponding to each of the sets of closest points may be determined as the generalized angle.

In some embodiments, if a point in the set of closest points is located at a bifurcation point of the skeleton, tangent lines of skeleton branches may be made using the bifurcation point used as the tangent point. A midline of each tangent line may be calculated, and the midline may be used as a tangent line of the skeleton at the bifurcation point.

In some embodiments, the generalized angle may be obtained in other ways. For example, a fitting straight line of each skeleton may be made, and an angle of the fitting straight lines may be used as the generalized angle.

Merely by way of example, FIGs. 22(a)-(b) shows a method for obtaining a distance based on the spatial distance and the generalized angle. FIG. 22(a) is a reconstructed local 3D image, and FIG. 22(b) is a skeleton simulation diagram corresponding to FIG. 22(a). For the convenience of explanation, three ducts in FIG. 22(a) are on the same plane in space (the same applies to ducts on different planes), and there are two reference duct skeletons suspected to be of the same category as the second duct skeleton 2210 (i.e., the undetermined duct skeleton), i.e., a reference duct skeleton 2220 and a reference duct skeleton 2230. That is, the one or more candidate duct skeletons include two candidate duct skeletons, and the sets of closest points of the two reference duct skeletons and the second duct skeleton 2210 may be (AAA₁ and CCC) and (AAA₂ and CCC), respectively. If a distance weight is f1 and an angle weight is f2 (e.g., f1=0.4, f2=0.6), a score of the reference duct skeleton 2220 may be S1 = f1 × distance (AAA₁, CCC) + f2 × β, and a score of the reference duct skeleton 2230 may be S2 = f1 × distance (AAA₂, CCC) + f2 × α. The processing device 130 may determine a type of the reference duct skeleton with the smallest score as the duct type of the second duct skeleton 2210. For example, if S1 is smaller, the duct type of the second duct skeleton 2210 may be consistent with that of the reference duct skeleton 2220.

The recognition accuracy can be improved by determining the type of the ducts in the organism through the connectivity, the set of closest points, and the generalized distance.

It should be noted that the above description of the process 2000 is only for example and explanation, and does not limit the scope of application of the present disclosure. For those skilled in the art, various modifications and changes can be made to the process 2000 under the guidance of the present disclosure. However, such modifications and changes are still within the scope of the present disclosure.

FIG. 23i is a schematic diagram illustrating an exemplary process of model training according to some embodiments of the present disclosure. In some embodiments, a process 2300 may be performed by the system 100 for puncture path planning (e.g., the processing device 130 of the system 100 for puncture path planning system 100) or the device 1600 for duct recognition (e.g., a training module). For example, the process 2300 may be stored in a storage device (e.g., the storage device 150, and the storage unit of the system) in the form of a program or instruction. The process 2300 may be implemented when the processor or the module shown in FIG. 16 executes the program or instruction.

As shown in FIG. 23, in some embodiments, an initial model 2310 may be trained based on a large number of labeled training samples to update parameters of the initial model to obtain a trained model 2320. The initial model 2310 may include an initial first segmentation model and/or an initial second segmentation model. Accordingly, the trained model 2320 may include a first segmentation model and/or a second segmentation model.

In some embodiments, the initial first segmentation model may be trained based on a large number of first training samples to update parameters of the initial first segmentation model to obtain the first segmentation model. In some embodiments, the first training samples may be input into the initial first segmentation model. The parameters of the initial first segmentation model may be updated through training iterations.

The first training samples may include historical target images for training the first segmentation model. The historical target images may include historical 3D medical image. Sample target images in the first training samples may be used as an input of the training model, and duct types of ducts in the sample target images may be used as labels. The duct type may include at least a first type and ae second type. There may be a third type or even more. For example, the duct type may include an abdominal portal vein and an abdominal artery. As another example, the duct type may include a hepatic portal vein, a hepatic vein, and a hepatic artery. In some embodiments, the first type of ducts in the sample target images may be labeled with a first grayscale value, the second type of ducts may be labeled with a second grayscale value, the third type of ducts may be labeled with a third grayscale value, etc. It is noted that the above labels only include the duct types of the ducts in the sample target images, and do not include levels of the ducts.

In some embodiments, the first training samples may only calibrate types of ducts that meet a condition. For example, the condition may include a preset range of contrast of the ducts in the image, a preset range of the duct level, or the like, or any combination thereof. In some embodiments, the condition may be set based on experience or demand. For example, different types of organisms, different portions, organs, tissues, etc. may correspond to different conditions. In some embodiments, the condition may be set by a user. In some embodiments, the condition may be that the levels of the ducts are less than a set level.

The levels of the ducts (e.g., blood vessel) refer to a relative relationship between the ducts and a primary duct. For example, the fewer branches that are from the primary duct to the duct, the smaller the level of the duct. For the thoracic artery, the thoracic aorta may be a level 1 duct, the primary pulmonary arteries on both sides may be level 2 ducts, the lobar arteries may be level 3 ducts, the segmental arteries may be level 4 ducts, the subsegmental pulmonary arteries may be level 5 ducts, and the subsegmental pulmonary arteries may be level 6 ducts. For the hepatic portal vein, the primary hepatic portal vein may be a level 1 duct, the left/right branch of the hepatic portal vein may be a level 2 duct, the hepatic lobar portal vein may be a level 3 duct, the segmental hepatic portal vein may be a level 4 duct, the subsegmental hepatic portal vein may be a level 5 duct, and the subsegmental hepatic portal vein may be a level 6 duct. For the hepatic vein, the primary hepatic vein may be a level 1 duct, the left/right branch of the hepatic vein may be a level 2 duct, the hepatic lobar vein may be a level 3 duct, the segmental hepatic vein may be a level 4 duct, the subsegmental hepatic portal vein may be a level 5 duct, and the subsegmental hepatic portal vein may be a level 6 duct. For the hepatic artery, the primary hepatic artery may be a level 1 duct, the left/right branch of the hepatic artery may be a level 2 duct, the hepatic lobar artery may be a level 3 duct, and the segmental hepatic artery may be a level 4 duct.

In some embodiments, the level of the duct may reflect the richness of an image or a detection result. For example, the greater the level, the better the richness. For example, a detection result containing ducts with a maximum level of 6 may be richer than a detection result containing ducts with a maximum level of 4.

The set level can be a preset level of the duct, such as level 5. The set level may be configured to guide ducts that need to be labeled (e.g., blood vessels with a level less than 5) and ducts that do not need to be labeled (e.g., blood vessels with a level greater than or equal to 5). The set level may be set according to demand and/or experience. In some embodiments, the set level may be set by a user.

Only labeling the ducts with levels lower than the set level is beneficial for the first segmentation model to focus on the segmentation and classification of the primary duct, thereby improving the accuracy of segmentation.

In some embodiments, an initial second segmentation model may be trained based on a large number of second training samples to update parameters of the initial second segmentation model to obtain the second segmentation model. In some embodiments, the second training samples may be input into the initial second segmentation model to update the parameters of the initial second segmentation model through training iterations.

The second training samples refer to sample target images used to train the second segmentation model. The sample target images may include historical 3D image data. In some embodiments, the sample target images in the second training samples may be used as an input of the training model, and ducts in the sample target images may be used as labels. For example, contours of the ducts in the sample target images may be circled. It is noted that the labels may include only the ducts (e.g., the blood vessels), and do not include types of the ducts (e.g., the hepatic portal vein, the hepatic vein, the hepatic artery, etc.).

In some embodiments, for example, in an embodiment where the sample target images are CT image data, the sample CT image data may be processed by adjusting a window width (a range of a CT value displayed on a CT image), a window position (a central value of the CT value), etc. to increase a grayscale difference between the structures in the image and/or enhance the contrast of small ducts, such that the labeling results of the first training samples and/or the second training samples may be more accurate (e.g., covering as many small ducts as possible, such that the second training samples may cover more levels of ducts). The labels of the first training samples and/or the second training samples may be added manually or automatically, or in other ways, which is not limited in this embodiment.

As mentioned above, in some embodiments, the first training samples may only label the types of the ducts that meet the condition. In some embodiments, at least one duct in the organism that does not meet the condition may be labeled in the second training samples. In other words, compared with the first training samples, the second training samples may label more (deeper bifurcations, and smaller) ducts. For example, if the set condition is that the levels of the ducts in the organism are less than level 5, the first training samples may only label the types of ducts of levels 1-4, while the second training samples may label ducts of levels 1-6 or even smaller. Covering as many small ducts as possible, and covering the ducts not covered by the first training samples is conducive to the second segmentation model learning the features of small ducts, thereby improving the richness of the segmentation.

In some embodiments, the plurality of first training samples and/or second training samples, including the corresponding labels, may be obtained by reading from a database or a storage device or calling a data interface.

In some embodiments, the sample target images of the first training samples may be input into the first segmentation model, and prediction results of the ducts in the sample target images may be obtained from the output of the first segmentation model; and/or the sample target images of the second training samples may be input into the second segmentation model, and the prediction results of the ducts in the sample target images may be obtained from the output of the second segmentation model.

In some embodiments, the processing device may construct a loss function based on the prediction results and the labels of the first training samples (or the second training samples). The loss function may reflect ae difference between the prediction results and the labels. The processing device may adjust the parameters of the first segmentation model (or the second segmentation model) based on the loss function to reduce the difference between the prediction results and the labels. For example, by continuously adjusting the parameters of the first segmentation model or the second segmentation model, the value of the loss function may be reduced or minimized.

In some embodiments, the first segmentation model and/or the second segmentation model may also be obtained according to other training methods. For example, a corresponding initial learning rate (e.g., 0.1) and a learning rate decay strategy may be set for the training process, which is not limited in the present disclosure.

It should be noted that the above description of the process 2300 is only for example and explanation, and does not limit the scope of application of the present disclosure. For those skilled in the art, various modifications and changes can be made to the process 2300 under the guidance of the present disclosure. However, such modifications and changes are still within the scope of the present disclosure.

FIG. 24 is a flowchart illustrating an exemplary method for puncture path planning according to some embodiments of the present disclosure. In some embodiments, a process 2400 may be performed by the system 100 for puncture path planning system (e.g., the processing device 130 of the system 100 for puncture path planning) or the device 300 for puncture path planning. For example, the process 2400 may be stored in a storage device (e.g., the storage device 150, and the storage unit of the system) in the form of a program or instruction. The process 2400 may be implemented when the processor or the module shown in FIG. 3 executes the program or instruction. As shown in FIG. 24, in some embodiments, the process 2400 may include the following operations.

In 2410, a target point may be determined based on a target image. In some embodiments, the operation 2410 may be performed by the processing device 130 or the data preprocessing module 310.

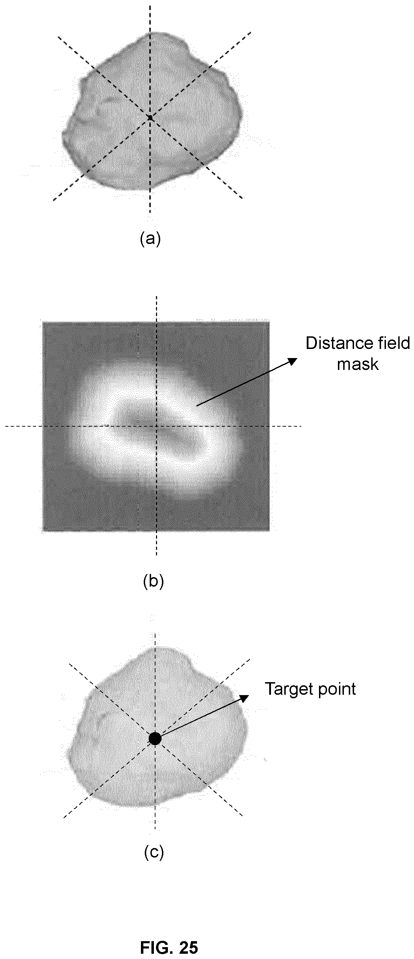

Referring to the above, the target point may be a volume center or a center of gravity of a lesion region or a region to be detected. In some embodiments, after organ or tissue segmentation (e.g., the process 600 is performed) is performed, the volume center or the center of gravity of a target organ may be determined in various ways. Merely by way of example, taking the puncture of the lesion region as an example, the processing device 130 may continuously erode a periphery of the lesion region inward by a boundary erosion mode to obtain a distance field, determine a voxel farthest from the boundary as the center of the lesion region, and determine the center as the target point. Specifically, the processing device 130 may (1) obtain a minimum distance value of three spaces X, Y, and Z in an original scale of the target image, resample the image based on the scale, and obtain a resampled image (e.g., an image shown in FIG., 25(a)); (2) perform recursive erosion using the boundary erosion mode, and calculate a minimum distance from the eroded voxel to the boundary according to a count of erosion times to form a distance field mask corresponding to the lesion region (e.g., a light gray irregular region with an approximate ellipse shown in FIG. 25 (b)); (3) calculate a maximum value of the distance field, when a count of voxels of the maximum value of the distance field is 2, calculate an average value for the neighboring 5*5*5 cubes of the voxel, and determine a point with the largest average value as the target point; when the count of voxels of the maximum value of the distance field is greater than 2, determine a minimum value of a sum of distances between the current voxel and the voxel points with a maximum boundary distance as a target function, and determine a voxel point corresponding to a value obtained by solving the target function as the target point (e.g., a black point shown in a central region of FIG. 25 (c)).

It can be understood that the above description regarding the determination of the target point is only an example and is not a limitation of the present disclosure. In some embodiments, the target point can be determined by other reasonable and feasible methods (e.g., directly determining the volume center of the target organ as the target point by an image recognition method, or determining an intersection of a major axis and a minor axis of the volume of the target organ as the target point, or determining the volume center as the target point by pixel statistics, etc.), which is not limited in the present disclosure.

In 2420, initial paths may be determined based on the target point and a first constraint. In some embodiments, the operation 2420 may be performed by the processing device 130 or the path screening module 320.

In some embodiments, the first constraint may include at least one of the following: the path is located in a slice layer adjacent to a slice layer where a target region is located, a needle entry point on a body contour that contacts a bed board is excluded, a puncture depth of the path is less than a preset depth threshold, or an angle between the path and a vertical line of a flat surface of a flat lesion is within a preset range, etc. For example, the first constraint may include that the path is located in the slice layer adjacent to the slice layer where the target region is located, the needle entry point on the body contour that contacts the bed board is excluded, and the puncture depth of the path is less than the preset depth threshold. As another example, the first constraint may include that the path is located in the slice layer adjacent to the slice layer where the target region is located, the needle entry point on the body contour that contacts the bed board is excluded, the puncture depth of the path is less than the preset depth threshold, and the angle between the path and the vertical line of the flat surface of the flat lesion is within the preset range. As another example, the first constraint may include that the path is located in the slice layer adjacent to the slice layer where the target region is located, the needle entry point on the body contour that contacts the bed board is excluded, or the puncture depth of the path is less than the preset depth threshold.

The target region refers to a region where the target organ is located. In some embodiments, the slice layer where the target region is located reflects a position (e.g., in a CT scan image, the target region may be one or more slice layers of the scan image) of the target region in the target image. The adjacent slice layers of the slice layer where the target region is located refer to adjacent slice layers located within a certain range of the slice layer where the target region is located.

By constraining the puncture path to be positioned in the slice layer adjacent to the slice layer where the target region is located, it is possible to avoid affecting the guidance evaluation of the puncture operation of the user (e.g., a doctor, and a nurse) caused by the inability to simultaneously observe the positions of the "needle head" and the "needle tail" in a scan image acquired during a puncture operation due to the situation that the target point and the needle entry point of the puncture path span too large a slice layer in a head-to-foot direction.

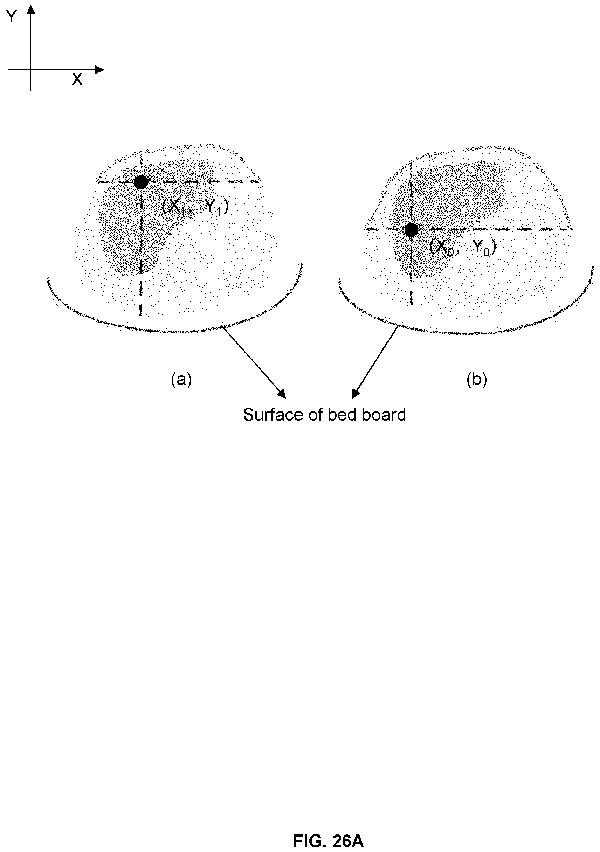

The hospital bed refers to a platform (e.g., a medical bed 115) on which a target object (e.g., a patient) lies when the puncture operation is performed. In some embodiments, a position of the needle entry point may be determined based on the target image/segmented image, and the needle entry point on the body contour that contacts with the bed board may be excluded. For example, the processing device 130 may determine the position of the bed board according to a lying posture of the patient in the target image (e.g., based on image segmentation recognition or posture feedback positioning of a hardware system, etc.), and calculate the position of the needle entry point according to the position of the bed board. Merely by way of example, FIG. 26A may be simply understood as a side view, where a bed surface is perpendicular to a paper surface. Assuming that the patient lies flat or lies on the stomach on the bed, the processing device 130 may establish a coordinate system with a horizontal right direction of the paper surface as a positive direction of an X-axis and a vertical upward direction as a positive direction of a Y-axis to calculate the position of the needle entry point and the position of the target point (e.g., a midpoint (X₁, Y₁) in FIG. 26A(a) or a midpoint (X₀, Y₀) in FIG. 26A(b)). When an ordinate of the needle entry point is greater than an ordinate of the target point (e.g., greater than Y₁ or Y₀), the corresponding needle entry point may be determined to be a positive needle entry point (i.e., a needle entry point on the body contour that does not contact with the bed board); otherwise, the corresponding needle entry point may be determined to be a reverse needle entry point (i.e., the needle entry point on the body contour that contacts with the bed board), and may be excluded.

By excluding the needle entry point on the body contour that contacts with the bed board, it is possible to avoid the planned path being impractical and impossible to execute due to needle entry from the side of the bed board, thereby improving the efficiency and accuracy of puncture path planning.

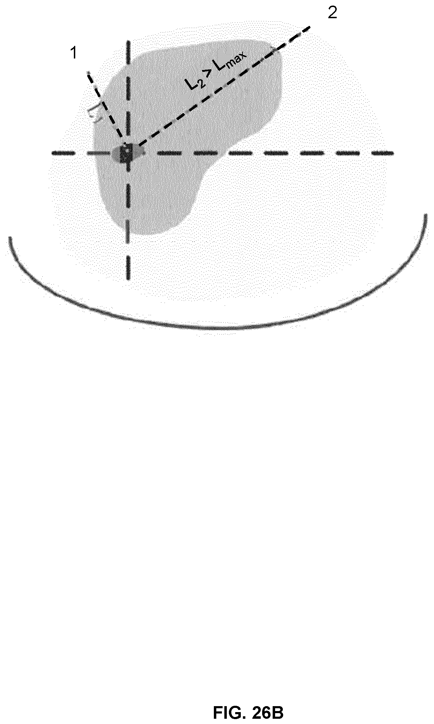

The puncture depth of the path may be a puncture distance from the needle entry point to the target point. In some embodiments, the initial path may be constrained to a puncture distance less than a preset depth threshold. In some embodiments, the preset depth threshold may be determined based on a length (e.g., a model length of a commonly used clinical instrument for puncture surgery) of the puncture needle. For example, a length of a longest puncture needle (e.g., a 120 mm puncture needle) supported by the system may be determined as the preset depth threshold, or a length of a medium puncture needle may be determined as the preset depth threshold, or a length of the shortest puncture needle may be determined as the preset depth threshold. In some embodiments, the preset depth threshold may be determined based on puncture information and/or patient information. For example, the puncture information may include target organ information, puncture purpose, etc.; the patient information may include patient age, gender, etc. Merely by way of example, when the target organ contains a relatively dangerous tissue (e.g., a blood vessel, a bone, etc.), the puncture purpose may be lesion detection, or when the patient is elder, the processing device 130 may determine a relatively small value (e.g., 3-5 mm plus the shortest distance between a skin layer and the target organ) as the preset depth threshold. As another example, the processing device 130 may determine a puncture needle model (e.g., a length and a diameter of the puncture needle) according to the target organ information, the puncture purpose, and other information, and determine the length of the puncture needle as the preset depth threshold according to the puncture needle model. In some embodiments, the planning of the initial path may be constrained based on a distance between the needle entry point and the target point. Merely by way of example, in FIG. 26B, 1 represents a path where a puncture depth L₁ is less than the preset depth threshold Lₘₐₓ, and 2 represents a path where a puncture depth L₂ is greater than the preset depth threshold. The processing device 130 may determine the path 1 as the initial path.

By excluding the paths where the puncture depth is greater than the preset depth threshold based on the length of the puncture needle, the puncture information, etc., the situation that puncture needle is prevented from reaching the target point due to needle model limitations can be avoided, the time the puncture needle stays and the distance the puncture needle passes in the human body can also be reduced, thereby reducing the risk of complications caused by the puncture.

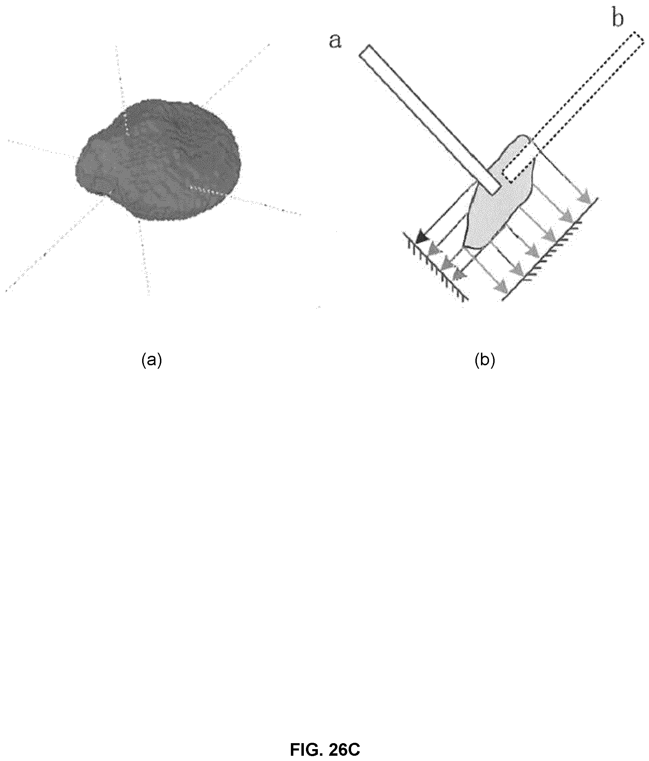

The flat lesion refers to a lesion (e.g., a lesion morphology shown in FIG. 26C) with a small volume and flat features. In some embodiments, the lesion morphology may be determined by pixel statistics, principal component analysis, image recognition, or the like. Merely by way of example, the processing device 130 may calculate directions and eigenvalues (r₀, r₁, r₂) of three principal axes X, Y, and Z by performing matrix decomposition based on spatial distribution points of lesion voxels in the target image or the segmented image. When 1 ≤ r₀/r₁ ≤2 and r₁/r₃ ≥ 3, the current lesion may be determined as the flat lesion. The eigenvalues r₀ ≥ r₁ ≥ r₂, and a size of the eigenvalue indicates the contribution of the corresponding eigenvector to the entire matrix after the matrix is orthogonalized (i.e., the description of an object size by the (x, y, z) value representing the object size in the coordinate system).

In some embodiments, when the lesion is flat, the puncture path may be constrained to be that an angle between the path and a vertical line of a flat surface of the flat lesion is within a preset range. In some embodiments, the flat surface of the flat lesion may be determined by plane projection, image recognition, pixel statistics, threshold segmentation, or the like. In some embodiments, the preset range may be any reasonable angle range, and the processing device 130 may determine the preset range based on parameters such as an area of the flat surface and a diameter of the puncture needle, which is not limited in the present disclosure. For example, the preset range may be [0°, 10°], [0°, 15°], [0°, 20°], [0°, 40°], [5°, 15°], [3°, 20°], [5°, 35°], [10°, 30°], [25°, 50°], or [0°, 60°], etc.

In some embodiments, paths of which angles between the path and the vertical line of the flat surface of the flat lesion are within the preset range may be determined based on a ratio (i.e., determining whether a cylinder formed by the puncture path contains most of the volume of the target organ) of a count of point clouds within a path projection surface to a count of point clouds within a flat lesion projection surface. In some embodiments, the processing device 130 may, (1) obtain a needle insertion direction corresponding to the current path; (2) calculate an equation of a projection plane perpendicular to the path according to the needle insertion direction; (3) obtain corresponding lesion projection point clouds and projection points of the target point by projecting the coordinates corresponding to the lesion region and the coordinates of the target point based on the equation of the projection plane; (4) draw a circle with the target projection point as a center and a safety radius(e.g., a preset distance threshold between the path and the dangerous region) of the path as a radius, and calculate a ratio of a count of projection point clouds in the circle to a total count of the lesion projection point clouds; wherein when the ratio is greater than a preset ratio (e.g., 0.6, 0.7, etc.), it means that most of the puncture lesion region along the direction may be on the puncture path, and the angle between the path and the vertical line of the flat surface of the flat lesion may be within the preset range (e.g., a path b in FIG. 26C(b)), and the path may be excluded; when the ratio is less than or equal to the preset ratio, it means that the angle between the path and the vertical line of the flat surface of the flat lesion may not be within the preset range (e.g., a path a in FIG. 26C(b)).

By constraining the puncture path to be that the angle between the path and the vertical line of the flat surface of the flat lesion is within the preset range, the puncture path of the flat lesion can be made to be punctured from a "big end" direction (i.e., a direction of the vertical line of the flat surface), while the puncture path is as perpendicular to the flat surface of the lesion as possible, meeting the clinical needs, and specifically determining a path with a shorter puncture depth and better effect, thereby improving the feasibility of the puncture path and the convenience of puncture, and ensuring the reliability of the sampling result/lesion puncture result.

In some embodiments, the initial paths that satisfy the first constraint may be determined in any reasonable order. For example, first initial paths located in the slice layer adjacent to the slice layer where the target region is located may be determined, and then paths of which the needle entry point on the body contour that contacts with the bed board in the first initial paths may be excluded to obtain second initial paths; further, paths of which puncture depths are less than the preset depth threshold are determined from the second initial paths as final initial paths. As another example, the first initial paths may be determined by first excluding the needle entry point on the body contour that contacts with the bed board, and then the paths located in the slice layer adjacent to the slice layer where the target region is located may be determined from the first initial paths as the final initial paths.

In 2430, one or more candidate paths may be determined from the initial paths based on a second constraint. In some embodiments, the operation 2430 may be performed by the processing device 130 or the path screening module 320.

In some embodiments, the second constraint may include that a distance between a path and a dangerous region is greater than a preset distance threshold.

The dangerous region refers to a region containing a dangerous tissue (e.g., a blood vessel, a bone, etc.). In some embodiments, an internal tissue of a target organ may be classified according to a result of tissue segmentation (e.g., the tissue segmentation may be achieved by performing the process 600) or a result of duct recognition (e.g., duct recognition may be achieved by performing process 1700), and the dangerous region may be determined based on a classification result and the path planning conditions (e.g., constraints). For example, the processing device 130 may give priority to all blood vessels that do not pass through the target organ (i.e., all the blood vessels may be determined as dangerous tissues) according to an average diameter of blood vessel segments. If no effective path is obtained in this case or the effective paths obtained are less, the influence of thin blood vessels may be weakened, and the thin blood vessels inside the target organ may be set as puncturable tissues (i.e., thick blood vessels may be determined as the dangerous tissues) to perform path planning. Specifically, the processing device 130 may obtain a blood vessel mask by first segmenting the blood vessels in the target organ by a deep learning method or the process 600 of image segmentation; then calculate blood vessel centerlines by corroding a boundary mask inward, determine points that cannot be further corroded as central points of the blood vessels, and calculate a minimum distance from the current central point to the boundary of the blood vessel and use the minimum distance as a blood vessel radius of the point; further, use intersections of central points of blood vessel branches as "nodes", and regard the blood vessel segments between the nodes as "edges", calculate the nodes and the associated blood vessel segments by a graph theory method, and obtain each segment of blood vessel branch by performing growth on the blood vessel masks between the nodes; finally, compare an average diameter of each blood vessel segment with a threshold Dt (e.g., 1 mm, and 2 mm ) for distinguishing the thickness of the blood vessels, and if the average diameter of each blood vessel segment is less than the threshold Dt, determine the blood vessel segment as a thin blood vessel, and if the average diameter of each blood vessel segment is greater than the threshold Dt, determine the blood vessel segment as a thick blood vessel, distinguish the thin blood vessels and the thick blood vessels by different labeling values, and refresh all the blood vessel segments to determine the dangerous region accordingly. For example, a region containing only the thick blood vessels may be determined as the dangerous region, or a region containing the thin blood vessels and the thick blood vessels may be determined as the dangerous region.

The preset distance threshold may be the shortest distance from an edge of the dangerous tissue to the path. In some embodiments, the preset distance threshold (e.g., 2 mm, 3 mm, 5 mm, or 7 mm, etc.) may be determined based on one or more parameters such as a distance between tissues, a tissue segmentation error, a registration error between the planned puncture and the actual puncture, and an execution error of an end effector (e.g., the end effector 120).

By constraining the distance between the puncture path and the dangerous region to be than the preset distance threshold, secondary injury to the patient caused by accidental injury to other tissues during the puncture due to the puncture path being too close to the dangerous tissues such as the blood vessels can be avoided.

In some embodiments, in the process of determining the one or more candidate paths, a path planning condition (e.g., the second constraint) may be adaptively adjusted based on a first preset condition. The path planning condition reflects a screening condition (e.g., a range of the dangerous region and/or the preset safety distance value) of the one or more candidate paths. In some embodiments, the adaptively adjusting the path planning condition based on the first preset condition may include: when a ratio of a count of the one or more candidate paths to a count of initial paths is less than a third threshold, adjusting the range of the dangerous region. The third threshold represents a change control coefficient (e.g., 0.2, and 0.3) of the dangerous tissue. For example, if the count of the initial paths is N₁, all the blood vessels are set as dangerous tissues in an initial path planning condition, and the count of the one or more candidate paths determined based on this screening is N₂, when N₂/N₁ ≤ H₁ (i.e., the third threshold), it means that most of the initial paths intersect with the dangerous tissues within the safety range, and the range of the dangerous region may be changed in this case (e.g., modifying label values of the blood vessels, setting the blood vessels of which diameters are less than 1.5 mm as penetrable tissues, and removing the blood vessels from the dangerous region).

In some embodiments, the one or more candidate paths may be determined from the initial paths based on the adjusted dangerous region. In response to determining that a ratio of the count of the one or more candidate paths obtained before adjustment to the count of the one or more candidate paths obtained after adjustment is less than a fourth threshold, the one or more candidate paths obtained after adjustment may be used as final candidate paths. In response to determining that the ratio of the count of the one or more candidate paths obtained before adjusting the range of the dangerous region to the count of the one or more candidate paths obtained after adjusting the range of the dangerous region is greater than the fourth threshold, the one or more candidate paths obtained before adjustment may be used as the final candidate paths. For example, according to the dangerous region determined when the blood vessels of which the diameters are less than 1.5 mm are set as the penetrable tissues (i.e., not included in the dangerous region), the initial paths of which distances from the dangerous region are greater than the preset distance threshold may be determined again, and a count N₃ of the one or more candidate paths after adjustment may be determined. When N₂/N₃ < H₂ (i.e., the fourth threshold), it means that the blood vessels of which the diameters are less than 1.5 mm have an impact on the planning of the puncture path. In this case, the one or more candidate paths corresponding to N₃ may be determined as the final candidate paths; when N₂ /N₃ > H₂, it means that a difference between the result of the one or more candidate paths obtained by setting the thin blood vessels of which the diameters are less than 1.5 mm as the penetrable tissues and the result of the one or more candidate paths obtained by setting all the blood vessels as non-puncturable tissues is relatively small. At this time, the one or more candidate paths corresponding to N₂ may be determined as the final candidate paths.

In some embodiments, the fourth threshold may be any reasonable value (e.g., 0.6, and 0.8), which is not limited here.

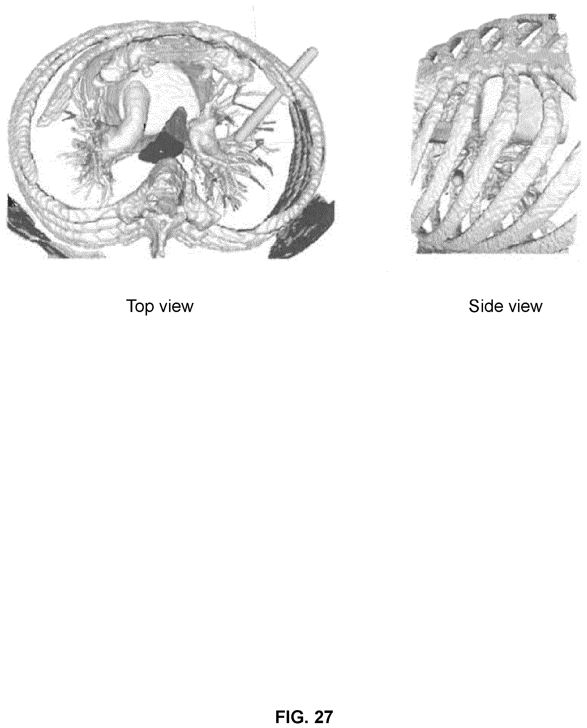

By adaptively adjusting the path planning condition (e.g., the range of the dangerous region) during the process of determining the one or more candidate paths, the impact of the dangerous tissues (e.g., the thick and thin blood vessels) on the puncture path planning can be fully considered, helping to balance (e.g., setting thin blood vessels as the puncturable and non-puncturable tissues) the safety risks and the diversity of recommended paths, and reducing the complications caused by the puncture. For example, as shown in FIG. 27, the puncture path may avoid blood vessels and sternal ribs.

In some embodiments, adaptively adjusting the path planning condition based on the first preset condition may also include: when no candidate path satisfies the path planning condition, resetting puncture parameters. For example, the puncture parameters may include but are not limited to a length and a diameter of a puncture needle, etc. In some embodiments, the initial paths may be determined based on the reset puncture parameters, and the one or more candidate paths may be determined based on the initial paths. Merely by way of example, the processing device 130 may determine the initial paths that satisfy the first constraint condition in the operation 2420 based on the length, the diameter and other parameters of a puncture needle 1 with the shortest puncture depth, and determine the initial path (i.e., the initial paths that satisfy the second constraint) of which the distances from the dangerous region are greater than the preset distance threshold as the one or more candidate paths. When there is no candidate path that satisfies the path planning condition, the system may adaptively change the puncture parameters to a length, a diameter and other parameters corresponding to a puncture needle 2 with a longer puncture depth, and perform the process of determining the initial paths and the candidate paths again (i.e., the operation 2420 and the operation 2430) until at least one candidate path that satisfies the path planning condition is determined.

In 2440, a target path may be determined based on the one or more candidate paths. In some embodiments, the operation 2440 may be performed by the processing device 130 or the path recommendation module 330.

Referring to the description above, in some embodiments, the target path may be determined based on coplanar and non-coplanar features of the one or more candidate paths.

In some embodiments, when the determined candidate paths include both a coplanar candidate path and a non-coplanar candidate path, the target path may be determined based on a shortest puncture depth D₁ in the one or more non-coplanar candidate paths, a shortest puncture depth D₂ in the one or more coplanar candidate paths with a small angle deflection perpendicular to a direction of a bed board, and a shortest puncture depth D₃ in the one or more coplanar candidate paths with a non-small angle deflection. The small angle deflection means that an angle between a vector N passing through a target point perpendicular to the bed board and pointed from a human body to the bed board and a direction vector T corresponding to the target point and a needle entry point is less than a preset threshold (e.g., 2°, 3°, 5°, 10°, 15°, etc.). The non-small angle deflection means that the angle between the vector N passing through the target point perpendicular to the bed board and pointed from the human body to the bed board and the direction vector T corresponding to the target point and the needle entry point is greater than the preset threshold. In some embodiments, the small angle deflection may be within a range of [0°, 15°], such as a coplanar path perpendicular to the direction of the bed board. The smaller the deflection angle corresponding to the puncture path, the more convenient the operation is, especially, the operation in the puncture path in the direction perpendicular to the bed board is most convenient. Specifically, when the shortest puncture depth D₂ or the shortest puncture depth D₃ is minimum, if an absolute value of a difference between the shortest puncture depth D₂ and the shortest puncture depth D₃ is less than a third preset value, the coplanar candidate path with the small angle deflection corresponding to the shortest puncture depth D₂ may be determined as the target path, otherwise, the coplanar candidate path corresponding to the minimum value of the shortest puncture depth D₂ and the shortest puncture depth D₃ may be determined as the target path. When the shortest puncture depth D₁ is minimum, if an absolute value of a difference between the minimum value of the shortest puncture depth D₂ and the shortest puncture depth D₃ and the shortest puncture depth D₁ is less than the third preset value, the coplanar candidate path corresponding to the minimum value may be determined as the target path, otherwise, the non-coplanar candidate path corresponding to the shortest puncture depth D₁ may be determined as the target path. In some embodiments, the third preset value may be determined based on one or more of user habits, puncture operation historical data, the patient information, etc. For example, when the puncture operation is performed manually, the third preset value may be set to a range value of 20 mm of a scan segment of the imaging device 110 based on the convenience of doctor reading.

Merely by way of example, when the determined candidate paths include both the coplanar candidate path and the non-coplanar candidate path, the processing device 130 may calculate the shortest puncture depth D₁ in the non-coplanar candidate path, the shortest puncture depth D₂ in the coplanar candidate path with the small angle deflection in the direction perpendicular to the bed board (e.g., the deflection angle is within a range of [0°, 15°] ), and the shortest puncture depth D₃ in the path with the non-small angle deflection in the direction perpendicular to the bed board. Further, when the minimum value of D₁, D₂, and D₃ corresponds to the coplanar candidate path (i.e., the shortest puncture depth D₂ or the shortest puncture depth D₃ is the minimum), the processing device 130 may compare the sizes of D₂ and D₃, and when D₂ corresponding to the small angle deflection is minimum, processing device 130 may determine the candidate path corresponding to D₂ as the target path; when D₃ corresponding to the non-small angle deflection is the minimum, if D₂-D ₃ < the third preset value (e.g., 20 mm), the processing device 130 may determine the coplanar candidate path corresponding to D₂ with the small angle deflection that is more convenient to operate as the target path; if D₂-D₃ ≥ the third preset value, the processing device 130 may determine the candidate path corresponding to D₃ with a shorter puncture depth as the target path taking puncture depth safety as the goal. When the minimum value of D₁, D₂, and D₃ corresponds to the non-coplanar candidate path (i.e., the shortest puncture depth D₁ is the minimum), the processing device 130 may calculate a minimum value Dₘᵢₙ of D₂ and D₃. If Dₘᵢₙ-D₁ < the third preset value (e.g., 20 mm), the coplanar candidate path corresponding to Dₘᵢₙ may be determined as the target path with the convenience of film reading as the goal; if Dₘᵢₙ-D₁ ≥ the third preset value, the non-coplanar candidate path corresponding to D₁ with a shorter puncture depth may be determined as the target path with the safety as the goal. In some embodiments, a preset value corresponding to the difference (i.e., D₂-D₃) between the shortest puncture depth D₂ and the shortest puncture depth D₃ and a preset value corresponding to a difference (i.e., Dₘᵢₙ₋D₁) between the minimum value of the shortest puncture depth D₂ and the shortest puncture depth D₃ and the shortest puncture depth D₁ may be the same or different values.

In some embodiments, when the one or more candidate paths include only the non-coplanar candidate path, the target path may be determined based on the shortest puncture depth D₁ in the non-coplanar candidate path (e.g., the non-coplanar candidate path corresponding to D₁ may be determined as the target path). In some embodiments, when the one or more candidate paths include only the coplanar candidate path, the target path may be determined based on the shortest puncture depth D₂ in the path of the coplanar candidate path with the small angle deflection perpendicular to the direction of the bed board and the shortest puncture depth D₃ in the path with non-small angle deflection. For example, the processing device 130 may compare the sizes of D₂ and D₃. When D₂ corresponding to the small angle deflection is the minimum, the candidate path corresponding to D₂ may be determined as the target path; when D₃ corresponding to the non-small angle deflection is the minimum, if D₂ -D ₃ < the third preset value (e.g., 20 mm), the coplanar candidate path corresponding to D₂ with the small-angle deflection that is more convenient to operate may be determined as the target path; if D₂ -D₃ ≥ the third preset value, the candidate path corresponding to D₃ with a shorter puncture depth may be determined as the target path with the puncture depth safety as the goal.

It should be noted that the above description of the process 2400 is only for example and explanation, and does not limit the scope of application of the present disclosure. For those skilled in the art, various modifications and changes can be made to the process 2400 under the guidance of the present disclosure. However, such modifications and changes are still within the scope of the present disclosure.

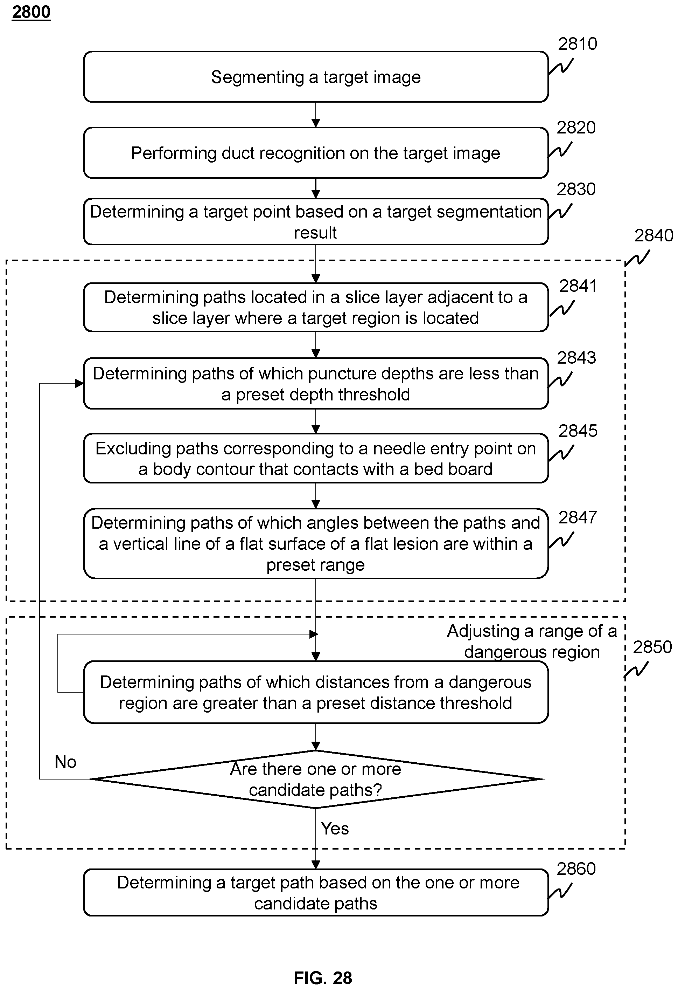

FIG. 28 is a schematic diagram illustrating an exemplary method for puncture path planning according to some embodiments of the present disclosure. In some embodiments, a process 2800 may be performed by the system 100 for puncture path planning (e.g., the processing device 130) or the device 200 for puncture path planning. For example, the process 2800 may be stored in a storage device (e.g., the storage device 150, and the storage unit of the system) in the form of a program or instruction. When the processor or the module shown in FIG. 3 performs the program or instruction, the process 2800 may be implemented.

Merely by way of example only, as shown in FIG. 28, after the processing device 130 obtains a target image of a target object from the imaging device 110 or the storage device 150, the target image may be segmented (e.g., by the segmentation method of the process 600 ), and a duct type in a segmented image may be determined (e.g., by the method for duct recognition of the process 1700), a target point may be determined based on a segmentation result, and then a target path may be determined based on the target point and constraints.

In 2810, a target image may be segmented.

In some embodiments, the processing device 130 may obtain a preliminary segmentation result by segmenting the target image using a deep learning model, threshold segmentation, etc. In some embodiments, the processing device 130 may obtain a target structure mask by performing rough segmentation on a target structure in the target image; determine positioning information of the target structure mask based on soft connected domain analysis; and obtain the preliminary segmentation result by performing precise segmentation on the target structure based on the positioning information of the target structure mask. More descriptions regarding obtaining the segmentation result through the rough segmentation and the precise segmentation may be found in the descriptions of FIGs. 6-16.

In 2820, duct recognition may be performed on the target image.

In some embodiments, a target segmentation result of the target image may be obtained by performing duct recognition based on the preliminary segmentation result. In some embodiments, the target segmentation result may include ducts of different levels and/or types of ducts.

In some embodiments, the processing device 130 may obtain a first segmentation result of the target image based on a first segmentation model; obtain a first duct skeleton set by performing skeletonization processing on the first segmentation result; obtain a second segmentation result of the target image based on a second segmentation model; and obtain a fusion result by fusing the first segmentation result and the second segmentation result. In some embodiments, the processing device 130 may obtain a second duct skeleton of a duct of an undetermined type by performing the skeletonization processing on the fusion result; obtain one or more first duct skeletons, a minimum spatial distance between each of the one or more first duct skeletons and the second duct skeleton being less than a second threshold, designate the one or more first duct skeletons as one or more reference duct skeletons; determine a spatial distance between the second duct skeleton and the reference duct skeleton, and determine two points with a minimum spatial distance as a set of closest points; and determine a duct type of one of the at least one duct of the undetermined type based on the set of closest points, thereby obtaining a target segmentation result. More descriptions regarding obtaining the duct type through the first segmentation model and the second segmentation model may be found in the related descriptions of FIGs. 17-23.

In some embodiments, the processing device 130 may further classify tissues inside a target organ based on the target segmentation result to determine dangerous tissues. For example, the processing device 130 may determine a central point of each blood vessel by boundary corrosion according to a blood vessel mask inside the target organ obtained by segmentation, calculate a minimum distance from the central point to the boundary of the blood vessel as a radius of the blood vessel of the point, and set, based on a preset blood vessel resolution threshold Dt, blood vessels less than the threshold Dt as thin blood vessels, and set blood vessels greater than the threshold Dt as thick blood vessels, and distinguish the thin blood vessels and the thick blood vessels with different labeling values.

In 2830, a target point may be determined based on the target segmentation result.

In some embodiments, the processing device 130 may determine a target region according to the target segmentation result, determine a volume center or a center of gravity of the target region by boundary erosion and other methods, and determine the volume center or the center of gravity of the target region as the target point. More descriptions may be found in the related descriptions in FIG. 24.

In 2840, initial paths may be determined according to the target point and a first constraint.

Merely by way of example, in 2841, the processing device 130 may determine, based on the target point, paths located in a slice layer adjacent to a slice layer where the target region is located as first initial paths; in 2843, the processing device 130 may determine, based on puncture parameters (e.g., a current set length of a puncture needle), paths in the first initial paths of which puncture depths are less than a preset depth threshold as second initial paths; in 2845, the processing device 130 may exclude second initial paths corresponding to a needle entry point on a body contour that contacts with a bed board to obtain third initial paths. In some embodiments, in case of a flat lesion, the processing device 130 may further perform operation 2847 to determine paths in the third initial paths of which angles between the paths and a vertical line of a flat surface of the flat lesion are within a preset range as final initial paths.

It is understood that the order of the operations 2841-2847 shown in FIG. 28 is only an example. In some embodiments, at least one of the operations 2841-2847 can be executed in any reasonable order (e.g., after the operation 2841, the operation 2845 may be executed first and then the operation 2843 may be performed), which is not limited in the present disclosure.

In 2850, one or more candidate paths may be determined from the initial paths.

In some embodiments, the processing device 130 may determine the one or more candidate paths from the initial paths based on a second constraint. In some embodiments, in the process of determining the one or more candidate paths, the processing device 130 may adaptively adjust a path planning condition based on a first preset condition. Merely by way of example, the processing device 130 may determine paths from the initial paths of which distances from a dangerous region are greater than a preset distance threshold, and when a ratio of a count of the one or more candidate paths to a count of initial paths is less than a third threshold, adjust a range of the dangerous region, and determine the one or more candidate paths from the initial paths again based on an adjusted dangerous region; when the ratio of the count of the one or more candidate paths obtained before adjusting the range of the dangerous region to the count of the one or more candidate paths obtained after adjustment is less than a fourth threshold, use the one or more candidate path obtained after adjustment as the final candidate paths; when the ratio of the count of the one or more candidate paths obtained before adjusting the range of the dangerous region to the count of the one or more candidate paths obtained after adjustment is greater than the fourth threshold, use the one or more candidate paths obtained before adjustment as the final candidate paths.

In some embodiments, when there is no candidate path that satisfies the path planning condition after the operation 2850 is performed, the processing device 130 may reset the puncture parameters (e.g., when the path cannot be effectively planned based on a preset depth threshold determined based on a length of a puncture needle, the length of the puncture needle may be increased, i.e., the preset depth threshold may be increased), and the operations 2840-2850 may be performed again according to the puncture parameters until the one or more candidate paths that satisfy the path planning condition are determined. If there are one or more candidate paths that satisfy the path planning condition, operation 2860 may be performed.

In 2860, the processing device 130 may determine a target path based on the one or more candidate paths. In some embodiments, the processing device 130 may calculate a shortest puncture depth D₁ in a non-coplanar candidate path, a shortest puncture depth D₂ in a path of a coplanar candidate path with a small angle deflection perpendicular to a direction of a bed board, and a shortest puncture depth D3 in a path with a non-small angle deflection, and determine the target path based on the shortest puncture depth D₁, the shortest puncture depth D₂, and the shortest puncture depth D₃. More descriptions may be found in FIG. 24 and related descriptions thereof, which are not repeated here.

In some embodiments, the processing device 130 may recommend the target path to a user, and/or control the end effector 120 to perform puncture according to user feedback (e.g., a target path selected by the user or a replanned puncture path).

It should be noted that the above description of the process 2800 is only for example and explanation, and does not limit the scope of application of the present disclosure. For those skilled in the art, various modifications and changes can be made to the process 2800 under the guidance of the present disclosure. For example, the operation 2810 and the operation 2820 may be performed at the same time. As another example, the operation 2830 may be performed first, and then the operation 2820 may be performed, i.e., the target point may be first determined based on the segmentation result obtained in the operation 2810, and then the dangerous region may be determined by performing duct recognition. However, such modifications and changes are still within the scope of the present disclosure.

In some embodiments of the present disclosure, with application of the method and/or system for puncture path planning, (1) the safe and feasible optimal puncture path is calculated using at least two constraints based on the clinical requirements of puncture biopsy, effectively shortening the planning time, improving the accuracy of puncture, and reducing complications; (2) the initial paths of which the distances from the dangerous regions are greater than the preset distance threshold are determined as the one or more candidate paths, which can effectively control the risk of the puncture operation; (3) the path planning process is adaptively adjusted, and safety and path planning diversity are fully considered, improving the accuracy and efficiency of path planning; (4) the final target path is determined by comprehensively considering the convenience and safety of the operation, ensuring the accuracy and safety of path planning; (5) by using the method of soft connected domain analysis in the rough segmentation stage, the target structure region can be accurately retained while the false positive region can be effectively excluded, which not only improves the accuracy of positioning of the target structure in the rough positioning stage, but also facilitates the subsequent precise segmentation; and (6) by performing duct growth on the segmentation result of the low-richness but high-accuracy first segmentation model using the segmentation result of the high-richness second segmentation model, the two models are fused, and the multi-class duct segmentation results with high richness and high accuracy can be accurately and effectively obtained.

It should be noted that different embodiments may produce different beneficial effects. In different embodiments, the beneficial effects that may be produced may be any one or a combination of the above, or any other beneficial effects that may be obtained.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and "some embodiments" mean that a particular feature, structure, or feature described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or features may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the spirit and scope of the disclosed embodiments. For example, although the implementation of various parts described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

In some embodiments, numbers describing the number of ingredients and attributes are used. It should be understood that such numbers used for the description of the embodiments use the modifier "about", "approximately", or "substantially" in some examples. Unless otherwise stated, "about", "approximately", or "substantially" indicates that the number is allowed to vary by ±20%. Correspondingly, in some embodiments, the numerical parameters used in the description and claims are approximate values, and the approximate values may be changed according to the required features of individual embodiments. In some embodiments, the numerical parameters should consider the prescribed effective digits and adopt the method of general digit retention. Although the numerical ranges and parameters used to confirm the breadth of the range in some embodiments of the present disclosure are approximate values, in specific embodiments, settings of such numerical values are as accurate as possible within a feasible range.

For each patent, patent application, patent application publication, or other materials cited in the present disclosure, such as articles, books, specifications, publications, documents, or the like, the entire contents of which are hereby incorporated into the present disclosure as a reference. The application history documents that are inconsistent or conflict with the content of the present disclosure are excluded, and the documents that restrict the broadest scope of the claims of the present disclosure (currently or later attached to the present disclosure) are also excluded. It should be noted that if there is any inconsistency or conflict between the description, definition, and/or use of terms in the auxiliary materials of the present disclosure and the content of the present disclosure, the description, definition, and/or use of terms in the present disclosure is subject to the present disclosure.

Finally, it should be understood that the embodiments described in the present disclosure are only used to illustrate the principles of the embodiments of the present disclosure. Other variations may also fall within the scope of the present disclosure. Therefore, as an example and not a limitation, alternative configurations of the embodiments of the present disclosure may be regarded as consistent with the teaching of the present disclosure. Accordingly, the embodiments of the present disclosure are not limited to the embodiments introduced and described in the present disclosure explicitly.

## Claims

1. A system for puncture path planning, comprising:
at least one storage medium including a set of instructions; and
one or more processors in communication with the at least one storage medium, wherein when executing the instructions, the one or more processors are configured to:
determine a target point based on a target image;
determine one or more candidate paths based on the target point and at least two constraints, wherein a path planning condition is adaptively adjusted based on a first preset condition during determining the one or more candidate paths; and
determine a target path based on the one or more candidate paths.

2. The system of claim 1, wherein the determining the target point based on a target image includes:
obtaining a target structure mask by performing rough segmentation on a target structure in the target image;
determining positioning information of the target structure mask based on soft connected domain analysis;
obtaining a segmentation result by performing precise segmentation on the target structure based on the positioning information of the target structure mask; and
determining the target point based on the segmentation result.

3. The system of claim 2, wherein the determining positioning information of the target structure mask based on soft connected domain analysis includes:
determining a count of connected domains in the target structure mask; and
determining the positioning information of the target structure mask based on the count of the connected domains.

4. The system of claim 3, wherein the determining the positioning information of the target structure mask based on the count of the connected domains includes:
in response to determining that the count of the connected domains is greater than a first preset value and less than a second preset value, determining a ratio of an area of a maximum connected domain to a total area of the connected domains in the target structure mask;
determining whether the ratio is greater than a first threshold;
in response to determining that the ratio is greater than the first threshold, determining that the maximum connected domain is a retained connected domain; or in response to determining that the ratio is less than or equal to the first threshold, determining that each connected domain in the target structure mask is the retained connected domain; and
determining the positioning information of the target structure mask based on the retained connected domain.

5. The system of claim 3, wherein the determining the positioning information of the target structure mask based on the count of the connected domains includes:
in response to determining that the count of the connected domains is greater than or equal to a second preset value, obtaining a sorting result by sorting the connected domains in the target structure mask in a descending order of areas of the connected domains;
determining top n connected domains as target connected domains based on the sorting result;
determining a retained connected domain from the target connected domains based on a second preset condition, the retained connected domain at least including a maximum connected domain in the target structure mask; and
determining the positioning information of the target structure mask based on the retained connected domain.

6. The system of claim 2, wherein
the positioning information of the target structure mask includes position information of a bounding rectangle of the target structure mask; and/or
the determining the positioning information of the target structure mask includes: positioning the target structure mask based on positioning coordinates of a preset structure.

7. The system of claim 2, wherein the performing precise segmentation on the target structure based on the positioning information of the target structure mask includes:
obtaining a preliminary precise segmentation result by performing preliminary precise segmentation on the target structure;
determining whether the positioning information of the target structure mask is accurate based on the preliminary precise segmentation result; and
in response to determining that the positioning information of the target structure mask is accurate, using the preliminary precise segmentation result as a target segmentation result; or in response to determining that the positioning information of the target structure mask is not accurate, determining the target segmentation result of the target structure by an adaptive sliding window mode.

8. The system of claim 7, wherein the determining the target segmentation result of the target structure by an adaptive sliding window mode includes:
determining a target direction, the positioning information having a deviation in the target direction; and
determining the target segmentation result of the target structure by performing adaptive sliding window calculation in the target direction based on an overlap rate parameter.

9. The system of claim 1, wherein the one or more processors are further configured to:
obtain a first segmentation result of the target image based on a first segmentation model;
obtain a first duct skeleton set by performing skeletonization processing on the first segmentation result, wherein the first duct skeleton set includes at least one first duct skeleton of a determined type;
obtain a second segmentation result of the target image based on a second segmentation model, wherein the second segmentation result includes at least one duct of an undetermined type;
obtain a fusion result by fusing the first segmentation result and the second segmentation result; and
determine a dangerous region based on the fusion result.

10. The system of claim 9, wherein
at least one duct in the second segmentation result is not included in the first segmentation result; and the determining a dangerous region based on the fusion result includes:
obtaining a second duct skeleton of one of the at least one duct of the undetermined type by performing the skeletonization processing on the fusion result;
obtaining one or more first duct skeletons, a minimum spatial distance between each of the one or more first duct skeletons and the second duct skeleton is less than a second threshold;
designating the one or more first duct skeletons as one or more reference duct skeletons;
determining one or more spatial distances each of which is between the second duct skeleton and one of the one or more reference duct skeletons;
determining two points with a minimum spatial distance among the one or more spatial distances as a set of closest points;
determining a duct type of the one of the at least one duct of the undetermined type based on the set of closest points; and
determining the dangerous region based on the duct type.

11. The system of claim 10, wherein the determining a duct type of the one of the at least one duct of the undetermined type based on the set of closest points includes:
in response to determining that a count of the one or more reference duct skeletons equals 1, determining the duct type of the one of the at least one duct of the undetermined type based on positions of the set of closest points;
in response to determining that a count of the one or more reference duct skeletons exceeds 1 determining one or more candidate duct skeletons based on the set of closest points, and determining the duct type of the one of the at least one duct of the undetermined type based on the one or more candidate duct skeletons.

12. The system of claim 10, wherein:
the second threshold is obtained at least based on a portion of an organism represented in the target image; and/or
the second threshold is obtained through a machine learning model based on a medical image of a portion corresponding to the same type of organism and a type determination result.

13. The system of claim 1, wherein the at least two constraints include:
a distance between a path and a dangerous region being greater than a preset distance threshold,
the path being located in a slice layer adjacent to a slice layer where a target region is located,
excluding a needle entry point on a body contour that contacts a bed board,
a puncture depth of the path being less than a preset depth threshold, or
an angle between the path and a vertical line of a flat surface of a flat lesion being within a preset range.

14. The system of claim 1, wherein the determining one or more candidate paths based on the target point and at least two constraints includes:
determining initial paths based on the target point and a first constraint;
determining the one or more candidate paths from the initial paths based on a second constraint; wherein
the first constraint includes that: the path is located in a slice layer adjacent to a slice layer where a target region is located, a needle entry point on a body contour that contacts a bed board is excluded, a puncture depth of the path is less than a preset depth threshold, or an angle between the path and a vertical line of a flat surface of a flat lesion is within a preset range; and
the second constraint includes that a distance between the path and a dangerous region is greater than a preset distance threshold.

15. The system of claim 14, wherein
the adaptively adjusting a path planning condition based on a first preset condition includes:
when a ratio of a count of the one or more candidate paths to a count of the initial paths is less than a third threshold, adjusting a range of the dangerous region; and
the determining the one or more candidate paths from the initial paths further includes:
determining one or more candidate paths from the initial paths based on an adjusted dangerous region; and
when a ratio of a count of the one or more candidate paths obtained before adjusting the range of the dangerous region to a count of the one or more candidate paths obtained after adjusting the range of the dangerous region is less than a fourth threshold, using the one or more candidate paths obtained after adjusting the range of the dangerous region as final candidate paths; or
when the ratio of the count of the one or more candidate paths obtained before adjusting the range of the dangerous region to the count of the one or more candidate paths obtained after adjusting the range of the dangerous region is greater than the fourth threshold, using the one or more candidate paths obtained before adjusting the range of the dangerous region as the final candidate paths.

16. The system of claim 1, wherein the adaptively adjusting a path planning condition based on a first preset condition includes:
when no candidate path meets the path planning condition, resetting puncture parameters, the puncture parameters including at least one of a length or a diameter of a puncture needle.

17. The system of claim 1, wherein
the determining a target path based on the one or more candidate paths includes:
in response to determining that the one or more candidate paths include one or more coplanar candidate paths and one or more non-coplanar candidate paths, determining the target path based on a shortest puncture depth D₁ in the one or more non-coplanar candidate paths, a shortest puncture depth D₂ in the one or more coplanar candidate paths with a small angle deflection perpendicular to a direction of a bed board, and a shortest puncture depth D₃ in the one or more coplanar candidate paths with a non-small angle deflection;
if each of the one or more candidate paths is a non-coplanar candidate path, determining the target path based on the D₁; and
if each of the one or more candidate paths is a coplanar candidate path, determining the target path based on the D₂ and the D₃.

18. The system of claim 17, wherein the determining the target path based on a shortest puncture depth D₁ in the one or more non-coplanar candidate paths, a shortest puncture depth D₂ in the one or more coplanar candidate paths with a small angle deflection perpendicular to a direction of a bed board, and a shortest puncture depth D₃ in the one or more coplanar candidate paths with a non-small angle deflection includes:
when the shortest puncture depth D₂ or the shortest puncture depth D₃ among the shortest puncture depth D₁, the shortest puncture depth D₂, and the shortest puncture depth D₃ is minimum, if an absolute value of a difference between the shortest puncture depth D₂ and the shortest puncture depth D₃ is less than a third preset value, determining a coplanar candidate path with the small angle deflection corresponding to the shortest puncture depth D₂ as the target path; or, determining a coplanar candidate path corresponding to a minimum value of the shortest puncture depth D₂ and the shortest puncture depth D₃ as the target path; and
when the shortest puncture depth D₁ among the shortest puncture depth D₁, the shortest puncture depth D₂, and the shortest puncture depth D₃ is minimum, if an absolute value of a difference between the shortest puncture depth D₁ and the minimum value of the shortest puncture depth D₂ and the shortest puncture depth D₃ is less than the third preset value, determining a coplanar candidate path corresponding to the minimum value as the target path; otherwise, determining the non-coplanar candidate path corresponding to the shortest puncture depth D₁ as the target path.

19. A system for medical image segmentation, comprising:
at least one storage medium including a set of instructions; and
one or more processors in communication with the at least one storage medium, wherein when executing the instructions, the one or more processors are configured to:
obtain a target image;
obtain a target structure mask by performing rough segmentation on a target structure in the target image;
determine positioning information of the target structure mask based on soft connected domain analysis; and
obtain a segmentation result by performing precise segmentation on the target structure based on the positioning information of the target structure mask.

20. The system of claim 19, wherein the determining positioning information of the target structure mask based on soft connected domain analysis includes:
determining a count of connected domains in the target structure mask; and
determining the positioning information of the target structure mask based on the count of the connected domains.

21. The system of claim 20, wherein the determining the positioning information of the target structure mask based on the count of the connected domains includes:
in response to determining that the count of the connected domains is greater than a first preset value and less than a second preset value, determining a ratio of an area of a maximum connected domain to a total area of the connected domains in the target structure mask;
determining whether the ratio is greater than a first threshold;
in response to determining that the ratio is greater than the first threshold, determining that the maximum connected domain is a retained connected domain; or in response to determining that the ratio is less than or equal to the first threshold, determining that each connected domain in the target structure mask is the retained connected domain; and
determining the positioning information of the target structure mask based on the retained connected domain.

22. The system of claim 20, wherein the determining the positioning information of the target structure mask based on the count of the connected domains includes:
in response to determining that the count of the connected domains is greater than or equal to a second preset value, obtaining a sorting result by sorting the connected domains in the target structure mask in a descending order of areas of the connected domains;
determining top n connected domains as target connected domains based on a sorting result;
determining a retained connected domain from the target connected domains based on a second preset condition, the retained connected domain at least including a maximum connected domain in the target structure mask; and
determining the positioning information of the target structure mask based on the retained connected domain.

23. The system of claim 19, wherein
the positioning information of the target structure mask includes position information of a bounding rectangle of the target structure mask; and/or
the determining the positioning information of the target structure mask includes: positioning the target structure mask based on positioning coordinates of a preset structure.

24. The system of claim 19, wherein the performing precise segmentation on the target structure based on the positioning information of the target structure mask includes:
obtaining a preliminary precise segmentation result by performing preliminary precise segmentation on the target structure;
determining whether the positioning information of the target structure mask is accurate based on the preliminary precise segmentation result; and
in response to determining that the positioning information of the target structure mask is accurate, using the preliminary precise segmentation result as a target segmentation result; or in response to determining that the positioning information of the target structure mask is not accurate, determining the target segmentation result of the target structure by an adaptive sliding window mode.

25. The system of claim 24, wherein the determining the target segmentation result of the target structure by an adaptive sliding window mode includes:
determining a target direction, the positioning information having a deviation in the target direction; and
determining the target segmentation result of the target structure by performing adaptive sliding window calculation in the target direction based on an overlap rate parameter.

26. A system for duct recognition in an organism, comprising:
at least one storage medium comprising a set of instructions; and
one or more processors in communication with the at least one storage medium, wherein when executing the instructions, the one or more processors are configured to:
obtain a target image of the organism;
obtain a first segmentation result of the target image based on a first segmentation model;
obtain a first duct skeleton set by performing skeletonization processing on the first segmentation result, wherein the first duct skeleton set includes at least one first duct skeleton of a determined type;
obtain a second segmentation result of the target image based on a second segmentation model,
wherein the second segmentation result includes at least one duct of an undetermined type; and
obtain a fusion result by fusing the first segmentation result and the second segmentation result.

27. The system of claim 26, wherein
at least one duct in the second segmentation result is not included in the first segmentation result; and the one or more processors are further configured to:
obtain a second duct skeleton of one of the at least one duct of the undetermined type by performing the skeletonization processing on the fusion result;
obtain one or more first duct skeletons of which a minimum spatial distance between each of the one or more first duct skeletons and the second duct skeleton is less than a second threshold;
designating the one or more first duct skeletons as one or more reference duct skeletons;
determine one or more spatial distances each of which is between the second duct skeleton and one of the one or more reference duct skeletons;
determine two points with a minimum spatial distance among the one or more spatial distances as a set of closest points; and
determine a duct type of the one of the at least one duct of the undetermined type based on the set of closest points.

28. The system of claim 27, wherein the determining a duct type of the duct of the undetermined type based on the set of closest points includes:
in response to determining that a count of the one or more reference duct skeletons equals 1,
determining the duct type of the one of the at least one duct of the undetermined type based on positions of the set of closest points;
in response to determining that a count of the one or more reference duct skeletons exceeds 1,
determining one or more candidate duct skeletons based on the set of closest points, and determining the duct type the one of the at least one duct of the undetermined type based on the one or more candidate duct skeletons.

29. The system of claim 27, wherein:
the second threshold is obtained at least based on a portion of an organism represented in the target image; and/or
the second threshold is obtained through a machine learning model based on a medical image of a portion corresponding to the same type of organism and a type determination result.
